(19) 

Europäisches
Patentamt
European
Patent Office
Office européen
des brevets

(11) **EP 4 257 603 A1**

(12) **EUROPEAN PATENT APPLICATION**
published in accordance with Art. 153(4) EPC

(43) Date of publication:
**11.10.2023 Bulletin 2023/41**

(21) Application number: **21900086.6**

(22) Date of filing: **03.12.2021**

(51) International Patent Classification (IPC):
*C07K 16/22* (2006.01)  *C12N 15/13* (2006.01)
*C12N 15/63* (2006.01)  *A61K 39/395* (2006.01)
*A61P 9/10* (2006.01)  *A61P 9/12* (2006.01)
*A61P 19/02* (2006.01)  *A61P 27/06* (2006.01)
*A61P 35/00* (2006.01)  *A61P 1/16* (2006.01)
*A61P 3/10* (2006.01)  *A61P 11/00* (2006.01)
*A61P 13/12* (2006.01)  *A61P 17/00* (2006.01)

(52) Cooperative Patent Classification (CPC):
**A61K 39/395; A61P 1/16; A61P 3/10; A61P 9/10;
A61P 9/12; A61P 11/00; A61P 13/12; A61P 17/00;
A61P 19/02; A61P 27/06; A61P 35/00;
C07K 16/00; C07K 16/22; C12N 15/63**

(86) International application number:
**PCT/CN2021/135203**

(87) International publication number:
**WO 2022/117060 (09.06.2022 Gazette 2022/23)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **03.12.2020 CN 202011405490**
**18.11.2021 CN 202111366667**

(71) Applicants:
• **Jiangsu Hengrui Pharmaceuticals Co., Ltd.
Lianyungang, Jiangsu 222047 (CN)**
• **Shanghai Hengrui Pharmaceutical Co., Ltd.
Shanghai 200245 (CN)**

(72) Inventors:
• **MA, Xiazhen**
**Shanghai 200245 (CN)**
• **WU, Tingting**
**Shanghai 200245 (CN)**
• **LIU, Xun**
**Shanghai 200245 (CN)**

(74) Representative: **Regimbeau**
**20, rue de Chazelles**
**75847 Paris Cedex 17 (FR)**

Remarks:
The complete document including Reference
Table(s) and the Sequence Listing(s) can be
downloaded from the EPO website

(54) **PHARMACEUTICAL COMPOSITION COMPRISING ANTI-CONNECTIVE TISSUE GROWTH FACTOR ANTIBODY**

(57)     Provided is a pharmaceutical composition comprising an anti-connective tissue growth factor antibody. In another aspect, further provided is a use of the pharmaceutical composition comprising an anti-connective tissue growth factor antibody.

EP 4 257 603 A1

**Description**

**TECHNICAL FIELD**

The present disclosure belongs to the field of pharmaceutical formulations and particularly relates to a pharmaceutical composition comprising an antibody and use thereof as a medicament.

**BACKGROUND**

The statements herein merely provide background information related to the present disclosure and may not necessarily constitute the prior art.

Expression of connective tissue growth factor (CTGF) is induced by members of the transforming growth factor β (TGF(β) superfamily. The superfamily includes TGFβ-1, -2, and -3, bone morphogenetic protein (BMP)-2, and activin. Many modulators including dexamethasone, thrombin, vascular endothelial growth factor (VEGF), and angiotensin II, and environmental stimuli including hyperglycemia and hypertension also induce CTGF expression.

TGFβ stimulation of CTGF expression is rapid and prolonged and does not require persistent application of TGFβ. TGFβ activates transcription via the DNA regulatory elements in the CTGF promoter, leading to enhanced CTGF expression. CTGF expression is up-regulated in glomerulonephritis, IgA nephropathy, focal and segmental glomerulosclerosis and diabetic nephropathy. An increase in the number of cells expressing CTGF is also observed at sites of chronic tubulointerstitial damage, and CTGF levels correlate with the degree of damage. Further, CTGF expression is increased in the glomeruli and tubulointerstitium in a variety of renal diseases associated with scarring and sclerosis of renal parenchyma. Elevated levels of CTGF are also associated with liver fibrosis, myocardial infarction, and pulmonary fibrosis. For example, in patients with idiopathic pulmonary fibrosis (IPF), CTGF is strongly up-regulated in biopsies and bronchoalveolar lavage fluid cells. Thus, CTGF is a valid therapeutic target in the diseases described above.

**SUMMARY**

The present disclosure provides a pharmaceutical composition comprising an anti-CTGF antibody. The composition has the advantages of having good stability, good lyophilization morphology, etc.

In some embodiments, the pharmaceutical composition according to any one of the above is provided, wherein the anti-CTGF antibody comprises a heavy chain variable region and a light chain variable region, wherein:

i) the heavy chain variable region comprises HCDR1, HCDR2, and HCDR3 having identical sequences to those of a heavy chain variable region set forth in SEQ ID NO: 6, and the light chain variable region comprises LCDR1, LCDR2, and LCDR3 having identical sequences to those of a light chain variable region set forth in SEQ ID NO: 7; or
ii) the heavy chain variable region comprises HCDR1, HCDR2, and HCDR3 having identical sequences to those of a heavy chain variable region set forth in SEQ ID NO: 8, and the light chain variable region comprises LCDR1, LCDR2, and LCDR3 having identical sequences to those of a light chain variable region set forth in SEQ ID NO: 9.

In some embodiments, the pharmaceutical composition according to any one of the above is provided, wherein the anti-CTGF antibody comprises a heavy chain variable region and a light chain variable region, wherein:

ii-i) the heavy chain variable region comprises HCDR1, HCDR2, and HCDR3 having identical sequences to those of a heavy chain variable region set forth in SEQ ID NO: 69, and the light chain variable region comprises LCDR1, LCDR2, and LCDR3 having identical sequences to those of a light chain variable region set forth in SEQ ID NO: 70; or
ii-ii) the heavy chain variable region comprises HCDR1, HCDR2, and HCDR3 having identical sequences to those of a heavy chain variable region set forth in SEQ ID NO: 85, and the light chain variable region comprises LCDR1, LCDR2, and LCDR3 having identical sequences to those of a light chain variable region set forth in SEQ ID NO: 70. In some embodiments, the pharmaceutical composition according to any one of the above is provided, wherein the anti-CTGF antibody comprises a heavy chain variable region and a light chain variable region, wherein:
iii) the heavy chain variable region comprises HCDR1, HCDR2, and HCDR3 set forth in SEQ ID NO: 10, SEQ ID NO: 11, and SEQ ID NO: 12, respectively, and the light chain variable region comprises LCDR1, LCDR2, and LCDR3 set forth in SEQ ID NO: 13, SEQ ID NO: 14, and SEQ ID NO: 15, respectively; or
iv) the heavy chain variable region comprises HCDR1, HCDR2, and HCDR3 set forth in SEQ ID NO: 16, SEQ ID NO: 17, and SEQ ID NO: 18, respectively, and the light chain variable region comprises LCDR1, LCDR2, and LCDR3 set forth in SEQ ID NO: 19, SEQ ID NO: 20, and SEQ ID NO: 21, respectively.

In some embodiments, the pharmaceutical composition according to any one of the above is provided, wherein the anti-CTGF antibody comprises a heavy chain variable region and a light chain variable region, wherein:

iv-i) the heavy chain variable region comprises HCDR1, HCDR2, and HCDR3 set forth in SEQ ID NO: 71, SEQ ID NO: 72, and SEQ ID NO: 73, respectively, and the light chain variable region comprises LCDR1, LCDR2, and LCDR3 set forth in SEQ ID NO: 74, SEQ ID NO: 75, and SEQ ID NO: 76, respectively; or
iv-ii) the heavy chain variable region comprises HCDR1, HCDR2, and HCDR3 set forth in SEQ ID NO: 102, SEQ ID NO: 103, and SEQ ID NO: 104, respectively, and the light chain variable region comprises LCDR1, LCDR2, and LCDR3 set forth in SEQ ID NO: 74, SEQ ID NO: 75, and SEQ ID NO: 76, respectively.

In some embodiments, the anti-CTGF antibody according to any one of the above is a murine antibody, a chimeric antibody, or a humanized antibody.

In some embodiments, the pharmaceutical composition according to any one of the above is provided, wherein the anti-CTGF antibody comprises a heavy chain variable region and a light chain variable region, wherein:

(v-1) the heavy chain variable region's amino acid sequence has at least 90% sequence identity to SEQ ID NO: 6, 27, 28, or 29, and the light chain variable region's amino acid sequence has at least 90% sequence identity to SEQ ID NO: 7, 22, 23, 24, 25, or 26;
(vi-1) the heavy chain variable region's amino acid sequence has at least 90% sequence identity to SEQ ID NO: 8, 33, 34, 35, or 36, and the light chain variable region's amino acid sequence has at least 90% sequence identity to SEQ ID NO: 9, 30, 31, or 32; or
(vi-i) the heavy chain variable region's amino acid sequence has at least 90% sequence identity to SEQ ID NO: 69, 81, 82, 83, 84, or 85, and the light chain variable region's amino acid sequence has at least 90% sequence identity to SEQ ID NO: 70, 77, 78, 79, or 80.

In some embodiments, the pharmaceutical composition according to any one of the above is provided, wherein the anti-CTGF antibody comprises a heavy chain variable region and a light chain variable region, wherein:

(v) the heavy chain variable region has at least 90%, 91%, 92%, 93%, 94%, 95%, 96%, 97%, 98%, 99%, or 100% identity to a heavy chain variable region set forth in SEQ ID NO: 6, 27, 28, or 29, and the light chain variable region has at least 90%, 91%, 92%, 93%, 94%, 95%, 96%, 97%, 98%, 99%, or 100% identity to a light chain variable region set forth in SEQ ID NO: 7, 22, 23, 24, 25, or 26; or
(vi) the heavy chain variable region has at least 90%, 91%, 92%, 93%, 94%, 95%, 96%, 97%, 98%, 99%, or 100% identity to a heavy chain variable region set forth in SEQ ID NO: 8, 33, 34, 35, or 36, and the light chain variable region has at least 90%, 91%, 92%, 93%, 94%, 95%, 96%, 97%, 98%, 99%, or 100% identity to a light chain variable region set forth in SEQ ID NO: 9, 30, 31, or 32; or
the heavy chain variable region has at least 90%, 91%, 92%, 93%, 94%, 95%, 96%, 97%, 98%, 99%, or 100% identity to the heavy chain variable region set forth in SEQ ID NO: 6, and the light chain variable region has at least 90%, 91%, 92%, 93%, 94%, 95%, 96%, 97%, 98%, 99%, or 100% identity to the light chain variable region set forth in SEQ ID NO: 7;
the heavy chain variable region has at least 90%, 91%, 92%, 93%, 94%, 95%, 96%, 97%, 98%, 99%, or 100% identity to the heavy chain variable region set forth in SEQ ID NO: 27, 28, or 29, and the light chain variable region has at least 90%, 91%, 92%, 93%, 94%, 95%, 96%, 97%, 98%, 99%, or 100% identity to the light chain variable region set forth in SEQ ID NO: 22, 23, 24, 25, or 26;
the heavy chain variable region has at least 90%, 91%, 92%, 93%, 94%, 95%, 96%, 97%, 98%, 99%, or 100% identity to the heavy chain variable region set forth in SEQ ID NO: 8, and the light chain variable region has at least 90%, 91%, 92%, 93%, 94%, 95%, 96%, 97%, 98%, 99%, or 100% identity to the light chain variable region set forth in SEQ ID NO: 9;
the heavy chain variable region has at least 90%, 91%, 92%, 93%, 94%, 95%, 96%, 97%, 98%, 99%, or 100% identity to the heavy chain variable region set forth in SEQ ID NO: 33, 34, 35, or 36, and the light chain variable region has at least 90%, 91%, 92%, 93%, 94%, 95%, 96%, 97%, 98%, 99%, or 100% identity to the light chain variable region set forth in SEQ ID NO: 30, 31, or 32.

In some embodiments, the pharmaceutical composition according to any one of the above is provided, wherein the anti-CTGF antibody comprises a heavy chain variable region and a light chain variable region, wherein:
(vi-ii) the heavy chain variable region has at least 90%, 91%, 92%, 93%, 94%, 95%, 96%, 97%, 98%, 99%, or 100% identity to a heavy chain variable region set forth in SEQ ID NO: 69, 81, 82, 83, 84, or 85, and the light chain variable region has at least 90%, 91%, 92%, 93%, 94%, 95%, 96%, 97%, 98%, 99%, or 100% identity to a light chain variable region set forth in SEQ ID NO: 70, 77, 78, 79, or 80.

In some embodiments, the pharmaceutical composition according to any one of the above is provided, wherein the anti-CTGF antibody is a humanized antibody comprising a framework region of a human antibody or a framework region variant thereof, and the framework region variant has at most 10 back mutations on a light chain framework region and/or a heavy chain framework region of the human antibody. In some embodiments, the pharmaceutical composition according to any one of the above is provided, wherein the anti-CTGF antibody comprises a heavy chain variable region and a light chain variable region as described below:

(a) the light chain variable region comprising LCDR1, LCDR2, and LCDR3 set forth in SEQ ID NO: 13, SEQ ID NO: 14, and SEQ ID NO: 15, respectively, and comprising one or more amino acid back mutations selected from the group consisting of 4L, 36F, 43S, 45K, 47W, 58V, and 71Y, and/or the heavy chain variable region comprising HCDR1, HCDR2, and HCDR3 set forth in SEQ ID NO: 10, SEQ ID NO: 11, and SEQ ID NO: 12, respectively, and comprising one or more amino acid back mutations selected from the group consisting of 28S, 30N, 49A, 75E, 76S, 93V, 94E, and 104D; or

(b) the light chain variable region comprising LCDR1, LCDR2, and LCDR3 set forth in SEQ ID NO: 19, SEQ ID NO: 20, and SEQ ID NO: 21, respectively, and comprising one or more back mutations selected from the group consisting of 36V, 44F, 46G, and 49G, and/or the heavy chain variable region comprising HCDR1, HCDR2, and HCDR3 set forth in SEQ ID NO: 16, SEQ ID NO: 17, and SEQ ID NO: 18, respectively, and comprising one or more back mutations selected from the group consisting of 44G, 49G, 27F, 48L, 67L, 71K, 78V, and 80F.

In some embodiments, the pharmaceutical composition according to any one of the above is provided, wherein the anti-CTGF antibody comprises a heavy chain variable region and a light chain variable region as described below:

(b-i) the light chain variable region comprising LCDR1, LCDR2, and LCDR3 set forth in SEQ ID NO: 74, SEQ ID NO: 75, and SEQ ID NO: 76, respectively, and comprising one or more back mutations selected from the group consisting of 45P, 46W, 48Y, 69S, and 70Y, and the heavy chain variable region comprising HCDR1, HCDR2, and HCDR3 set forth in SEQ ID NO: 71, SEQ ID NO: 72, and SEQ ID NO: 73, respectively, and comprising one or more of back mutations 27F, 38K, 48I, 67K, 68A, 70L, and 72F; or

(b-ii) the light chain variable region comprising LCDR1, LCDR2, and LCDR3 set forth in SEQ ID NO: 74, SEQ ID NO: 75, and SEQ ID NO: 76, respectively, and comprising one or more back mutations selected from the group consisting of 45P, 46W, 48Y, 69S, and 70Y, and the heavy chain variable region comprising HCDR1, HCDR2, and HCDR3 set forth in SEQ ID NO: 102, SEQ ID NO: 103, and SEQ ID NO: 104, respectively, and comprising one or more of back mutations 27F, 38K, 48I, 67K, 68A, 70L, and 72F.

In some embodiments, the pharmaceutical composition according to any one of the above is provided, wherein the anti-CTGF antibody comprises a heavy chain variable region and a light chain variable region as shown below:

(vii) the heavy chain variable region having a sequence set forth in SEQ ID NO: 6 and the light chain variable region having a sequence set forth in SEQ ID NO: 7;

(viii) the heavy chain variable region having a sequence set forth in SEQ ID NO: 27, 28, or 29 and the light chain variable region having a sequence set forth in SEQ ID NO: 22, 23, 24, 25, or 26;

(ix) the heavy chain variable region having a sequence set forth in SEQ ID NO: 8 and the light chain variable region having a sequence set forth in SEQ ID NO: 9; or (x) the heavy chain variable region having a sequence set forth in SEQ ID NO: 33, 34, 35, or 36 and the light chain variable region having a sequence set forth in SEQ ID NO: 30, 31, or 32.

In some embodiments, the pharmaceutical composition according to any one of the above is provided, wherein the anti-CTGF antibody comprises a heavy chain variable region and a light chain variable region as shown below:

(xi) the heavy chain variable region having an amino acid sequence set forth in SEQ ID NO: 69, and the light chain variable region having an amino acid sequence set forth in SEQ ID NO: 70; or

(xii) the heavy chain variable region having an amino acid sequence set forth in SEQ ID NO: 81, 82, 83, 84, or 85, and the light chain variable region having an amino acid sequence set forth in SEQ ID NO: 77, 78, 79, or 80.

In some embodiments, the pharmaceutical composition according to any one of the above is provided, wherein the anti-CTGF antibody comprises a heavy chain variable region and a light chain variable region as shown in Table 1, Table 2, and Table 3 below:

Table 1: Light and heavy chain variable region combinations of a humanized antibody derived from mab147

| | Hu147-VL1 SEQ ID NO: 22 | Hu147-VL2 SEQ ID NO: 23 | Hu147-VL3 SEQ ID NO: 24 | Hu147-VL4 SEQ ID NO: 25 | Hu147-VLS SEQ ID NO: 26 |
|---|---|---|---|---|---|
| Hu147-VH1 SEQ ID NO: 27 | Hu147-11 | Hu147-12 | Hu147-13 | Hu147-14 | Hu147-15 |
| Hu147-VH2 SEQ ID NO: 28 | Hu147-21 | Hu147-22 | Hu147-23 | Hu147-24 | Hu147-25 |
| Hu147-VH3 SEQ ID NO: 29 | Hu147-31 | Hu147-32 | Hu147-33 | Hu147-34 | Hu147-35 |

Table 2: Light and heavy chain variable region combinations of a humanized antibody of mab 164

| | Hu164-VH5 SEQ ID NO: 33 | Hu164-VH6 SEQ ID NO: 34 | Hu164-VH7 SEQ ID NO: 35 | Hu164-VH8 SEQ ID NO: 36 |
|---|---|---|---|---|
| Hu164-VL7 SEQ ID NO: 30 | Hu164-57 | Hu164-67 | Hu164-77 | Hu164-87 |
| Hu164-VL8 SEQ ID NO: 31 | Hu164-58 | Hu164-68 | Hu164-78 | Hu164-88 |
| Hu164-VL9 SEQ ID NO: 32 | Hu164-59 | Hu164-69 | Hu164-79 | Hu164-89 |

Table 3: Light and heavy chain variable region combinations of a humanized antibody of mab95

| | Hu95-VH1 SEQ ID NO: 81 | Hu95-VH2 SEQ ID NO: 82 | Hu95-VH3 SEQ ID NO: 83 | Hu95-VH4 SEQ ID NO: 84 | Hu95-VH5 SEQ ID NO: 85 |
|---|---|---|---|---|---|
| Hu95-VL1 SEQ ID NO: 77 | Hu95-11 | Hu95-21 | Hu95-31 | Hu95-41 | Hu95-51 |
| Hu95-VL2 SEQ ID NO: 78 | Hu95-12 | Hu95-22 | Hu95-32 | Hu95-42 | Hu95-52 |
| Hu95-VL3 SEQ ID NO: 79 | Hu95-13 | Hu95-23 | Hu95-33 | Hu95-43 | Hu95-53 |
| Hu95-VL4 SEQ ID NO: 80 | Hu95-14 | Hu95-24 | Hu95-34 | Hu95-44 | Hu95-54 |

In some embodiments, the pharmaceutical composition according to any one of the above is provided, wherein the anti-CTGF antibody comprises a heavy chain variable region and a light chain variable region as shown below:

(xiii) the heavy chain variable region having an amino acid sequence set forth in SEQ ID NO: 27, and the light chain variable region having an amino acid sequence set forth in SEQ ID NO: 22;
(xiv) the heavy chain variable region having an amino acid sequence set forth in SEQ ID NO: 34, and the light chain variable region having an amino acid sequence set forth in SEQ ID NO: 30; or
(xv) the heavy chain variable region having an amino acid sequence set forth in SEQ ID NO: 85, and the light chain variable region having an amino acid sequence set forth in SEQ ID NO: 77.

In some embodiments, the pharmaceutical composition according to any one of the above is provided, wherein the anti-CTGF antibody further comprises an antibody heavy chain constant region and an antibody light chain constant region; preferably, the heavy chain constant region is selected from the group consisting of constant regions of human IgG1, IgG2, IgG3 and IgG4 and conventional variants thereof, and the light chain constant region is selected from the group consisting of constant regions of human antibody κ and λ chains and conventional variants thereof; more preferably, the antibody comprises a heavy chain constant region set forth in SEQ ID NO: 37 or 38 and a light chain constant region set forth in SEQ ID NO: 39 or 40.

In some embodiments, the pharmaceutical composition according to any one of the above is provided, wherein the anti-CTGF antibody comprises:

(c) a heavy chain having at least 85%, 90%, 91%, 92%, 93%, 94%, 95%, 96%, 97%, 98%, 99%, or 100% identity to a heavy chain having a sequence set forth in SEQ ID NO: 41, 43, 44, 45, 46, 47, or 48 and a light chain having at least 85%, 90%, 91%, 92%, 93%, 94%, 95%, 96%, 97%, 98%, 99%, or 100% identity to a light chain having a sequence set forth in SEQ ID NO: 42, 49, 50, 51, 52, or 53;

(d) a heavy chain having at least 85%, 90%, 91%, 92%, 93%, 94%, 95%, 96%, 97%, 98%, 99%, or 100% identity to a heavy chain having a sequence set forth in SEQ ID NO: 54, 56, 57, 58, 59, 60, 61, 62, or 63 and a light chain having at least 85%, 90%, 91%, 92%, 93%, 94%, 95%, 96%, 97%, 98%, 99%, or 100% identity to a light chain having a sequence set forth in SEQ ID NO: 55, 64, 65, or 66;

(e) a heavy chain having a sequence set forth in SEQ ID NO: 41, 43, 44, 45, 46, 47, or 48 and a light chain having a sequence set forth in SEQ ID NO: 42, 49, 50, 51, 52, or 53; or

(f) a heavy chain having a sequence set forth in SEQ ID NO: 54, 56, 57, 58, 59, 60, 61, 62, or 63 and a light chain having a sequence set forth in SEQ ID NO: 55, 64, 65, or 66. In some embodiments, the pharmaceutical composition according to any one of the above is provided, wherein the anti-CTGF antibody comprises:

(g) a heavy chain having at least 85%, 90%, 91%, 92%, 93%, 94%, 95%, 96%, 97%, 98%, 99%, or 100% sequence identity to SEQ ID NO: 86, 88, 89, 90, 91, 92, 93, 94, 95, 96, or 97, and a light chain having at least 85%, 90%, 91%, 92%, 93%, 94%, 95%, 96%, 97%, 98%, 99%, or 100% sequence identity to SEQ ID NO: 87, 98, 99, 100, or 101; or

(h) a heavy chain set forth in SEQ ID NO: 86, 88, 89, 90, 91, 92, 93, 94, 95, 96, or 97 and a light chain set forth in SEQ ID NO: 87, 98, 99, 100, or 101.

In some embodiments, the pharmaceutical composition according to any one of the above is provided, wherein the anti-CTGF antibody comprises:

(j) a heavy chain set forth in SEQ ID NO: 46 and a light chain set forth in SEQ ID NO: 49;

(k) a heavy chain set forth in SEQ ID NO: 61 and a light chain set forth in SEQ ID NO: 64; or

(1) a heavy chain set forth in SEQ ID NO: 97 and a light chain set forth in SEQ ID NO: 98.

In some embodiments, the pharmaceutical composition according to any one of the above is provided, wherein the pharmaceutical composition further comprises a buffer. In some embodiments, the buffer is a histidine salt buffer, an acetate buffer, a citrate buffer, a succinate buffer, or a phosphate buffer. In some embodiments, the buffer is a histidine-hydrochloride buffer or a histidine-acetate buffer. In some embodiments, the buffer is a histidine-hydrochloride buffer.

In some embodiments, the pharmaceutical composition according to any one of the above is provided, wherein the pharmaceutical composition has a pH of about 5.0 to about 6.5. In some embodiments, the pharmaceutical composition has a pH of about 5.0 to about 6.0. In some embodiments, the pharmaceutical composition has a pH of about 5.0, about 5.1, about 5.2, about 5.3, about 5.4, about 5.5, about 5.6, about 5.7, about 5.8, about 5.9, about 6.0, about 6.1, about 6.2, about 6.3, about 6.4, or about 5.5. In some embodiments, the pharmaceutical composition has a pH of 5.0-6.5. In some embodiments, the pharmaceutical composition has a pH of 5.0-6.0. In some embodiments, the pharmaceutical composition has a pH of 5.5-5.7. In some embodiments, the pharmaceutical composition has a pH of 5.0, 5.1, 5.2, 5.3, 5.4, 5.5, 5.6, 5.7, 5.8, 5.9, 6.0, 6.1, 6.2, 6.3, 6.4, or 6.5, or any range between these point values.

In some embodiments, the pharmaceutical composition according to any one of the above is provided, wherein the anti-CTGF antibody is at a concentration of about 100 mg/mL to about 200 mg/mL. In some embodiments, the anti-CTGF antibody is at a concentration of about 150 mg/mL to about 200 mg/mL. In some embodiments, the anti-CTGF antibody is at a concentration of about 100 mg/mL, about 110 mg/mL, about 120 mg/mL, about 130 mg/mL, about 140 mg/mL, about 150 mg/mL, about 160 mg/mL, about 170 mg/mL, about 180 mg/mL, about 190 mg/mL, or about 200 mg/mL. In some embodiments, the anti-CTGF antibody is at a concentration of 100 mg/mL to 200 mg/mL. In some embodiments, the anti-CTGF antibody is at a concentration of 150 mg/mL to 200 mg/mL. In some embodiments, the anti-CTGF antibody is at a concentration of 100 mg/mL, 110 mg/mL, 120 mg/mL, 130 mg/mL, 140 mg/mL, 150 mg/mL, 160 mg/mL, 170 mg/mL, 180 mg/mL, 190 mg/mL, or 200 mg/mL, or any range between these point values.

In some embodiments, the pharmaceutical composition according to any one of the above is provided, wherein the pharmaceutical composition further comprises a surfactant. In some embodiments, the surfactant is a nonionic surfactant. In some embodiments, the surfactant is selected from the group consisting of polysorbate, polysorbate 20, polysorbate 80, poloxamer, Triton, sodium dodecyl sulfonate, sodium lauryl sulfonate, sodium octyl glycoside, lauryl-sulfobetaine, myristyl-sulfobetaine, linoleyl-sulfobetaine, stearyl-sulfobetaine, lauryl-sarcosine, myristyl-sarcosine, linoleyl-sarcosine, stearyl-sarcosine, linoleyl-betaine, myristyl-betaine, cetyl-betaine, lauramido propyl-betaine, cocaramide propyl-betaine, linoleinamide propyl-betaine, myristylamide propyl-betaine, palmitamide propyl-betaine, isostearamide propyl-betaine, myristylamide propyl-dimethylamine, palmitamide propyl-dimethylamine, isostearamide propyl-dimethylamine, sodium methyl cocoyl, sodium methyl oleyl taurate, polyethylene glycol, polypropylene glycol, copolymer of ethylene and propylene glycol, and the like. In some embodiments, the surfactant is a polysorbate or poloxamer. In some embodiments, the surfactant is polysorbate 80, polysorbate 20, or poloxamer 188. In some embodiments, the surfactant is polysorbate 80.

In some embodiments, the pharmaceutical composition according to any one of the above is provided, wherein the surfactant is at a concentration of about 0.05 mg/mL to about 1.0 mg/mL. In some embodiments, the surfactant is at a concentration of about 0.2 mg/mL to about 0.6 mg/mL. In some embodiments, the surfactant is at a concentration of about 0.05 mg/mL, about 0.1 mg/mL, about 0.2 mg/mL, about 0.3 mg/mL, about 0.4 mg/mL, about 0.5 mg/mL, about 0.6 mg/mL, about 0.7 mg/mL, about 0.8 mg/mL, about 0.9 mg/mL, or about 1.0 mg/mL. In some embodiments, the surfactant is at a concentration of 0.05 mg/mL to 1.0 mg/mL. In some embodiments, the surfactant is at a concentration of 0.2 mg/mL to 0.6 mg/mL. In some embodiments, the surfactant is at a concentration of 0.05 mg/mL, 0.1 mg/mL, 0.2 mg/mL, 0.3 mg/mL, 0.4 mg/mL, 0.5 mg/mL, 0.6 mg/mL, 0.7 mg/mL, 0.8 mg/mL, 0.9 mg/mL, or 1.0 mg/mL, or any range between these point values.

In some embodiments, the pharmaceutical composition according to any one of the above is provided, wherein the pharmaceutical composition further comprises a sugar. In some embodiments, the sugar is selected from the group consisting of conventional composition $(CH_2O)_n$ and derivatives thereof, including monosaccharides, disaccharides, trisaccharides, polysaccharides, sugar alcohols, reducing sugars, non-reducing sugars, and the like. The sugar may be selected from the group consisting of glucose, sucrose, trehalose, lactose, fructose, maltose, dextran, glycerin, erythritol, glycerol, arabitol, sylitol, sorbitol, mannitol, mellibiose, melezitose, raffinose, mannotriose, stachyose, maltose, lactulose, maltulose, glucitol, maltitol, lactitol, iso-maltulose, and the like. In some embodiments, the sugar is selected from one or more of sucrose, mannitol, and trehalose. In some embodiments, the sugar is sucrose.

In some embodiments, the pharmaceutical composition according to any one of the above is provided, wherein the sugar is at a concentration of about 20 mg/mL to about 100 mg/mL. In some embodiments, the sugar is at a concentration of about 40 mg/mL to about 80 mg/mL. In some embodiments, the sugar is at a concentration of about 60 mg/mL to about 80 mg/mL. In some embodiments, the sugar is at a concentration of about 20 mg/mL, about 30 mg/mL, about 40 mg/mL, about 50 mg/mL, about 60 mg/mL, about 70 mg/mL, about 80 mg/mL, about 90 mg/mL, or about 100 mg/mL. In some embodiments, the sugar is at a concentration of 20 mg/mL to 100 mg/mL. In some embodiments, the sugar is at a concentration of 40 mg/mL to 80 mg/mL. In some embodiments, the sugar is at a concentration of 60 mg/mL to 80 mg/mL. In some embodiments, the sugar is at a concentration of 20 mg/mL, 30 mg/mL, 40 mg/mL, 50 mg/mL, 60 mg/mL, 70 mg/mL, 80 mg/mL, 90 mg/mL, or 100 mg/mL, or any range between these point values.

In some embodiments, the pharmaceutical composition according to any one of the above is provided, wherein the buffer is at a concentration of about 5 mM to about 100 mM. In some embodiments, the buffer is at a concentration of about 30 mM to about 70 mM. In some embodiments, the buffer is at a concentration of about 5 mM, about 10 mM, about 20 mM, about 30 mM, about 40 mM, about 50 mM, about 60 mM, about 70 mM, about 80 mM, about 90 mM, or about 100 mM. In some embodiments, the buffer is at a concentration of 5 mM to 100 mM. In some embodiments, the buffer is at a concentration of 30 mM to 70 mM. In some embodiments, the buffer is at a concentration of 5 mM, 10 mM, 20 mM, 30 mM, 40 mM, 50 mM, 60 mM, 70 mM, 80 mM, 90 mM, or 100 mM, or any range between these point values.

In some embodiments, the pharmaceutical composition according to any one of the above is provided, wherein the pharmaceutical composition further comprises an additional stabilizer. In some embodiments, the additional stabilizer is optionally a salt, a sugar alcohol, a surfactant, a polyether, an amino acid, a chelating agent, or the like. In some embodiments, the additional stabilizer is selected from the group consisting of PEG (polyethylene glycol), arginine, and EDTA. In some embodiments, the PEG is PEG 3350 or PEG 4000. In some embodiments, the PEG is at a concentration of 10 mg/mL to 50 mg/mL. In some embodiments, the PEG is at a concentration of 10 mg/mL, 20 mg/mL, 30 mg/mL, 40 mg/mL, or 50 mg/mL. In some embodiments, the arginine is at a concentration of 10 mM to 100 mM. In some embodiments, the arginine is at a concentration of 10 mM, 20 mM, 30 mM, 40 mM, 50 mM, 60 mM, 70 mM, 80 mM, 90 mM, or 100 mM. In some embodiments, the EDTA is at a concentration of 0.5 mM to 10 mM. In some embodiments, the EDTA is at a concentration of 0.5 mM, 1 mM, 2 mM, 3 mM, 4 mM, 5 mM, 6 mM, 7 mM, 8 mM, 9 mM, or 10 mM, or any range between these point values.

In some embodiments, the pharmaceutical composition according to any one of the above comprises the following components:
(a) the anti-CTGF antibody at about 100 mg/mL to about 200 mg/mL, (b) about 0.05 mg/mL to about 1 mg/mL surfactant, (c) about 20 mg/mL to about 100 mg/mL sugar, and (d) about 5 mM to about 100 mM histidine salt buffer; the pharmaceutical composition having a pH of about 5.0 to about 6.5.

In some embodiments, the pharmaceutical composition according to any one of the above comprises the following components:
(a) the anti-CTGF antibody at about 150 mg/mL to about 200 mg/mL, (b) about 0.2 mg/mL to about 0.6 mg/mL polysorbate, (c) about 40 mg/mL to about 80 mg/mL sugar, and (d) about 30 mM to about 70 mM histidine salt buffer; the pharmaceutical composition having a pH of about 5.0 to about 6.0.

In some embodiments, the pharmaceutical composition according to any one of the above comprises the following components:
(a) the anti-CTGF antibody at about 150 mg/mL, (b) about 0.4 mg/mL polysorbate 80, (c) about 70 mg/mL sucrose, and (d) about 50 mM histidine-hydrochloride buffer; the pharmaceutical composition having a pH of about 5.5 to about 5.7.

In some embodiments, the pharmaceutical composition according to any one of the above comprises the following components:
(a) the anti-CTGF antibody at about 150 mg/mL, (b) about 0.4 mg/mL polysorbate 80, (c) about 70 mg/mL sucrose, and (d) about 50 mM histidine-hydrochloride buffer; the pharmaceutical composition having a pH of about 5.5.

In some embodiments, the pharmaceutical composition according to any one of the above comprises the following components:
(a) the anti-CTGF antibody at 150 mg/mL, (b) 0.4 mg/mL polysorbate 80, (c) 70 mg/mL sucrose, and (d) 50 mM histidine-hydrochloride buffer; the pharmaceutical composition having a pH of 5.5 to 5.7.

In some embodiments, the pharmaceutical composition according to any one of the above comprises the following components:
(a) the anti-CTGF antibody at 150 mg/mL, (b) 0.4 mg/mL polysorbate 80, (c) 70 mg/mL sucrose, and (d) 50 mM histidine-hydrochloride buffer; the pharmaceutical composition having a pH of 5.5.

In some embodiments, the pharmaceutical composition according to any one of the above comprises the following components:
(a) the anti-CTGF antibody at 200 mg/mL, (b) 0.4 mg/mL polysorbate 80, (c) 70 mg/mL sucrose, and (d) 50 mM acetate buffer; the pharmaceutical composition having a pH of 5.0-5.5.

In some embodiments, the pharmaceutical composition according to any one of the above comprises the following components:
(a) the anti-CTGF antibody at 200 mg/mL, (b) 0.4 mg/mL polysorbate 80, (c) 70 mg/mL sucrose, and (d) 50 mM histidine-acetate buffer; the pharmaceutical composition having a pH of 5.5.

In some embodiments, the pharmaceutical composition according to any one of the above comprises the following components:
(a) the anti-CTGF antibody at 200 mg/mL, (b) 0.4 mg/mL polysorbate 80, (c) 70 mg/mL sucrose, and (d) 50 mM succinate buffer; the pharmaceutical composition having a pH of 5.5.

In some embodiments, the pharmaceutical composition according to any one of the above comprises the following components:
(a) the anti-CTGF antibody at 200 mg/mL, (b) 0.4 mg/mL polysorbate 80, (c) 70 mg/mL sucrose, and (d) 50 mM citrate buffer; the pharmaceutical composition having a pH of 5.5.

In some embodiments, the pharmaceutical composition according to any one of the above comprises the following components:
(a) the anti-CTGF antibody at 200 mg/mL, (b) 0.4 mg/mL polysorbate 80, (c) 70 mg/mL sucrose, and (d) 50 mM histidine-hydrochloride buffer; the pharmaceutical composition having a pH of 5.5-6.5. In some embodiments, the pharmaceutical composition has a pH of 5.5, 6.0, or 6.5.

In some embodiments, the pharmaceutical composition according to any one of the above comprises the following components:
(a) the anti-CTGF antibody at 200 mg/mL, (b) 0.4 mg/mL polysorbate 80, (c) 70 mg/mL sucrose, and (d) 50 mM phosphate buffer; the pharmaceutical composition having a pH of 6.0-6.5.

In some embodiments, the pharmaceutical composition according to any one of the above comprises the following components:
(a) the anti-CTGF antibody at 150 mg/mL, (b) 0.4 mg/mL polysorbate 80, (c) 70 mg/mL sucrose, and (d) 50 mM histidine-hydrochloride buffer; the pharmaceutical composition having a pH of 6.0.

In some embodiments, the pharmaceutical composition according to any one of the above comprises the following components:
(a) the anti-CTGF antibody at 150 mg/mL, (b) 0.6 mg/mL polysorbate 20, (c) 70 mg/mL sucrose, and (d) 50 mM histidine-hydrochloride buffer; the pharmaceutical composition having a pH of 5.5.

In some embodiments, the pharmaceutical composition according to any one of the above comprises the following components:
(a) the anti-CTGF antibody at 150 mg/mL, (b) 0.2 mg/mL polysorbate 80, (c) 70 mg/mL sucrose, and (d) 50 mM histidine-hydrochloride buffer; the pharmaceutical composition having a pH of 5.5.

In some embodiments, the pharmaceutical composition according to any one of the above comprises the following components:
(a) the anti-CTGF antibody at 150 mg/mL, (b) 0.6 mg/mL polysorbate 80, (c) 70 mg/mL sucrose, and (d) 50 mM histidine-hydrochloride buffer; the pharmaceutical composition having a pH of 5.5.

In some embodiments, the pharmaceutical composition according to any one of the above comprises the following components:
(a) the anti-CTGF antibody at 150 mg/mL, (b) 0.4 mg/mL poloxamer 188, (c) 70 mg/mL sucrose, and (d) 50 mM histidine-hydrochloride buffer; the pharmaceutical composition having a pH of 5.5.

In some embodiments, the pharmaceutical composition according to any one of the above comprises the following components:
(a) the anti-CTGF antibody at 150 mg/mL, (b) 0.4 mg/mL poloxamer 188, (c) 70 mg/mL sucrose, and (d) 50 mM histidine-hydrochloride buffer; the pharmaceutical composition having a pH of 5.5.

In some embodiments, the pharmaceutical composition according to any one of the above comprises the following components:
(a) the anti-CTGF antibody at 150 mg/mL, (b) 0.4 mg/mL polysorbate 80, (c) 70 mg/mL sucrose, (d) 50 mM histidine-hydrochloride buffer, and (e) 2% PEG 3350; the pharmaceutical composition having a pH of 5.5.

In some embodiments, the pharmaceutical composition according to any one of the above comprises the following components:
(a) the anti-CTGF antibody at 150 mg/mL, (b) 0.4 mg/mL polysorbate 80, (c) 70 mg/mL sucrose, (d) 50 mM histidine-hydrochloride buffer, and (e) 50 mM arginine; the pharmaceutical composition having a pH of 5.5.

In some embodiments, the pharmaceutical composition according to any one of the above comprises the following components:
(a) the anti-CTGF antibody at 150 mg/mL, (b) 0.4 mg/mL polysorbate 80, (c) 70 mg/mL sucrose, (d) 50 mM histidine-hydrochloride buffer, and (e) 1% mannitol; the pharmaceutical composition having a pH of 5.5.

In some embodiments, the pharmaceutical composition according to any one of the above comprises the following components:
(a) the anti-CTGF antibody at 150 mg/mL, (b) 0.4 mg/mL polysorbate 80, (c) 70 mg/mL sucrose, (d) 50 mM histidine-hydrochloride buffer, and (e) 0.5-10 mM EDTA; the pharmaceutical composition having a pH of 5.5. In some embodiments, the EDTA is at a concentration of 0.5 mM, 5 mM, or 10 mM.

In some embodiments, the pharmaceutical composition according to any one of the above comprises the following components:
(a) the anti-CTGF antibody at 150 mg/mL, (b) 0.4 mg/mL polysorbate 80, (c) 70 mg/mL trehalose, and (d) 50 mM histidine-hydrochloride buffer; the pharmaceutical composition having a pH of 5.5.

In some embodiments, the pharmaceutical composition according to any one of the above is provided, wherein the pharmaceutical composition is a liquid formulation. In some embodiments, the liquid formulation contains water as solvent.

The present disclosure further provides a lyophilized formulation, wherein the lyophilized formulation can form, upon reconstitution, the pharmaceutical composition according to any one of the above.

The present disclosure further provides a method for preparing a lyophilized formulation, which comprises a step of lyophilizing the pharmaceutical composition according to any one of the above.

The present disclosure further provides a lyophilized formulation, which is obtained by lyophilizing the pharmaceutical composition according to any one of the above. In some embodiments, the lyophilization according to any one of the above comprises steps of pre-freezing, primary drying, and secondary drying in sequence. In some embodiments, the lyophilized formulation is stable at 40 °C for at least 7 days, at least 14 days, or at least 28 days.

The present disclosure further provides a reconstituted solution, wherein the reconstituted solution is prepared by reconstituting the lyophilized formulation according to any one of the above; optionally, the reconstituted solution is obtained by reconstituting the lyophilized formulation according to any one of the above in a physiologically acceptable solvent, including but not limited to water for injection, normal saline, and buffers.

In some embodiments, the reconstituted solution described above comprises:
(a) the anti-CTGF antibody at about 150 mg/mL to about 200 mg/mL, (b) about 0.2 mg/mL to about 0.6 mg/mL polysorbate, (c) about 40 mg/mL to about 80 mg/mL sugar, and (d) about 30 mM to about 70 mM histidine salt buffer; the pharmaceutical composition having a pH of about 5.0 to about 6.0.

In some embodiments, the reconstituted solution described above comprises:
(a) the anti-CTGF antibody at about 150 mg/mL, (b) about 0.4 mg/mL polysorbate 80, (c) about 70 mg/mL sucrose, and (d) about 50 mM histidine-hydrochloride buffer; the pharmaceutical composition having a pH of about 5.5 to about 5.7.

In some embodiments, the reconstituted solution described above comprises:

(a) the anti-CTGF antibody at about 150 mg/mL, (b) about 0.4 mg/mL polysorbate 80, (c) about 70 mg/mL sucrose, and (d) about 50 mM histidine-hydrochloride buffer; the pharmaceutical composition having a pH of about 5.5.

In some embodiments, the pharmaceutical composition or reconstituted solution according to any one of the above is a formulation for intravenous, subcutaneous, intraperitoneal, or intramuscular injection; in some embodiments, the pharmaceutical composition or reconstituted solution according to any one of the above is a formulation for intravenous injection. In some embodiments, the pharmaceutical composition or reconstituted solution according to any one of the above is suitable for intravenous, subcutaneous, intraperitoneal, or intramuscular injection. In some embodiments, the pharmaceutical composition or reconstituted solution or lyophilized formulation according to any one of the above is for use in the preparation of a medicament for intravenous, subcutaneous, intraperitoneal, or intramuscular injection.

The present disclosure further provides an article of manufacture, which comprises a container containing the pharmaceutical composition according to any one of the above, the lyophilized formulation according to any one of the above, or the reconstituted solution according to any one of the above.

In some embodiments, the present disclosure further provides use of the pharmaceutical composition according to any one of the above, the lyophilized formulation according to any one of the above, the reconstituted solution according to any one of the above, or the article of manufacture according to any one of the above in the preparation of a medicament for treating a CTGF-associated disease.

The present disclosure further provides a method for treating or preventing a CTGF-associated disease, which comprises administering to a patient an effective amount of the pharmaceutical composition according to any one of the above, the lyophilized formulation according to any one of the above, the reconstituted solution according to any one of the above, or the article of manufacture according to any one of the above.

In some embodiments, the present disclosure further provides the pharmaceutical composition according to any one of the above, the lyophilized formulation according to any one of the above, the reconstituted solution according to any one of the above, or the article of manufacture according to any one of the above for use in the treatment of a CTGF-associated disease.

In some embodiments, the CTGF-associated disease is a fibrotic disease, hypertension, diabetes, myocardial infarction, arthritis, a CTGF-associated disease of cellular proliferation, atherosclerosis, glaucoma, or cancer. In some embodiments, the fibrotic disease is selected from the group consisting of: idiopathic pulmonary fibrosis, diabetic nephropathy, diabetic retinopathy, osteoarthritis, scleroderma, chronic heart failure, liver cirrhosis, and renal fibrosis. In some embodiments, the cancer is selected from the group consisting of: acute lymphoblastic leukemia, dermatofibroma, breast cancer, angiolipoma, angioleiomyoma, desmoplastic cancer, prostate cancer, ovarian cancer, colorectal cancer, pancreatic cancer, gastrointestinal cancer, and liver cancer.

## BRIEF DESCRIPTION OF THE DRAWINGS

FIG. 1: the affinity of a humanized antibody derived from mab164 for human CTGF, as measured by ELISA.

FIG. 2: the experimental results of the inhibition of SU86.86 xenograft tumors in mice by a humanized antibody derived from mab147 or mab164.

## DETAILED DESCRIPTION

### Terms

In order to facilitate the understanding of the present disclosure, some technical and scientific terms are specifically defined below. Unless otherwise specifically defined herein, all other technical and scientific terms used herein have the meanings generally understood by those of ordinary skill in the art to which the present disclosure belongs. The three-letter and single-letter codes for amino acids used herein are as described in *J. biol. chem,* 243, p3558 (1968).

Connective tissue growth factor (CTGF) is a 36 kD, cysteine-rich, heparin-binding, secreted glycoprotein originally isolated from human umbilical vein endothelial cells. CTGF belongs to the CCN (CTGF, Cyr61, Nov) family of proteins (secreted glycoproteins), which includes the serum-induced immediate early gene product Cyr61, the putative oncogene Nov, the ECM-associated protein FISP-12, the src-induced gene CEF-10, the Wnt-induced secreted protein WISP-3, and the anti-proliferative protein HICP/rCOP. CCN proteins are characterized by 38 conserved cysteine residues that make up more than 10% of the total amino acid content and form a modular structure with N-terminal and C-terminal domains. The modular structure of CTGF includes conserved motifs for insulin-like growth factor binding protein (IGF-BP) and von Willebrand factor (VWC) in the N-terminal domain, and thrombospondin (TSP1) and a cysteine-knot motif in the C-terminal domain. The CTGF of the present disclosure includes wild-type proteins of any origin or variants that retain the function thereof. The term "antibody" of the present disclosure is used in its broadest sense and encompasses a variety of antibody structures, including but not limited to monoclonal antibodies, polyclonal antibodies, multispecific antibodies (e.g., bispecific antibodies), full-length antibodies or antigen-binding fragments thereof (also known as "antigen-binding moieties"), murine antibodies, chimeric antibodies or humanized antibodies, or affinity-matured antibodies, so long as they exhibit the desired antigen-binding activity. Native antibodies refer to naturally-occurring immunoglobulin molecules. For example, native IgG antibodies are heterotetrameric glycoproteins of about 150,000 Daltons composed of two identical light chains and two identical heavy chains that are disulfide-bonded. From N- to C-terminus, each heavy chain has a variable region (VH), also known as variable heavy domain or heavy chain variable domain, followed by three constant domains (CH1, CH2, and CH3). Similarly, from N-to C-terminus, each light chain has a variable region (VL), also known as variable light domain or light chain variable domain, followed by a constant light (CL) domain. "Full-length antibody", "intact antibody", and "whole antibody" are used herein interchangeably to refer to an antibody having a structure substantially similar to a native antibody structure or having heavy chains that comprise an Fc region as defined herein. In some embodiments, the full-length antibodies of the present disclosure include full-length antibodies formed by linking a light chain variable region and a heavy chain variable region from the light and heavy chain variable region combinations in Table 1, Table 2, and Table 3 to a light chain constant region and a heavy chain constant region, respectively. Those skilled in the art can select different antibody-derived light chain constant regions and heavy chain constant regions according to actual needs, for example, human antibody-derived light chain constant regions and heavy chain constant regions.

"Variable region" or "variable domain" refers to a domain in the heavy or light chain of an antibody that is involved in the binding of the antibody to an antigen. Each VH and VL comprises four conserved framework regions (FRs) and three complementarity-determining regions (CDRs). The term "complementarity-determining region" or "CDR" refers to the regions within a variable domain that primarily contribute to antigen binding; "framework" or "FR" refers to variable domain residues other than CDR residues. A VH comprises 3 CDR regions: HCDR1, HCDR2, and HCDR3; a VL comprises 3 CDR regions: LCDR1, LCDR2, and LCDR3. Each VH and VL consists of three CDRs and four FRs arranged from amino-terminus to carboxy-terminus in the following order: FR1, CDR1, FR2, CDR2, FR3, CDR3, and FR4. A single VH or VL may be sufficient to confer antigen-binding specificity.

The boundaries of the amino acid sequences of CDRs can be determined using a variety of well-known schemes, such as the "Kabat" numbering scheme (see Kabat et al., Sequences of Proteins of Immunological Interest, 5th edition, Public Health Service, National Institutes of Health, Bethesda, MD, 1991), the "Chothia" numbering scheme, the "AbM" numbering scheme, the "contact" numbering scheme (see Martin, ACR. Protein Sequence and Structure Analysis of Antibody Variable Domains[J]. 2001), and the ImMunoGenTics (IMGT) numbering scheme (see Lefranc M.P., Dev. Comp. Immunol., 27, 55-77(2003)). The relationships between the numbering schemes including, for example, the Kabat numbering scheme and the IMGT numbering scheme, are well known to those skilled in the art and are shown in Table 4 below.

Table 4. The relationships between the numbering schemes for CDRs

|  | IMGT | Kabat | AbM | Chothia | Contact |
|---|---|---|---|---|---|
| HCDR1 | 27-38 | 31-35 | 26-35 | 26-32 | 30-35 |
| HCDR2 | 50-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 50-65 | 50-56 | 50-56 | 50-56 | 46-55 |
| LCDR3 | 105-117 | 89-97 | 89-97 | 89-97 | 89-96 |

The "class" of an antibody refers to the type of the constant region its heavy chains contain. According to the amino acid sequences of the constant regions, the light chains of antibodies fall into two categories: kappa (x) and lambda ($\lambda$). According to differences in the amino acid composition and the order of arrangement of the heavy chain constant regions of antibodies, antibodies can be divided into five classes, otherwise called antibody isotypes, namely IgM, IgD, IgG, IgA, and IgE, the corresponding heavy chains of which are $\mu$ chain, $\delta$ chain, $\gamma$ chain, $\alpha$ chain, and $\epsilon$ chain, respectively. Ig of the same class can be divided into different subclasses according to differences in the amino acid composition of the hinge regions and the number and positions of disulfide bonds of the heavy chains; for example, IgG can be divided into $IgG_1$, $IgG_2$, $IgG_3$, and $IgG_4$. Each of the five classes of Ig may have a $\kappa$ chain or $\lambda$ chain. The "conventional variant" of the human antibody heavy chain constant region and the human antibody light chain constant region described herein refers to a variant of the heavy chain constant region or light chain constant region derived from humans that has been disclosed in the prior art and does not change the structure and function of the antibody variable region. Exemplary variants include $IgG_1$, $IgG_2$, $IgG_3$, and $IgG_4$ heavy chain constant region variants with site-directed modifications and amino acid substitutions in the heavy chain constant region. In some embodiments, the substitutions are YTE mutations (M252Y/S254T/T256E), the L234A mutation and/or the L235A mutation, the S228P mutation, and/or mutations that give rise to knob-into-hole structures. These mutations have been shown to confer new properties on antibodies without altering the function of the variable regions of the antibodies. In some embodiments, the antibody is of the $IgG_1$ isotype, with the P329, P234, and P235 mutations in the hinge region to reduce effector function. In some embodiments, the antibody is of the $IgG_2$ isotype. In some embodiments, the antibody is of the $IgG_4$ isotype, with the S228P mutation in the hinge region to improve the stability of the $IgG_4$ antibody.

"Antibody fragment" refers to a molecule different from an intact antibody; it comprises a portion of an intact antibody, and the portion binds to an antigen to which the intact antibody binds. Examples of antibody fragments include, but are not limited to, Fv, Fab, Fab', Fab'-SH, $F(ab')_2$, single-domain antibodies, diabodies, linear antibodies, single-chain antibody molecules (e.g., scFv), and multispecific antibodies formed from antibody fragments.

"Fc region" or "fragment crystallizable region" is used to define the C-terminal region of the heavy chain of an antibody, including native sequence Fc regions and variant Fc regions. In some embodiments, the Fc region of the human IgG heavy chain is defined as extending from the amino acid residue at position Cys226 or from Pro230 to its carboxy-terminus. The boundaries of the Fc region of the heavy chain of an antibody may also be varied, for example, by deleting the C-terminal lysine of the Fc region (residue 447 according to the EU numbering scheme) or deleting the C-terminal glycine and lysine of the Fc region (residues 446 and 447 according to the EU numbering scheme). Accordingly, in some embodiments, a composition of intact antibodies may comprise antibody populations with all K447 residues and/or G446 + K447 residues removed. In some embodiments, a composition of intact antibodies may comprise antibody populations without K447 residues and/or G446 + K447 residues removed. In some embodiments, a composition of intact antibodies comprises antibody populations having a mixture of antibodies with and without K447 residues and/or G446 + K447 residues. Suitable native sequence Fc regions for the antibodies described herein include human IgG1, IgG2 (IgG2A, IgG2B), IgG3, and IgG4. Unless otherwise specified herein, the numbering of amino acid residues in the Fc region or constant region conforms the EU numbering scheme, also known as the EU index, as described in Kabat et al., Sequences of Proteins of Immunological Interest, 5th edition, Public Health Service, National Institutes of Health, Bethesda, MD, 1991.

"Murine antibody" in the present disclosure is a monoclonal antibody against human CTGF which is derived from mice and is prepared according to the knowledge and skill in the art. During the preparation, a test subject is injected with a CTGF antigen, and then hybridoma of antibodies expressing the desired sequence or functional properties is isolated. In one preferred embodiment of the present disclosure, the murine anti-CTGF antibody or the antigen-binding fragment thereof may further comprise the light chain constant region of a murine κ or λ chain or a variant thereof, or further comprise the heavy chain constant region of murine $IgG_1$, $IgG_2$, or $IgG_3$, or a variant thereof.

The term "chimeric antibody" refers to an antibody obtained by fusing a variable region of a murine antibody and a constant region of a human antibody, which can reduce an immune response induced by the murine antibody. The chimeric antibody is established by firstly establishing hybridoma secreting murine specific monoclonal antibody, then cloning a variable region gene from the mouse hybridoma cells, cloning a constant region gene of human antibody as required, connecting the mouse variable region gene and the human constant region gene into a chimeric gene, inserting the chimeric gene into an expression vector, and finally expressing chimeric antibody molecules in a eukaryotic system or prokaryotic system. In a preferred embodiment of the present disclosure, the antibody light chain of the chimeric antibody further comprises the light chain constant region of a human κ or λ chain or a variant thereof. The antibody heavy chain of the CTGF chimeric antibody further comprises the heavy chain constant region of human IgG1, IgG2, IgG3, or IgG4, or a variant thereof, preferably the heavy chain constant region of human IgG1, IgG2, or IgG4, or an IgG1, IgG2 or IgG4 variant using an amino acid mutation (e.g., an L234A and/or L235A mutation, and/or an S228P mutation).

"Humanized antibody", also known as a CDR-grafted antibody, refers to an antibody produced by grafting murine CDR sequences into a human antibody variable region framework, i.e., a different type of antibody framework sequence. Such an antibody can overcome the heterogeneous reaction induced by the chimeric antibody because of carrying a large amount of mouse protein component. Such framework sequences can be obtained from public DNA databases or published references that include germline antibody gene sequences. For example, germline DNA sequences of genes of the human heavy and light chain variable regions can be found in the "VBase" human species sequence database, as well as in Kabat, E. A. et al., 1991 Sequences of Proteins of Immunological Interest, 5th edition. To avoid the decrease in activity caused by the decrease in immunogenicity, the framework sequence in human antibody variable region can be subjected to minimum reverse mutation or back mutation to maintain or enhance activity. The humanized antibody of the present disclosure also includes humanized antibodies that were further subjected to CDR affinity maturation mutation by yeast display.

"Affinity" refers to the overall strength of a non-covalent interaction between a single binding site of a molecule (e.g., an antibody) and its binding partner (e.g., an antigen). Unless otherwise indicated, as used herein, "binding affinity" refers to an internal binding affinity that reflects a 1:1 interaction between members of a binding pair (e.g., an antibody and an antigen). The affinity of a molecule X for its ligand Y can be generally expressed by the dissociation constant (KD). Affinity can be determined by conventional methods known in the art, including those described herein. Specific illustrative and exemplary examples for measuring binding affinity are described below. "kassoc" or "ka" refers to the association rate of a particular antibody-antigen interaction, while the term "kdis" or "kd" as used herein is intended to refer to the dissociation rate of a particular antibody-antigen interaction. As used herein, the term "KD" refers to the dissociation constant, which is obtained from the ratio of kd to ka (i.e., kd/ka) and expressed as a molar concentration (M). The KD value of an antibody can be determined using methods well known in the art. Methods for determining antibody KD include measuring surface plasmon resonance using a biosensing system, such as a system, or measuring affinity in solution by solution equilibrium titration (SET) assay.

An "affinity-matured" antibody is one with one or more alterations in one or more CDRs thereof which result in an improvement in the affinity of the antibody for antigens, compared to the parent antibody which does not contain these alterations. Preferably, in some embodiments, an affinity-matured antibody has nanomolar or even picomolar affinity for the target antigen. Affinity-matured antibodies can be produced using procedures known in the art.

"Amino acid mutation" encompasses amino acid substitutions, deletions, insertions, and modifications. Any combination of substitutions, deletions, insertions, and modifications can be made to arrive at the final construct, provided that the final construct possesses the desired properties. Amino acid sequence deletions and insertions include amino-terminal and/or carboxyl-terminal deletions and amino acid insertions. Particular amino acid mutations may be amino acid substitutions. In one embodiment, amino acid mutations are non-conservative amino acid substitutions-that is, one amino acid is replaced with another amino acid having different structural and/or chemical properties. Amino acid substitutions include replacement with non-naturally occurring amino acids or with derivatives of the 20 native amino acids (e.g., 4-hydroxyproline, 3-methylhistidine, ornithine, homoserine, and 5-hydroxylysine). Amino acid mutations can be generated using genetic or chemical methods well known in the art. Genetic methods may include site-directed mutagenesis, PCR, gene synthesis, and the like. It is contemplated that methods for altering amino acid side chain groups other than genetic engineering, such as chemical modification, may also be used. Various names may be used herein to indicate the same amino acid mutation.

"About" described in the disclosure means that it is within an acceptable error range for a particular value as determined by one of ordinary skill in the art, which will depend in part on how the value is measured or determined, i.e., the limitations of the measurement system. In the context of a particular assay, result or embodiment, "about" means a given value $\pm$ a range within 5%, unless otherwise explicitly stated in the example or elsewhere in the specification.

"Buffer" refers to a buffer that resists changes in pH by the action of its acid-base conjugate components. Examples of buffers that control the pH in an appropriate range include acetate, succinate, gluconate, histidine salt, oxalate, lactate, phosphate, citrate, tartrate, fumarate, glycylglycine, and other organic acid buffers.

"Histidine salt buffer" is a buffer comprising histidine ions. Examples of histidine salt buffers include histidine-hydrochloride buffer, histidine-acetate buffer, histidine-phosphate buffer, histidine-sulfate buffer, and the like, and the histidine-hydrochloride buffer or histidine-acetate buffer is preferred. The histidine-acetate buffer is prepared with histidine and acetic acid, and the histidine hydrochloride buffer is prepared with histidine and histidine hydrochloride, or histidine and hydrochloric acid.

"Citrate buffer" is a buffer comprising citrate ions. Examples of citrate buffers include citric acid-sodium citrate, citric acid-potassium citrate, citric acid-calcium citrate, citric acid-magnesium citrate, and the like. The preferred citrate buffer is citric acid-sodium citrate.

"Succinate buffer" is a buffer comprising succinate ions. Examples of succinate buffers include succinic acid-sodium succinate, succinic acid-potassium succinate, succinic acid-calcium succinate, and the like. The preferred succinate buffer is succinic acid-sodium succinate. Illustratively, the succinic acid-sodium succinate may be prepared with succinic acid and sodium hydroxide, or succinic acid and sodium succinate.

"Phosphate buffer" is a buffer comprising phosphate ions. Examples of phosphate buffers include disodium hydrogen phosphate-sodium dihydrogen phosphate, disodium hydrogen phosphate-potassium dihydrogen phosphate, disodium hydrogen phosphate-citric acid, and the like. The preferred phosphate buffer is disodium hydrogen phosphate-sodium dihydrogen phosphate.

"Acetate buffer" is a buffer comprising acetate ions. Examples of acetate buffers include acetic acid-sodium acetate, acetic acid histidine salt, acetic acid-potassium acetate, acetic acid-calcium acetate, acetic acid-magnesium acetate, and the like. The preferred acetate buffer is acetic acid-sodium acetate.

"Stabilizer" refers to a component that helps maintain the structural integrity of a biopharmaceutical drug, particularly during freezing and/or lyophilization and/or storage (particularly when exposed to stress). This stabilizing effect may arise for a variety of reasons, and typically, such stabilizers may act as osmotic agents which reduce protein denaturation. The additional stabilizer herein refers to a stabilizer other than buffer systems, surfactants, and sugar alcohols, for example, a stabilizer selected from the group consisting of PEG, arginine, and EDTA.

"Pharmaceutical composition" refers to a mixture comprising one or more of the antibodies described herein and other chemical components, for example, physiological/pharmaceutically acceptable carriers and excipients. The purpose of the pharmaceutical composition is to maintain the stability of the active ingredient and promote the administration to an organism, which facilitates the absorption of the active ingredient, thereby exerting biological activity.

As used herein, "pharmaceutical composition" and "formulation" are not mutually exclusive.

Unless otherwise stated, the solvent in the pharmaceutical composition described herein in solution form is water.

"Lyophilized formulation" refers to a formulation or a pharmaceutical composition obtained by freeze-drying a pharmaceutical composition in liquid or solution form or a liquid or solution formulation in vacuum.

While the present disclosure provides content ranges or values, those of ordinary skill in the art will appreciate that the content ranges or values encompass acceptable error ranges for the particular values determined.

The pharmaceutical composition described herein can achieve an effect of being stable: a pharmaceutical composition in which the antibody substantially retains its physical and/or chemical stability and/or biological activity after storage; preferably, the pharmaceutical composition substantially retains its physical and chemical stability as well as its biological activity after storage. The storage period is generally selected based on a predetermined shelf life of the pharmaceutical composition. There are a variety of analytical techniques currently available for determining protein stability, and the stability after storage for a selected period of time at a selected temperature can be determined.

A stable formulation is one in which no significant change is observed under the following conditions: stored at refrigeration temperature (2-8 °C) for at least 3 months, preferably 6 months, more preferably 1 year, and even more preferably up to 2 years. In addition, stable liquid formulations include liquid formulations that exhibit desirable features after storage at temperatures including 25 °C for periods including 1 month, 3 months, and 6 months. Typical examples for stability are as follows: typically, no more than about 10%, preferably no more than about 5%, of antibody monomers aggregate or are degraded as measured by SEC-HPLC. The formulation is a pale yellow, nearly colorless and clear liquid, or a colorless and clear liquid, or is clear to slightly opalescent, by visual analysis. The concentration, pH, and osmolality of the formulation have no more than ±10% change. Typically, no more than about 10%, preferably no more than about 5%, of decrease is observed. Typically, no more than about 10%, preferably no more than about 5%, of aggregation is formed.

An antibody "retains its physical stability" in a pharmaceutical formulation if it shows no significant increase in aggregation, precipitation and/or denaturation upon visual inspection of color and/or clarity, or as determined by UV light scattering, size exclusion chromatography (SEC), and dynamic light scattering (DLS). Changes in protein conformation can be assessed by fluorescence spectroscopy (which determines the protein tertiary structure) and by FTIR spectroscopy (which determines the protein secondary structure).

An antibody "retains its chemical stability" in a pharmaceutical formulation if it shows no significant chemical change. Chemical stability can be assessed by detecting and quantifying chemically changed forms of the protein. Degradation processes that often change the chemical structure of proteins include hydrolysis or clipping (evaluated by methods such as size exclusion chromatography and CE-SDS), oxidation (evaluated by methods such as peptide mapping in conjunction with mass spectroscopy or MALDI/TOF/MS), deamidation (evaluated by methods such as ion-exchange chromatography, capillary isoelectric focusing, peptide mapping, and isoaspartic acid measurement), and isomerization (evaluated by measuring the isoaspartic acid content, peptide mapping, etc.).

An antibody "retains its biological activity" in a pharmaceutical formulation if the biological activity of the antibody at a given time is within a predetermined range of the biological activity exhibited during the preparation of the pharmaceutical formulation. "Administrating", "giving", and "treating", when applied to animals, humans, experimental subjects, cells, tissues, organs or biological fluids, refer to contact of an exogenous drug, a therapeutic agent, a diagnostic agent or a composition with the animals, humans, subjects, cells, tissues, organs or biological fluids. "Administering", "giving", and "treating" can refer to, for example, therapeutic, pharmacokinetic, diagnostic, research, and experimental methods. The treatment of cells comprises making the reagent in contact with the cells and making the reagent in contact with fluid, where the fluid is in contact with the cells. "Administering", "giving", and "treating" also refer to treating, for example, cells by reagents, diagnosis, binding compositions or by another cell *in vitro* and *ex vivo*. "Treating", when applied to humans, veterinary, or research subjects, refers to therapeutic treatment, preventive or prophylactic measures, and research and diagnostic applications.

"Treatment" refers to administering a therapeutic agent, for example, comprising any one of the pharmaceutical compositions of the present disclosure, either internally or externally to a patient with one or more symptoms of a disease on which the therapeutic agent is known to have a therapeutic effect. Generally, the therapeutic agent is administered in an amount effective to alleviate one or more symptoms of the disease in the patient or population being treated to induce regression of such symptoms or inhibit the development of such symptoms to any clinically measurable degree. The amount of therapeutic agent effective to alleviate the symptoms of any particular disease (also known as a "therapeutically effective amount") may vary depending on a variety of factors, such as the disease state, age, and weight of the patient, and the ability of the drug to produce a desired therapeutic effect in the patient. Whether a symptom of a disease has been alleviated can be evaluated by any clinical testing methods commonly used by doctors or other health care professionals to evaluate the severity or progression of the symptom. Although the embodiments of the present disclosure (for example, treatment methods or articles of manufacture) may not be effective in alleviating the symptoms of each disease of interest, they shall reduce the symptoms of a disease of interest in a statistically significant number of patients, as determined according to any statistical testing methods known in the art, such as Student t-test, chi-square test, Mann and Whitney's U test, Kruskal-Wallis test (H test), Jonckheere-Terpstra test, and Wilcoxon test.

There is no limitation for a CTGF-associated disease in the present disclosure, as long as it is a disease associated with CTGF. For example, the therapeutic response induced by the molecule of the present disclosure can be achieved by binding human CTGF and then blocking the binding of CTGF to its receptors/ligands, or killing tumor cells over-expressing CTGF. Thus, the molecules of the present disclosure are very useful, when in preparations and formulations suitable for therapeutic applications, for people who have tumors or cancer, optionally including lung fibrosis, kidney fibrosis, tumor formation and growth, glaucoma, diseases of cell proliferation, cataracts, choroidal neovascularization, retinal detachment, proliferative vitreoretinopathy, macular degeneration, diabetic retinopathy, corneal scarring and corneal haze, cysts, reduced vascular calcification, pancreatic ductal adenocarcinoma, pancreatic cancer, melanoma, radiation-induced fibrosis (RIF), idiopathic pulmonary fibrosis, and pulmonary remodeling diseases selected from the group consisting of asthma, chronic bronchitis, chronic obstructive pulmonary disease (COPD), cystic fibrosis, emphysema, and the like, preferably pancreatic cancer, pulmonary fibrosis, and kidney fibrosis.

The details of one or more embodiments of the present disclosure are set forth in the specification above. Although any methods and materials similar or identical to those described herein can be used in the practice or testing of the present disclosure, the preferred methods and materials are described below. Other features, objects, and advantages of the present disclosure will be apparent from the specification and the claims. In the specification and claims, singular forms include plural referents unless otherwise indicated clearly in the context. Unless otherwise defined, all technical and scientific terms used herein have the meanings generally understood by those of ordinary skill in the art to which the present disclosure belongs. All the patents and publications cited in the specification are incorporated by reference. The following examples are set forth in order to more fully illustrate the preferred embodiments of the present disclosure. These examples should not be construed in any way as limiting the scope of the present disclosure, which is defined by the claims.

## Examples

The present disclosure is further described below with reference to examples, which, however, are not intended to limit the scope of the present disclosure. The experimental methods in the examples of the present disclosure in which specific conditions are not specified are generally performed under conventional conditions, for example, by referring to Antibodies: A Laboratory Manual and Molecular Cloning: A Laboratory Manual by Cold Spring Harbor Laboratory, or under conditions recommended by the manufacturer of the raw material or the goods. Reagents without specific origins indicated were commercially available conventional reagents.

### I. Antibodies

### Example 1-1. Preparation of CTGF Antigen and Antibodies

### I. Antigen design and expression

With human CTGF protein (Genbank No. NM_001901.2) as a template, the amino acid sequences of the antigen and the protein for assays were designed; optionally, the CTGF protein or fragments were fused with different tags. The sequences were cloned onto pTT5 vectors or pXC vectors and transiently expressed in 293 cells or stably expressed in LONZA CHO cells to obtain the encoded antigen and protein for assays of the present disclosure. The following CTGF antigens are all human CTGF unless otherwise specified.

Human CTGF full-length protein (for immunization), SEQ ID NO: 1)

QNCSGPCRCPDEPAPRCPAGVSLVLDGCGCCRVCAKQLGELCTERDPCDPHKGL
FCDFGSPANRKIGVCTAKDGAPCIFGGTVYRSGESFQSSCKYQCTCLDGAVGCM
PLCSMDVRLPSPDCPFRRVKLPGKCCEEWVCDEPKDQTVVGPALAAYRLEDTF
GPDPTMIRANCLVQTTEWSACSKTCGMGISTRVTNDNASCRLEKQSRLCMVRP
CEADLEENIKKGKKCIRTPKISKPIKFELSGCTSMKTYRAKFCGVCTDGRCCTPH
RTTTLPVEFKCPDGEVMKKNMMFIKTCACHYNCPGDNDIFESLYYRKMYGDM
A

CTGF-his (a fusion protein of the human CTGF mature protein and a his tag), can be used for assays, SEQ ID NO: 2

MEFGLSWLFLVAILKGVQC*QNCSGPCRCPDEPAPRCPAGVSLVLDGCGCCRVCAK*
*QLGELCTERDPCDPHKGLFCDFGSPANRKIGVCTAKDGAPCIFGGTVYRSGESFQSS*
*CKYQCTCLDGAVGCMPLCSMDVRLPSPDCPFRRVKLPGKCCEEWVCDEPKDQT*
*VVGPALAAYRLEDTFGPDPTMIRANCLVQTTEWSACSKTCGMGISTRVTNDNASCRL*
*EKQSRLCMVRPCEADLEENIKKGKKCIRTPKISKPIKFELSGCTSMKTYRAKFCGVCT*
*DGRCCTPHRTTTLPVEFKCPDGEVMKKNMMFIKTCACHYNCPGDNDIFESLYYRK*
*MYGDMA*HHHHHH

(Note: the signal peptide is single-underlined, the human CTGF coding region is italicized, and the his tag is double-underlined.)
mCTGF-mFc (a fusion protein of the mouse CTGF coding region and a mouse Fc tag), can be used for assays, SEQ ID NO: 3

MEFGLSWLFLVAILKGVQC*QDCSAQCQCAAEAAPHCPAGVSLVLDGCGCCRVCAK*
*QLGELCTERDPCDPHKGLFCDFGSPANRKIGVCTAKDGAPCVFGGSVYRSGESFQS*
*SCKYQCTCLDGAVGCVPLCSMDVRLPSPDCPFRRVKLPGKCCEEWVCDEPKDRT*
*AVGPALAAYRLEDTFGPDPTMMRANCLVQTTEWSACSKTCGMGISTRVTNDNTFCR*
*LEKQSRLCMVRPCEADLEENIKKGKKCIRTPKIAKPVKFELSGCTSVKTYRAKFCGV*
*CTDGRCCTPHRTTTLPVEFKCPDGEIMKKNMMFIKTCACHYNCPGDNDIFESLYYR*
*KMYGDMA*EPRGPTIKPCPPCKCPAPNLLGGPSVFIFPPKIKDVLMISLSPIVTCVV
VDVSEDDPDVQISWFVNNVEVHTAQTQTHREDYNSTLRVVSALPIQHQDWMS
GKEFKCKVNNKDLPAPIERTISKPKGSVRAPQVYVLPPPEEEMTKKQVTLTCMV
TDFMPEDIYVEWTNNGKTELNYKNTEPVLDSDGSYFMYSKLRVEKKNWVERN
SYSCSVVHEGLHNHHTTKSFSRTPGK

(Note: the signal peptide is single-underlined, the mouse CTGF coding region is italicized, and the mFc tag is double-underlined.)
Mouse CTGF protein-His tag, SEQ ID NO: 4

MEFGLSWLFLVAILKGVQC*QDCSAQCQCAAEAAPHCPAGVSLVLDGCGCCRVCAK QLGELCTERDPCDPHKGLFCDFGSPANRKIGVCTAKDGAPCVFGGSVYRSGESFQS SCKYQCTCLDGAVGCVPLCSMDVRLPSPDCPFPRRVKLPGKCCEEWVCDEPKDRT AVGPALAAYRLEDTFGPDPTMMRANCLVQTTEWSACSKTCGMGISTRVTNDNTFCR LEKQSRLCMVRPCEADLEENIKKGKKCIRTPKIAKPVKFELSGCTSVKTYRAKFCGV CTDGRCCTPHRTTTLPVEFKCPDGEIMKKNMMFIKTCACHYNCPGDNDIFESLYYR KMYGDMA*HHHHHH

(Note: the signal peptide is single-underlined, the mouse CTGF coding region is italicized, and the his tag is double-underlined.)
Cynomolgus monkey CTGF-Fc (a fusion protein of the cynomolgus monkey CTGF coding region and Fc), can be used for assays, SEQ ID NO: 5

MEFGLSWLFLVAILKGVQC*QNCSGPCRCPAEQAPRCPAGVSLVLDGCGCCRVCAK QLGELCTERDPCDPHKGLFCDFGSPANRKIGVCTAKDGAPCIFGGTVYRSGESFQSS CKYQCTCLDGAVGCMPLCSMDVRLPSPDCPFPRRVKLPGKCCEEWVCDEPKDQT VVGPALAAYRLEDTFGPDPTMIRANCLVQTTEWSACSKTCGMGISTRVTNDNASCRL EKQSRLCMVRPCEADLEENIKKGKKCIRTPKISKPIKFELSGCTSVKTYRAKFCGVCT DGRCCTPHRTTTLPVEFKCPDGEVMKKNMMFIKTCACHYNCPGDNDIFESLYYRK MYGDMA*EPKSSDKTHTCPPCPAPELLGGPSVFLFPPKPKDTLMISRTPEVTCVVV DVSHEDPEVKFNWYVDGVEVHNAKTKPREEQYNSTYRVVSVLTVLHQDWLN GKEYKCKVSNKALPAPIEKTISKAKGQPREPQVYTLPPSRDELTKNQVSLTCLVK GFYPSDIAVEWESNGQPENNYKTTPPVLDSDGSFFLYSKLTVDKSRWQQGNVFS CSVMHEALHNHYTQKSLSLSPGK

(Note: the signal peptide is single-underlined, the cynomolgus monkey CTGF coding region is italicized, and the Fc tag is double-underlined.)

**II. Purification of CTGF-associated recombinant protein and purification of anti-human CTGF hybridoma antibody and recombinant antibody**

**1. Separation and purification of hybridoma supernatant by ProteinG affinity chromatography**

For the purification of mouse hybridoma supernatant, Protein G is preferred for affinity chromatography. The cultured hybridoma was centrifuged to obtain the supernatant, and 10-15% by volume of 1 M Tris-HCl (pH 8.0-8.5) was added based on the volume of the supernatant to adjust the pH of the supernatant to neutrality. The Protein G column was washed with 3-5 column volumes of 6 M guanidine hydrochloride, then with 3-5 column volumes of pure water; the chromatographic column was equilibrated with 3-5 column volumes of, for example, 1× PBS (pH 7.4); the cell supernatant was loaded on the column at a low flow rate for binding, with the flow rate controlled to allow for a retention time of about 1 min or longer; the chromatographic column was washed with 3-5 column volumes of 1× PBS (pH 7.4) until the UV absorbance fell back to baseline; the sample was eluted with a 0.1 M acetic acid/sodium acetate (pH 3.0) buffer, the elution peaks were collected by UV detection, and the eluted product was rapidly adjusted to pH 5-6 with 1 M Tris-HCl (pH 8.0). For the eluted product, solution exchange may be performed by methods well known to those skilled in the art, such as ultrafiltration concentration using an ultrafiltration tube to exchange the solution to a desired buffer system, or exchange with a desired buffer system by size exclusion (e.g., G-25 desalination), or removal of polymer components from the eluted product using a high-resolution molecular exclusion column such as Superdex 200 to improve sample purity.

**2. Purification of antibody by Protein A affinity chromatography**

Firstly, the cell culture supernatant expressing the antibody was centrifuged at high speed, and the supernatant was collected. The Protein A affinity column was washed with 3-5 column volumes of 6 M guanidine hydrochloride, then with 3-5 column volumes of pure water. The chromatographic column was equilibrated with 3-5 column volumes of, for example, $1\times$ PBS (pH 7.4). The cell supernatant was loaded on the column at a low flow rate for binding, with the flow rate controlled to allow for a retention time of about 1 min or longer. After the binding was completed, the chromatographic column was washed with 3-5 column volumes of $1\times$ PBS (pH 7.4) until the UV absorbance fell back to baseline. The sample was eluted with a 0.1 M acetic acid/sodium acetate (pH 3.0-3.5) buffer, the elution peaks were collected by UV detection, and the eluted product was rapidly adjusted to pH 5-6 with 1 M Tris-HCl (pH 8.0). For the eluted product, solution exchange may be performed by methods well known to those skilled in the art, such as ultrafiltration concentration using an ultrafiltration tube to exchange the solution to a desired buffer system, or exchange with a desired buffer system by size exclusion (e.g., G-25 desalination), or removal of polymer components from the eluted product using a high-resolution molecular exclusion column such as Superdex 200 to improve sample purity.

**3. Nickel column purification of human CTGF-his and other CTGF-associated recombinant proteins**

The cell expression supernatant sample was centrifuged at high speed to remove impurities, and the buffer was exchanged with PBS, followed by the addition of imidazole to make a final concentration of 5 mM. A nickel column was equilibrated with a PBS solution containing 5 mM imidazole and washed with 2-5 column volumes. The supernatant sample after exchange was loaded on the column for binding. Nickel columns from different companies could be selected as the medium. The column was washed with a PBS solution containing 5 mM imidazole until A280 reading dropped to baseline. The chromatographic column was then washed with a mixture of PBS and 10 mM imidazole to remove non-specifically bound impure proteins, and the effluent was collected. The target protein was eluted with a PBS solution containing 300 mM imidazole and the elution peaks were collected.

After being concentrated, the collected eluted product could be further purified by gel chromatography Superdex200 (GE) with PBS as the mobile phase to remove aggregates and impurity protein peaks, and the elution peak of the target product was collected. The obtained protein was identified by electrophoresis, peptide mapping, and LC-MS, and then aliquoted for later use if it was determined to be correct.

**4. Purification of human CTGF-Fc, cynomolgus monkey CTGF-Fc, and other CTGF-associated recombinant proteins by Protein A affinity chromatography**

Firstly, the culture supernatant was centrifuged at high speed, and the supernatant was collected. The Protein A affinity column was washed with 3-5 column volumes of 6 M guanidine hydrochloride, then with 3-5 column volumes of pure water. The chromatographic column was equilibrated with 3-5 column volumes of, for example, $1\times$ PBS (pH 7.4). The cell supernatant was loaded on the column at a low flow rate for binding, with the flow rate controlled to allow for a retention time of about 1 min or longer. After the binding was completed, the chromatographic column was washed with 3-5 column volumes of $1\times$ PBS (pH 7.4) until the UV absorbance fell back to baseline. The sample was eluted with a 0.1 M acetic acid/sodium acetate (pH 3.0-3.5) buffer, and the elution peak was collected by UV detection.

After being concentrated, the collected eluted product could be further purified by gel chromatography Superdex200 (GE) with PBS as the mobile phase to remove aggregates and impurity protein peaks, and the elution peak of the target product was collected. The obtained protein was identified by electrophoresis, peptide mapping, and LC-MS, and then aliquoted for later use if it was determined to be correct.

**Example 1-2. Preparation of Anti-Human CTGF Monoclonal Antibodies**

**I. Immunization**

Anti-human CTGF monoclonal antibodies were produced by immunizing mice. Laboratory SJL white mice, female, 6-8 weeks of age (Beijing Vital River Laboratory Animal Technology Co., Ltd., animal production license number: SCXK(Beijing)2012-0001). Housing environment: SPF. The purchased mice were housed in a laboratory environment for 1 week, in 12/12 hour light/dark cycles, at a temperature of 20-25 °C, with humidity at 40-60%. The acclimatized mice were immunized according to the following scheme. The immune antigens were CTGF (R&D) and mCTGF-mFc.

Immunization scheme: mice were immunized with CTGF protein at 25 $\mu$g/mouse/time by intraperitoneal injection. On day 0, each mouse was injected intraperitoneally (IP) with 100 $\mu$L and was administered a boost immunization every 14 days. Blood was collected on days 21, 35, 49, and 63, and the antibody titer in mouse serum was determined by ELISA. After 7-9 immunizations, mice in which the antibody titer in serum was high and was reaching a plateau were selected for splenocyte fusion. A boost immunization was performed 3 days before the splenocyte fusion, and a solution of CTGF protein antigen in normal saline was intraperitoneally (IP) injected at 25 μg/mouse.

## II. Splenocyte fusion

Spleen lymphocytes and Sp2/0 myeloma cells (ATCC® CRL-8287™) were fused by following a PEG-mediated fusion procedure to obtain hybridoma cells. The resulting hybridoma cells were resuspended in a complete medium (an IMDM medium containing 20% FBS, 1× HAT, and 1× OPI) at a density of 0.5-1 × 10$^6$/mL and seeded in a 96-well plate at 100 μL/well. The plate was incubated at 37 °C with 5% $CO_2$ for 3-4 days, supplemented with the HAT complete medium at 100 μL/well, and incubated for another 3-4 days to form pinpoint-like clones. The supernatant was removed, and an HT complete medium (an IMDM medium containing 20% FBS, 1× HT, and 1× OPI) was added at 200 μL/well. The plate was incubated at 37 °C with 5% $CO_2$ for 3 days, followed by an ELISA assay.

## III. Screening of hybridoma cells

Hybridoma culture supernatants were tested by a binding ELISA method according to the growth density of hybridoma cells. Then the cell supernatants from positive wells as determined by binding ELISA were subjected to a binding experiment with monkey CTGF/mouse CTGF/human CTGF proteins (using the same method as Example 3). The cells from positive wells as determined by the protein ELISA binding experiment were expanded and cryopreserved in time and subcloned 2 to 3 times until single cell clones were obtained.

A CTGF binding ELISA assay was also performed for each cell subcloning. Hybridoma clones were obtained by the above screening process, and antibodies were further prepared using a serum-free cell culture method. The antibodies were purified, according to the purification example, for use in the test examples.

## IV. Sequencing of hybridoma positive clones

The cloning of sequences from positive hybridomas is as follows. Hybridoma cells in the logarithmic growth phase were harvested, and RNA was extracted with Trizol (Invitrogen, Cat No. 15596-018) by following the procedures in the kit instructions and reverse-transcribed using a PrimeScript™ Reverse Transcriptase kit (Takara, Cat No. 2680A). The cDNA obtained by reverse transcription was amplified by PCR using mouse Ig-Primer Set (Novagen, TB326 Rev.B 0503), and then the products of the amplification were sent to a sequencing company for sequencing. The amino acid sequences of the variable regions of the murine anti-CTGF antibodies mab 147, mab 164, and mab95 obtained by sequencing are as follows:

The amino acid sequence of the VH of mab 147 is set forth in SEQ ID NO: 6,

*EVQLVESGGGLVQPEGSLKLSCAASGFSFNTYAMNWVRQAPGKGLEWVARIRTKSN
NYATYYADSVKDRFTISRDDSESMLYLQMNNLKTEDTAMYYCVETGFAYWDQGTLVT
VSA*

The amino acid sequence of the VL of mab 147 is set forth in SEQ ID NO: 7,

*QIVLTQSPAIMSASPGEKVTITCSASSSVSYMHWFQQKPGTSPKLWIYSTSNLASGVPAR
FSGSGSGTSYSLTISRMEAEDAATYYCQQRSSYPLTFGAGTKLELK*

The amino acid sequence of the VH of mab164 is set forth in SEQ ID NO: 8,

*QVQLKQSGPGLVQPSQSLSITCTVSGFSLTTFGVHWIRQSPGKGLEWLGVIWRRGGT*

*DYNAAFMSRLSITKDNSRSHVFFKMTSLQTDDSAIYYCARDGGFDYWGQGTTLTVSS*

The amino acid sequence of the VL of mab 164 is set forth in SEQ ID NO: 9,

*QAVVTQESALTTSPGGTVILTCRSSIGAVTTSNYANWVQEKPDHLFTGLIGGTSNRAPG
VPVRFSGSLIGDKAALTITGAQAEDDAMYFCALWYSTHYVFGGGTKVTVL*

The amino acid sequence of the VH of mab95 is set forth in SEQ ID NO: 69,

*EVLLQQSGPVLVKPGPSVKISCKASGFTFTNYDIHWVKQSHGKSLEWIGLVYPYNGG
TAYNQKFKDKATLTFDTSSSTAYMELNSLTSEDSAVYYCARWGLIPGTTSYFDVWGTG
TTVTVSS*

The amino acid sequence of the VL of mab95 is set forth in SEQ ID NO: 70,

*QIVLSQSPPILSASPGEKVTMTCRASSSVSYIHWYQQKPRSSPKPWIYATSNLASGVPAR
FSGSGSGTSYSLTISRVEAEDAATYYCQQWNSNPWTFGGGTRLEIK*

In the variable region sequences of the mab147, mab164 and mab95 antibodies, the FR sequences are italicized, the CDR sequences are underlined and italicized, and the sequence order is FR1-CDR1-FR2-CDR2-FR3-CDR3-FR4.

Table 5. The amino acid sequences of the CDR regions of the heavy and light chains of anti-CTGF antibodies*

| Antibody | Heavy chain | | Light chain | |
|---|---|---|---|---|
| mab147 | HCDR1 | TYAMN SEQ ID NO: 10 | LCDR1 | SASSSVSYMH SEQ ID NO: 13 |
| | HCDR2 | RIRTKSNNYATYYADSVKD SEQ ID NO: 11 | LCDR2 | STSNLAS SEQ ID NO: 14 |
| | HCDR3 | TGFAY SEQ ID NO: 12 | LCDR3 | QQRSSYPLT SEQ ID NO: 15 |
| mab164 | HCDR1 | TFGVH SEQ ID NO: 16 | LCDR1 | RSSIGAVTTSNYAN SEQ ID NO: 19 |
| | HCDR2 | VIWRRGGTDYNAAFMS SEQ ID NO: 17 | LCDR2 | GTSNRAP SEQ ID NO: 20 |
| | HCDR3 | DGGFDY SEQ ID NO: 18 | LCDR3 | ALWYSTHYV SEQ ID NO: 21 |
| mab95 | HCDR1 | NYDIH SEQ ID NO: 71 | LCDR1 | RASSSVSYIH SEQ ID NO: 74 |
| | HCDR2 | LVYPYNGGTAYNQKFKD SEQ ID NO: 72 | LCDR2 | ATSNLAS SEQ ID NO: 75 |
| | HCDR3 | WGLIPGTTSYFDV SEQ ID NO: 73 | LCDR3 | QQWNSNPWT SEQ ID NO: 76 |
| * The CDRs in the table are determined by the Kabat numbering scheme. | | | | |

**V. Preparation of human IgG1 chimeric antibodies**

The variable region coding gene sequences could be obtained by amplifying and sequencing candidate molecules mab147, mab164, and mab95, a head-tail primer was designed using the sequences obtained by sequencing, a VH/VK gene fragment was constructed for each antibody by PCR using the sequenced gene as a template, and homologously recombined with an expression vector pHr (with a signal peptide and an hIgG1/hkappa/hlambda constant region gene (CH1-Fc/CL) fragment) to construct a recombinant chimeric antibody full-length expression plasmid VH-CH1-Fc-pHr/VL-CL-pHr, and to form three chimeric antibodies: Ch147, Ch164, and Ch95.

**VI. Humanization of murine anti-human CTGF antibodies**

By comparing the IMGT (http://imgt.cines.fr) human antibody heavy and light chain variable region germline gene database with the MOE (molecular operating environment) software, heavy and light chain variable region germline genes with high homology to the murine antibodies were selected as templates, and CDRs of the murine antibodies were grafted into corresponding humanized templates to form variable region sequences in the following order: FR1-CDR1-FR2-CDR2-FR3-CDR3-FR4.

**1. Humanization of mab147**

The humanization light chain templates for the murine antibody mab147 are IGKV3-11*01 and IGKJ2*01 or IGKV1-39*01 and IGKJ2*01, and the humanization heavy chain templates are IGHV3-72*01 and IGHJ1*01. The CDRs of the murine antibody mab147 were grafted into its humanization templates, and further, some amino acids of the FR of the humanized antibody were modified with back mutations, wherein back mutations for light chain variable regions include one or more of 4L, 36F, 43S, 45K, 47W, 58V, and 71Y, and back mutations for heavy chain variable regions include one or more of 28S, 30N, 49A, 75E, 76S, 93V, 94E, and 104D (the positions of the back mutations in the light and heavy chain variable regions are determined by the Kabat numbering scheme); light and heavy chain variable regions with different sequences were obtained. Thus, a humanized antibody comprising the CDRs of mab147 was obtained, and the variable region sequences thereof are as follows:

The specific sequences of the variable regions of the mAb147 humanized antibody are as follows:

The amino acid sequence of Hu147-VL1 is set forth in SEQ ID NO: 22:

*EIVLTQSPATLSLSPGERATLSCSASSSVSYMHWFQQKPGQSPRLLIYSTSNLASGIPARF SGSGSGTDYTLTISSLEPEDFAVYYCQQRSSYPLTFGQGTKLEIK*

The amino acid sequence of Hu147-VL2 is set forth in SEQ ID NO: 23:

*EIVLTQSPATLSLSPGERATLSCSASSSVSYMHWFQQKPGQSPKLLIYSTSNLASGIPAR FSGSGSGTDYTLTISSLEPEDFAVYYCQQRSSYPLTFGQGTKLEIK*

The amino acid sequence of Hu147-VL3 is set forth in SEQ ID NO: 24:

*EIVLTQSPATLSLSPGERATLSCSASSSVSYMHWFQQKPGQSPKLWIYSTSNLASGVPAR FSGSGSGTDYTLTISSLEPEDFAVYYCQQRSSYPLTFGQGTKLEIK*

The amino acid sequence of Hu147-VL4 is set forth in SEQ ID NO: 25:

*DIQMTQSPSSLSASVGDRVTITCSASSSVSYMHWFQQKPGKSPKLLIYSTSNLASGVPS RFSGSGSGTDYTLTISSLQPEDFATYYCQQRSSYPLTFGQGTKLEIK*

The amino acid sequence of Hu147-VL5 is set forth in SEQ ID NO: 26:

*DIQLTQSPSSLSASVGDRVTITCSASSSVSYMHWFQQKPGKSPKLWIYSTSNLASGVPS RFSGSGSGTDYTLTISSLQPEDFATYYCQQRSSYPLTFGQGTKLEIK*

The amino acid sequence of Hu147-VH1 is set forth in SEQ ID NO: 27:

*EVQLVESGGGLVQPGGSLRLSCAASGFTFSTYAMNWVRQAPGKGLEWVGRIRTKSN*

*NYATYYADSVKDRFTISRDDSKNSLYLQMNSLKTEDTAVYYCVETGFAYWDQGTLVTV*
*SS*

The amino acid sequence of Hu147-VH2 is set forth in SEQ ID NO: 28:

*EVQLVESGGGLVQPGGSLRLSCAASGFTFNTYAMNWVRQAPGKGLEWVARIRTKSN*
*NYATYYADSVKDRFTISRDDSKNSLYLQMNSLKTEDTAVYYCVETGFAYWDQGTLVTV*
*SS*

The amino acid sequence of Hu147-VH3 is set forth in SEQ ID NO: 29:

*EVQLVESGGGLVQPGGSLRLSCAASGFSFNTYAMNWVRQAPGKGLEWVARIRTKSN*
*NYATYYADSVKDRFTISRDDSESSLYLQMNSLKTEDTAVYYCVETGFAYWDQGTLVTVS*
*S.*

Note: The CDR sequences defined by the Kabat numbering scheme are underlined and the order is FR1-CDR1-FR2-CDR2-FR3- CDR3-FR4.

### 2. Humanization of mab164

The humanization light chain template for the murine antibody mab164 consists of IGLV7-43*01 and IGLJ2*01, IGLV8-61*01 and IGLJ2*01, or IGLV1-40*02 and IGLJ2*01, and the humanization heavy chain template consists of IGHV2-26*01 and IGHJ6*01, or IGHV4-31*02 and IGHJ6*01. The CDRs of the murine antibody mab164 were grafted into its humanization templates, and further, some amino acids of the FR of the humanized antibody were modified with back mutations, wherein back mutations for light chain variable regions include one or more of 36V, 44F, 46G, and 49G, and back mutations for heavy chain variable regions include one or more of 44G, 49G, 27F, 48L, 67L, 71K, 78V, and 80F (the positions of the back mutations in the light and heavy chain variable regions are determined by the Kabat numbering scheme); thus, the humanized antibody heavy chain and the humanized antibody light chain of mab164 were obtained, and the variable region sequences thereof are as follows:

The specific sequences of the variable regions of the mAb164 humanized antibody are as follows:

The amino acid sequence of Hu164-VL7 is set forth in SEQ ID NO: 30:

*ETVVTQEPSLTVSPGGTVTLTCRSSIGAVTTSNYANWVQQKPGQAFRGLIGGTSNRAP*
*WTPARFSGSLLGGKAALTLSGVQPEDEAEYYCALWYSTHYVFGGGTKLTVL*

The amino acid sequence of Hu164-VL8 is set forth in SEQ ID NO: 31:

*ETVVTQEPSFSVSPGGTVTLTCRSSIGAVTTSNYANWVQQTPGQAFRGLIGGTSNRAP*
*GVPDRFSGSILGNKAALTITGAQADDESDYYCALWYSTHYVFGGGTKLTVL*

The amino acid sequence of Hu164-VL9 is set forth in SEQ ID NO: 32:

*ESVVTQPPSVSGAPGQRVTISCRSSIGAVTTSNYANWVQQLPGTAFKGLIGGTSNRAP*
*GVPDRFSGSKSGTSASLAITGLQAEDEADYYCALWYSTHYVFGGGTKLTVL*

The amino acid sequence of Hu164-VHS is set forth in SEQ ID NO: 33:

*EVTLKESGPVLVKPTETLTLTCTVSGFSLSTFGVHWIRQPPGKALEWLAVIWRRGGTD YNAAFMSRLTISKDTSKSQVVLTMTNMDPVDTATYYCARDGGFDYWGQGTTVTVSS*

The amino acid sequence of Hu164-VH6 is set forth in SEQ ID NO: 34:

*EVTLKESGPVLVKPTETLTLTCTVSGFSLSTFGVHWIRQPPGKGLEWLGVIWRRGGT DYNAAFMSRLTISKDTSKSQVVFTMTNMDPVDTATYYCARDGGFDYWGQGTTVTVS S*

The amino acid sequence of Hu164-VH7 is set forth in SEQ ID NO: 35:

*EVQLQESGPGLVKPSQTLSLTCTVSGFSISTFGVHWIRQHPGKGLEWIGVIWRRGGT DYNAAFMSRVTISKDTSKNQVSLKLSSVTAADTAVYYCARDGGFDYWGQGTTVTVSS*

The amino acid sequence of Hu164-VH8 is set forth in SEQ ID NO: 36:

*EVQLQESGPGLVKPSQTLSLTCTVSGFSISTFGVHWIRQHPGKGLEWLGVIWRRGGT DYNAAFMSRLTISKDTSKNQVSFKLSSVTAADTAVYYCARDGGFDYWGQGTTVTVSS.*

Note: in the above Hu164 antibody sequences, the CDR sequences are underlined and the order is FR1-CDR1-FR2-CDR2- FR3-CDR3-FR4, wherein the CDR sequences are defined by the Kabat numbering scheme.

## 3. Humanization of mab95

The humanization light chain templates for the murine antibody mab95 are IGKV3-20*02 and IGKV1-40∗01, and the humanization heavy chain template is IGHV1-3∗01. The CDRs of the murine antibody mab95 were grafted into its humanization templates, and further, some amino acids of the FR of the humanized antibody were modified with back mutations, wherein back mutations for light chain variable regions include one or more of 45P, 46W, 48Y, 69S, and 70Y, and back mutations for heavy chain variable regions include one or more of 27F, 38K, 48I, 67K, 68A, 70L, and 72F (the positions of the back mutations in the light and heavy chain variable regions are determined by the Kabat numbering scheme); light and heavy chain variable regions with different sequences were obtained. Thus, a humanized antibody comprising the CDRs of mab95 was obtained, and the variable region sequences thereof are as follows:

The specific sequences of the variable regions of the mAb95 humanized antibody are as follows:

The amino acid sequence of Hu95-VL1 is set forth in SEQ ID NO: 77:

*EIVLTQSPATLSLSPGERATLSCRASSSVSYIHWYQQKPGQAPRPWIYATSNLASGIPARF SGSGSGTDYTLTISRLEPEDFAVYYCQQWNSNPWTFGGGTKVEIK*

The amino acid sequence of Hu95-VL2 is set forth in SEQ ID NO: 78:

*EIVLTQSPATLSLSPGERATLSCRASSSVSYIHWYQQKPGQSPRPWIYATSNLASGVPAR FSGSGSGTSYTLTISRLEPEDFAVYYCQQWNSNPWTFGGGTKVEIK*

The amino acid sequence of Hu95-VL3 is set forth in SEQ ID NO: 79:

*ESVLTQPPSVSGAPGQRVTISCRASSSVSYIHWYQQLPGTAPKPWIYATSNLASGVPDR FSGSKSGTSYSLAITGLQAEDEADYYCQQWNSNPWTFGGGTKVEIK*

The amino acid sequence of Hu95-VL4 is set forth in SEQ ID NO: 80:

*EIVLTQPPSVSGAPGQRVTISCRASSSVSYIHWYQQLPGTSPKPWIYATSNLASGVPDR*
*FSGSKSGTSYSLAITGLQAEDEADYYCQQWNSNPWTFGGGTKVEIK*

The amino acid sequence of Hu95-VH1 is set forth in SEQ ID NO: 81:

*EVQLVQSGAEVKKPGASVKVSCKASGFTFTNYDIHWVRQAPGQRLEWMGLVYPYNG*
*GTAYNQKFKDRVTLTFDTSASTAYMELSSLRSEDTAVYYCARWGLIPGTTSYFDVWGQ*

*GTTVTVSS*

The amino acid sequence of Hu95-VH2 is set forth in SEQ ID NO: 82:

*EVQLVQSGAEVKKPGASVKVSCKASGFTFTNYDIHWVRQAPGQRLEWIGLVYPYNG*
*GTAYNQKFKDRATLTFDTSASTAYMELSSLRSEDTAVYYCARWGLIPGTTSYFDVWGQ*
*GTTVTVSS*

The amino acid sequence of Hu95-VH3 is set forth in SEQ ID NO: 83:

*EVQLVQSGAEVKKPGASVKVSCKASGFTFTNYDIHWVKQAPGQRLEWMGLVYPYNG*
*GTAYNQKFKDKVTLTFDTSASTAYMELSSLRSEDTAVYYCARWGLIPGTTSYFDVWGQ*
*GTTVTVSS*

The amino acid sequence of Hu95-VH4 is set forth in SEQ ID NO: 84:

*EVQLVQSGAEVKKPGASVKVSCKASGFTFTNYDIHWVKQAPGQRLEWIGLVYPYNG*
*GTAYNQKFKDKATLTFDTSASTAYMELSSLRSEDTAVYYCARWGLIPGTTSYFDVWGQ*
*GTTVTVSS*

The amino acid sequence of Hu95-VH5 is set forth in SEQ ID NO: 85:

*EVQLVQSGAEVKKPGASVKVSCRASGFTFTNYAIHWVKQAPGQRLEWMGLVYPYTG*
*GTAYNQKFKDKVTLTFDTSASTAYMELSSLRSEDTAVYYCARWGMIPGTNSYFDVWG*
*QGTTVTVSS.*

Note: The CDR sequences defined by the Kabat numbering scheme are underlined and the order is FR1-CDR1-FR2-CDR2-FR3- CDR3-FR4.

The CDR regions of Hu95-VH5 were further modified. HCDR1 is set forth in SEQ ID NO: 102 (NYAIH), HCDR2 is set forth in SEQ ID NO: 103 (LVYPYTGGTAYNQKFKD), and HCDR3 is set forth in SEQ ID NO: 104 (WGMIPGTNSYFDV).

## 4. Construction and expression of anti-CTGF humanized full-length antibodies

A primer was designed, and then a VH/VL gene fragment of each humanized antibody was constructed by PCR and subjected to homologous recombination with an expression vector pHr (with a signal peptide and a constant region gene (CH/CL) fragment), thus constructing an expression vector VH-CH -pHr/VL-CL-pHr for a full-length antibody. The constant region of the humanized antibody may be selected from the group consisting of the light chain constant regions of human κ and λ chains, and from the group consisting of the heavy chain constant regions of IgG1, IgG2, IgG3, and IgG4, and variants thereof. Non-limiting examples include optimizing the constant regions of human IgG1, IgG2, and IgG4 to improve antibody function; for example, the half-life of the antibody can be prolonged by M252Y/S254T/T256E ("YTE") point mutations in the constant region, S228P, F234A, and L235A, and other point mutations in the constant region, and the like.

Exemplary anti-CTGF humanized antibody constant region sequences are as follows: The heavy chain constant region amino acid sequence is set forth in SEQ ID NO: 37 (w is suffixed to the name of the formed full-length antibody heavy chain):

ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTC

PPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPE
NNYKTTPPVLDSDGSFFLYSKLTVDKSRWQQGNVFSCSVMHEALHNHYTQKSL
SLSPGK

The heavy chain constant region amino acid sequence of the YTE-modified antibody is set forth in SEQ ID NO: 38 (y is suffixed to the name of the formed full-length antibody heavy chain):

ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTC
PPCPAPELLGGPSVFLFPPKPKDTLYITREPEVTCVVVDVSHEDPEVKFNWYVDG
VEVHNAKTKPREEQYNSTYRVVSVLTVLHQDWLNGKEYKCKVSNKALPAPIEK
TISKAKGQPREPQVYTLPPSRDELTKNQVSLTCLVKGFYPSDIAVEWESNGQPEN
NYKTTPPVLDSDGSFFLYSKLTVDKSRWQQGNVFSCSVMHEALHNHYTQKSLS
LSPGK

The light chain kappa constant region amino acid sequence of the anti-CTGF humanized antibody is set forth in SEQ ID NO: 39 (k is suffixed to the name of the formed full-length antibody heavy chain):

RTVAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGEC

The light chain lambda constant region amino acid sequence of the anti-CTGF humanized antibody is set forth in SEQ ID NO: 40 (1 is suffixed to the name of the formed full-length antibody heavy chain):

GQPKANPTVTLFPPSSEELQANKATLVCLISDFYPGAVTVAWKADGSPVKAGVE
TTKPSKQSNNKYAASSYLSLTPEQWKSHRSYSCQVTHEGSTVEKTVAPTEC

Full-length humanized antibodies derived from mab147 formed by linking a heavy chain variable region of the humanized antibody to the heavy chain constant region set forth in SEQ ID NO: 37 and linking a light chain variable region of the humanized antibody to the light chain kappa constant region set forth in SEQ ID NO: 39 include:

Table 6

|  | Hu147-VL1 | Hu147-VL2 | Hu147-VL3 | Hu147-VL4 | Hu147-VL5 |
|---|---|---|---|---|---|
| Hu147-VH1 | Hu147-11wk | Hu147-12wk | Hu147-13wk | Hu147-14wk | Hu147-15wk |
| Hu147-VH2 | Hu147-21wk | Hu147-22wk | Hu147-23wk | Hu147-24wk | Hu147-25wk |
| Hu147-VH3 | Hu147-31wk | Hu147-32wk | Hu147-33wk | Hu147-34wk | Hu147-35wk |

Full-length humanized antibodies derived from mab147 formed by linking a heavy chain variable region of the humanized antibody to the heavy chain constant region YTE variant set forth in SEQ ID NO: 38 and linking a light chain variable region of the humanized antibody to the light chain kappa constant region set forth in SEQ ID NO: 39 include:

Table 7

|  | Hu147-VL1 | Hu147-VL2 | Hu147-VL3 | Hu147-VL4 | Hu147-VLS |
|---|---|---|---|---|---|
| Hu147-VH1 | Hu147-11yk | Hu147-12yk | Hu147-13yk | Hu147-14yk | Hu147-15yk |
| Hu147-VH2 | Hu147-21yk | Hu147-22yk | Hu147-23yk | Hu147-24yk | Hu147-25yk |
| Hu147-VH3 | Hu147-31yk | Hu147-32yk | Hu147-33yk | Hu147-34yk | Hu147-35yk |

Full-length humanized antibodies derived from mab164 formed by linking a heavy chain variable region of the humanized antibody to the heavy chain constant region set forth in SEQ ID NO: 37 and linking a light chain variable region of the humanized antibody to the light chain lambda constant region set forth in SEQ ID NO: 40 include:

Table 8

|  | Hu164-VHS | Hu164-VH6 | Hu164-VH7 | Hu164-VH8 |
|---|---|---|---|---|
| Hu164-VL7 | Hu164-57wl | Hu164-67wl | Hu164-77wl | Hu164-87wl |
| Hu164-VL8 | Hu164-5 8wl | Hu164-68wl | Hu164-78wl | Hu164-88wl |
| Hu164-VL9 | Hu164-59wl | Hu164-69wl | Hu164-79wl | Hu164-89wl |

Full-length humanized antibodies derived from mab164 formed by linking a heavy chain variable region of the humanized antibody to the heavy chain constant region YTE variant set forth in SEQ ID NO: 38 and linking a light chain variable region of the humanized antibody to the light chain lambda constant region set forth in SEQ ID NO: 40 include:

Table 9

|  | Hu164-VHS | Hu164-VH6 | Hu164-VH7 | Hu164-VH8 |
|---|---|---|---|---|
| Hu164-VL7 | Hu164-57yl | Hu164-67yl | Hu164-77yl | Hu164-87yl |
| Hu164-VL8 | Hu164-58yl | Hu164-68yl | Hu164-78yl | Hu164-88yl |
| Hu164-VL9 | Hu164-59yl | Hu164-69yl | Hu164-79yl | Hu164-89yl |

Full-length humanized antibodies derived from mab95 formed by linking a heavy chain variable region of the humanized antibody to the heavy chain constant region set forth in SEQ ID NO: 37 and linking a light chain variable region of the humanized antibody to the light chain kappa constant region set forth in SEQ ID NO: 39 include:

Table 10

|  | Hu95-VH1 | Hu95-VH2 | Hu95-VH3 | Hu95-VH4 | Hu95-VH5 |
|---|---|---|---|---|---|
| Hu95-VL1 | Hu95-11wk | Hu95-21wk | Hu95-31wk | Hu95-41wk | Hu95-51wk |
| Hu95-VL2 | Hu95-12wk | Hu95-22wk | Hu95-32wk | Hu95-42wk | Hu95-52wk |
| Hu95-VL3 | Hu95-13wk | Hu95-23wk | Hu95-33wk | Hu95-43wk | Hu95-53wk |

(continued)

| Hu95-VL4 | Hu95-14wk | Hu95-24wk | Hu95-34wk | Hu95-44wk | Hu95-54wk |
|---|---|---|---|---|---|

Full-length humanized antibodies derived from mab95 formed by linking a heavy chain variable region of the humanized antibody to the heavy chain constant region YTE variant set forth in SEQ ID NO: 38 and linking a light chain variable region of the humanized antibody to the light chain kappa constant region set forth in SEQ ID NO: 39 include:

Table 11

|  | Hu95-VH1 | Hu95-VH2 | Hu95-VH3 | Hu95-VH4 | Hu95-VH5 |
|---|---|---|---|---|---|
| Hu95-VL1 | Hu95-11yk | Hu95-21yk | Hu95-31yk | Hu95-41yk | Hu95-51yk |
| Hu95-VL2 | Hu95-12yk | Hu95-22yk | Hu95-32yk | Hu95-42yk | Hu95-52yk |
| Hu95-VL3 | Hu95-13yk | Hu95-23yk | Hu95-33yk | Hu95-43yk | Hu95-53yk |
| Hu95-VL4 | Hu95-14yk | Hu95-24yk | Hu95-34yk | Hu95-44yk | Hu95-54yk |

Exemplary anti-CTGF antibody full-length amino acid sequences are as follows:

Table 12. Light or heavy chains of full-length antibodies

| Light or heavy chains of full-length antibodies (SEQ ID NO) | Amino acid sequence |
|---|---|
| Ch 147 heavy chain (SEQ ID NO: 41) | *EVQLVESGGGLVQPEGSLKLSCAASGFSFNTYAMNWVRQAPGKGLEWVARI RTKSNNYATYYADSVKDRFTISRDDSESMLYLQMNNLKTEDTAMYYCVETGF AYWDQGTLVTVSA*ASTKGPSVFPLAPSSKSTSGGTAALGCLVKDYFPEPV TVSWNSGALTSGVHTFPAVLQSSGLYSLSSVVTVPSSSLGTQTYICNVN HKPSNTKVDKKVEPKSCDKTHTCPPCPAPELLGGPSVFLFPPKPKDTLM ISRTPEVTCVVVDVSHEDPEVKFNWYVDGVEVHNAKTKPREEQYNST YRVVSVLTVLHQDWLNGKEYKCKVSNKALPAPIEKTISKAKGQPREPQ VYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLS PGK |
| Ch147 light chain (SEQ ID NO: 42) | *QIVLTQSPAIMSASPGEKVTITCSASSSVSYMHWFQQKPGTSPKLWIYSTSNLA SGVPARFSGSGSGTSYSLTISRMEAEDAATYYCQQRSSYPLTFGAGTKLELK*R TVAAPSVFIFPPSDEQLKSGTASVVCLLNNFYPREAKVQWKVDNALQS GNSQESVTEQDSKDSTYSLSSTLTLSKADYEKHKVYACEVTHQGLSSPV TKSFNRGEC |
| Full-length heavy chains of humanized antibodies Hu147-11wk, Hu147-12wk, Hu147-13 wk, Hu147-14wk, and Hu147-15wk (SEQ ID NO: 43) | *EVQLVESGGGLVQPGGSLRLSCAASGFTFSTYAMNWVRQAPGKGLEWVGR IRTKSNNYATYYADSVKDRFTISRDDSKNSLYLQMNSLKTEDTAVYYCVETGF AYWDQGTLVTVSS*ASTKGPSVFPLAPSSKSTSGGTAALGCLVKDYFPEPV TVSWNSGALTSGVHTFPAVLQSSGLYSLSSVVTVPSSSLGTQTYICNVN HKPSNTKVDKKVEPKSCDKTHTCPPCPAPELLGGPSVFLFPPKPKDTLM ISRTPEVTCVVVDVSHEDPEVKFNWYVDGVEVHNAKTKPREEQYNST YRVVSVLTVLHQDWLNGKEYKCKVSNKALPAPIEKTISKAKGQPREPQ VYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLS PGK |

(continued)

| Light or heavy chains of full-length antibodies (SEQ ID NO) | Amino acid sequence |
|---|---|
| Full-length heavy chains of humanized antibodies Hu147-21wk, Hu147-22wk, Hu147-23wk, Hu147-24wk, and Hu147-24wk (SEQ ID NO: 44) | *EVQLVESGGGLVQPGGSLRLSCAASGFTFNTYAMNWVRQAPGKGLEWVAR IRTKSNNYATYYADSVKDRFTISRDDSKNSLYLQMNSLKTEDTAVYYCVETGF AYWDQGTLVTVSS*ASTKGPSVFPLAPSSKSTSGGTAALGCLVKDYFPEPV TVSWNSGALTSGVHTFPAVLQSSGLYSLSSVVTVPSSSLGTQTYICNVN HKPSNTKVDKKVEPKSCDKTHTCPPCPAPELLGGPSVFLFPPKPKDTLM ISRTPEVTCVVVDVSHEDPEVKFNWYVDGVEVHNAKTKPREEQYNST YRVVSVLTVLHQDWLNGKEYKCKVSNKALPAPIEKTISKAKGQPREPQ VYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLS PGK |
| Full-length heavy chains of humanized antibodies Hu147-3lwk, Hu147-32wk, Hu147-33wk, Hu147-34wk, and Hu147-35wk (SEQ ID NO: 45) | *EVQLVESGGGLVQPGGSLRLSCAASGFSFNTYAMNWVRQAPGKGLEWVARI RTKSNNYATYYADSVKDRFTISRDDSESSLYLQMNSLKTEDTAVYYCVETGFA YWDQGTLVTVSS*ASTKGPSVFPLAPSSKSTSGGTAALGCLVKDYFPEPVT VSWNSGALTSGVHTFPAVLQSSGLYSLSSVVTVPSSSLGTQTYICNVNH KPSNTKVDKKVEPKSCDKTHTCPPCPAPELLGGPSVFLFPPKPKDTLMIS RTPEVTCVVVDVSHEDPEVKFNWYVDGVEVHNAKTKPREEQYNSTYR VVSVLTVLHQDWLNGKEYKCKVSNKALPAPIEKTISKAKGQPREPQVY TLPPSRDELTKNQVSLTCLVKGFYPSDIAVEWESNGQPENNYKTTPPVL DSDGSFFLYSKLTVDKSRWQQGNVFSCSVMHEALHNHYTQKSLSLSPG K |
| Full-length heavy chains of humanized antibodies Hu147-11yk, Hu147-12yk, Hu147-13yk, Hu147-14yk, and Hu147-15yk (SEQ ID NO: 46) | *EVQLVESGGGLVQPGGSLRLSCAASGFTFSTYAMNWVRQAPGKGLEWVGR IRTKSNNYATYYADSVKDRFTISRDDSKNSLYLQMNSLKTEDTAVYYCVETGF AYWDQGTLVTVSS*ASTKGPSVFPLAPSSKSTSGGTAALGCLVKDYFPEPV TVSWNSGALTSGVHTFPAVLQSSGLYSLSSVVTVPSSSLGTQTYICNVN HKPSNTKVDKKVEPKSCDKTHTCPPCPAPELLGGPSVFLFPPKPKDTLYI TREPEVTCVVVDVSHEDPEVKFNWYVDGVEVHNAKTKPREEQYNSTY RVVSVLTVLHQDWLNGKEYKCKVSNKALPAPIEKTISKAKGQPREPQV YTLPPSRDELTKNQVSLTCLVKGFYPSDIAVEWESNGQPENNYKTTPPV LDSDGSFFLYSKLTVDKSRWQQGNVFSCSVMHEALHNHYTQKSLSLSP GK |
| Full-length heavy chains of humanized antibodies Hu147-2lyk, Hu147-22yk, Hu147-23yk, Hu147-24yk, and Hu147-24yk (SEQ ID NO: 47) | *EVQLVESGGGLVQPGGSLRLSCAASGFTFNTYAMNWVRQAPGKGLEWVAR IRTKSNNYATYYADSVKDRFTISRDDSKNSLYLQMNSLKTEDTAVYYCVETGF AYWDQGTLVTVSS*ASTKGPSVFPLAPSSKSTSGGTAALGCLVKDYFPEPV TVSWNSGALTSGVHTFPAVLQSSGLYSLSSVVTVPSSSLGTQTYICNVN HKPSNTKVDKKVEPKSCDKTHTCPPCPAPELLGGPSVFLFPPKPKDTLYI TREPEVTCVVVDVSHEDPEVKFNWYVDGVEVHNAKTKPREEQYNSTY RVVSVLTVLHQDWLNGKEYKCKVSNKALPAPIEKTISKAKGQPREPQV YTLPPSRDELTKNQVSLTCLVKGFYPSDIAVEWESNGQPENNYKTTPPV LDSDGSFFLYSKLTVDKSRWQQGNVFSCSVMHEALHNHYTQKSLSLSP GK |

(continued)

| Light or heavy chains of full-length antibodies (SEQ ID NO) | Amino acid sequence |
|---|---|
| Full-length heavy chains of humanized antibodies<br><br>Hu147-31yk, Hu147-32yk, Hu147-33yk, Hu147-34yk, and Hu147-35yk (SEQ ID NO: 48) | *EVQLVESGGGLVQPGGSLRLSCAASGFSFN<u>TYAMN</u>WVRQAPGKGLEWVARI RTKSNNYATYYADSVKDRFTISRDDSESSLYLQMNSLKTEDTAVYYCVET<u>GFA YWDQGTLVTVSS</u>*ASTKGPSVFPLAPSSKSTSGGTAALGCLVKDYFPEPVT VSWNSGALTSGVHTFPAVLQSSGLYSLSSVVTVPSSSLGTQTYICNVNH KPSNTKVDKKVEPKSCDKTHTCPPCPAPELLGGPSVFLFPPKPKDTLYIT REPEVTCVVVDVSHEDPEVKFNWYVDGVEVHNAKTKPREEQYNSTYR VVSVLTVLHQDWLNGKEYKCKVSNKALPAPIEKTISKAKGQPREPQVY TLPPSRDELTKNQVSLTCLVKGFYPSDIAVEWESNGQPENNYKTTPPVL DSDGSFFLYSKLTVDKSRWQQGNVFSCSVMHEALHNHYTQKSLSLSPG K |
| Full-length light chains of humanized antibodies<br><br>Hu147-11wk, Hu147-21wk, and Hu147-31wk (SEQ ID NO: 49) | *EIVLTQSPATLSLSPGERATLSC<u>SASSSVSYMH</u>WFQQKPGQSPRLLIY<u>STSNLA</u> SGIPARFSGSGSGTDYTLTISSLEPEDFAVYYC<u>QQRSSYPLT</u>FGQGTKLEIK*RT VAAPSVFIFPPSDEQLKSGTASVVCLLNNFYPREAKVQWKVDNALQSG NSQESVTEQDSKDSTYSLSSTLTLSKADYEKHKVYACEVTHQGLSSPVT KSFNRGEC<br><br>(it is also the full-length light chains of Hu147-11yk, Hu147-21yk, and Hu147-31yk) |
| Full-length light chains of humanized antibodies | *EIVLTQSPATLSLSPGERATLSC<u>SASSSVSYMH</u>WFQQKPGQSPKLLIY<u>STSNLA</u> SGIPARFSGSGSGTDYTLTISSLEPEDFAVYYC<u>QQRSSYPLT</u>FGQGTKLEIK*RT VAAPSVFIFPPSDEQLKSGTASVVCLLNNFYPREAKVQWKVDNALQSG |
| Hu147-12wk, Hu147-22wk, and Hu147-32wk (SEQ ID NO: 50) | NSQESVTEQDSKDSTYSLSSTLTLSKADYEKHKVYACEVTHQGLSSPVT KSFNRGEC<br><br>(it is also the full-length light chains of Hu147-12yk, Hu147-22yk, and Hu147-32yk) |
| Full-length light chains of humanized antibodies<br><br>Hu147-13 wk, Hu147-23wk, and Hu147-3 3wk (SEQ ID NO: 51) | *EIVLTQSPATLSLSPGERATLSC<u>SASSSVSYMH</u>WFQQKPGQSPKLWIY<u>STSNLA</u> SGVPARFSGSGSGTDYTLTISSLEPEDFAVYYC<u>QQRSSYPLT</u>FGQGTKLEIK*RT VAAPSVFIFPPSDEQLKSGTASVVCLLNNFYPREAKVQWKVDNALQSG NSQESVTEQDSKDSTYSLSSTLTLSKADYEKHKVYACEVTHQGLSSPVT KSFNRGEC<br><br>(it is also the full-length light chains of Hu147-13yk, Hu147-23yk, and Hu147-33yk) |
| Full-length light chains of humanized antibodies<br><br>Hu147-14wk, Hu147-24wk, and Hu147-34wk (SEQ ID NO: 52) | *DIQMTQSPSSLSASVGDRVTITC<u>SASSSVSYMH</u>WFQQKPGKSPKLLIY<u>STSNL AS</u>GVPSRFSGSGSGTDYTLTISSLQPEDFATYYC<u>QQRSSYPLT</u>FGQGTKLEIK*RTVAAPSVFIFPPSDEQLKSGTASVVCLLNNFYPREAKVQWKVDNALQ SGNSQESVTEQDSKDSTYSLSSTLTLSKADYEKHKVYACEVTHQGLSSP VTKSFNRGEC<br><br>(it is also the full-length light chains of Hu147-14yk, Hu147-24yk, and Hu147-34yk) |

(continued)

| Light or heavy chains of full-length antibodies (SEQ ID NO) | Amino acid sequence |
|---|---|
| Full-length light chains of humanized antibodies Hu147-15wk, Hu147-25wk, and Hu147-35wk (SEQ ID NO: 53) | *DIQLTQSPSSLSASVGDRVTITCSASSSVSYMHWFQQKPGKSPKLWIYSTSNLASGVPSRFSGSGSGTDYTLTISSLQPEDFATYYCQQRSSYPLTFGQGTKLEIK*RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC<br><br>(it is also the full-length light chains of Hu147-l5yk, Hu147-25yk, and Hu147-35yk) |
| Ch164 heavy chain (SEQ ID NO: 54) | *QVQLKQSGPGLVQPSQSLSITCTVSGFSLTTFGVHWIRQSPGKGLEWLGVIWRRGGTDYNAAFMSRLSITKDNSRSHVFFKMTSLQTDDSAIYYCARDGGFDYWGQGTTLTVSS*ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| Ch164 light chain (SEQ ID NO: 55) | *QAVVTQESALTTSPGGTVILTCRSSIGAVTTSNYANWVQEKPDHLFTGLIGGTSNRAPGVPVRFSGSLIGDKAALTITGAQAEDDAMYFCALWYSTHYVFGGGTKVTVL*GQPKANPTVTLFPPSSEELQANKATLVCLISDFYPGAVTVAWKADGSPVKAGVETTKPSKQSNNKYAASSYLSLTPEQWKSHRSYSCQVTHEGSTVEKTVAPTEC |
| Full-length heavy chains of Hu164-57wl, Hu164-58wl, and Hu164-59wl (SEQ ID NO: 56) | *EVTLKESGPVLVKPTETLTLTCTVSGFSLSTFGVHWIRQPPGKALEWLAVIWRRGGTDYNAAFMSRLTISKDTSKSQVVLTMTNMDPVDTATYYCARDGGFDYWGQGTTVTVSS*ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| Full-length heavy chains of Hu164-67wl, Hu164-68w1, and Hu164-69wl (SEQ ID NO: 57) | *EVTLKESGPVLVKPTETLTLTCTVSGFSLSTFGVHWIRQPPGKGLEWLGVIWRRGGTDYNAAFMSRLTISKDTSKSQVVFTMTNMDPVDTATYYCARDGGFDYWGQGTTVTVSS*ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRV |
| | VSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWOOGNVFSCSVMHEALHNHYTOKSLSLSPGK |

(continued)

| Light or heavy chains of full-length antibodies (SEQ ID NO) | Amino acid sequence |
|---|---|
| Full-length heavy chains of<br><br>Hu164-77wl, Hu164-78wl, and Hu164-79wl (SEQ ID NO: 58) | *EVQLQESGPGLVKPSQTLSLTCTVSGFSIS<u>TFGVH</u>WIRQHPGKGLEWIG<u>VIW RRGGTDYNAAFMS</u>RVTISKDTSKNQVSLKLSSVTAADTAVYYCARD<u>GGFDY</u> WGQGTTVTVS*SASTKGPSVFPLAPSSKSTSGGTAALGCLVKDYFPEPVTV SWNSGALTSGVHTFPAVLQSSGLYSLSSVVTVPSSSLGTQTYICNVNHKP SNTKVDKKVEPKSCDKTHTCPPCPAPELLGGPSVFLFPPKPKDTLMISRT PEVTCVVVDVSHEDPEVKFNWYVDGVEVHNAKTKPREEQYNSTYRV VSVLTVLHQDWLNGKEYKCKVSNKALPAPIEKTISKAKGQPREPQVYT LPPSRDELTKNQVSLTCLVKGFYPSDIAVEWESNGQPENNYKTTPPVLD SDGSFFLYSKLTVDKSRWOOGNVFSCSVMHEALHNHYTOKSLSLSPGK |
| Full-length heavy chains of<br><br>Hu164-87wl, Hu164-88wl, and Hu164-89wl (SEQ ID NO: 59) | *EVQLQESGPGLVKPSQTLSLTCTVSGFSIS<u>TFGVH</u>WIRQHPGKGLEWLG<u>VI WRRGGTDYNAAFMS</u>RLTISKDTSKNQVSFKLSSVTAADTAVYYCARD<u>GGFD YW</u>GQGTTVTVS*SASTKGPSVFPLAPSSKSTSGGTAALGCLVKDYFPEPVT VSWNSGALTSGVHTFPAVLQSSGLYSLSSVVTVPSSSLGTQTYICNVNH KPSNTKVDKKVEPKSCDKTHTCPPCPAPELLGGPSVFLFPPKPKDTLMIS RTPEVTCVVVDVSHEDPEVKFNWYVDGVEVHNAKTKPREEQYNSTYR VVSVLTVLHQDWLNGKEYKCKVSNKALPAPIEKTISKAKGQPREPQVY TLPPSRDELTKNQVSLTCLVKGFYPSDIAVEWESNGQPENNYKTTPPVL DSDGSFFLYSKLTVDKSRWQQGNVFSCSVMHEALHNHYTQKSLSLSPG K |
| Full-length heavy chains of<br><br>Hu164-57yl, Hu164-58yl, and Hu164-59yl (SEQ ID NO: 60) | *EVTLKESGPVLVKPTETLTLTCTVSGFSLS<u>TFGVH</u>WIRQPPGKALEWLA<u>VIW RRGGTDYNAAFMS</u>RLTISKDTSKSQVVLTMTNMDPVDTATYYCARD<u>GGFDY</u> WGQGTTVTVS*SASTKGPSVFPLAPSSKSTSGGTAALGCLVKDYFPEPVTV SWNSGALTSGVHTFPAVLQSSGLYSLSSVVTVPSSSLGTQTYICNVNHKP SNTKVDKKVEPKSCDKTHTCPPCPAPELLGGPSVFLFPPKPKDTLYITRE PEVTCVVVDVSHEDPEVKFNWYVDGVEVHNAKTKPREEQYNSTYRV VSVLTVLHQDWLNGKEYKCKVSNKALPAPIEKTISKAKGQPREPQVYT LPPSRDELTKNQVSLTCLVKGFYPSDIAVEWESNGQPENNYKTTPPVLD SDGSFFLYSKLTVDKSRWQQGNVFSCSVMHEALHNHYTQKSLSLSPGK |
| Full-length heavy chains of<br><br>Hu164-67yl, Hu164-68yl, and Hu164-69yl (SEQ ID NO: 61) | *EVTLKESGPVLVKPTETLTLTCTVSGFSLS<u>TFGVH</u>WIRQPPGKGLEWLG<u>VIW RRGGTDYNAAFMS</u>RLTISKDTSKSQVVFTMTNMDPVDTATYYCARD<u>GGFDY</u> WGQGTTVTVS*SASTKGPSVFPLAPSSKSTSGGTAALGCLVKDYFPEPVTV SWNSGALTSGVHTFPAVLQSSGLYSLSSVVTVPSSSLGTQTYICNVNHKP SNTKVDKKVEPKSCDKTHTCPPCPAPELLGGPSVFLFPPKPKDTLYITRE PEVTCVVVDVSHEDPEVKFNWYVDGVEVHNAKTKPREEQYNSTYRV VSVLTVLHQDWLNGKEYKCKVSNKALPAPIEKTISKAKGQPREPQVYT LPPSRDELTKNQVSLTCLVKGFYPSDIAVEWESNGQPENNYKTTPPVLD SDGSFFLYSKLTVDKSRWQQGNVFSCSVMHEALHNHYTQKSLSLSPGK |

(continued)

| Light or heavy chains of full-length antibodies (SEQ ID NO) | Amino acid sequence |
|---|---|
| Full-length heavy chains of Hu164-77yl, Hu164-78yl, and Hu164-79yl (SEQ ID NO: 62) | *EVQLQESGPGLVKPSQTLSLTCTVSGFSISTFGVHWIRQHPGKGLEWIGVIW RRGGTDYNAAFMSRVTISKDTSKNQVSLKLSSVTAADTAVYYCARDGGFDY WGQGTTVTVSS*ASTKGPSVFPLAPSSKSTSGGTAALGCLVKDYFPEPVTV SWNSGALTSGVHTFPAVLQSSGLYSLSSVVTVPSSSLGTQTYICNVNHKP SNTKVDKKVEPKSCDKTHTCPPCPAPELLGGPSVFLFPPKPKDTLYITRE PEVTCVVVDVSHEDPEVKFNWYVDGVEVHNAKTKPREEQYNSTYRV VSVLTVLHQDWLNGKEYKCKVSNKALPAPIEKTISKAKGQPREPQVYT LPPSRDELTKNQVSLTCLVKGFYPSDIAVEWESNGQPENNYKTTPPVLD SDGSFFLYSKLTVDKSRWQQGNVFSCSVMHEALHNHYTQKSLSLSPGK |
| Full-length heavy chains of Hu164-87yl, Hu164-88yl, and Hu164-89yl (SEQ ID NO: 63) | *EVQLQESGPGLVKPSQTLSLTCTVSGFSISTFGVHWIRQHPGKGLEWLGVI WRRGGTDYNAAFMSRLTISKDTSKNQVSFKLSSVTAADTAVYYCARDGGFD YWGQGTTVTVSS*ASTKGPSVFPLAPSSKSTSGGTAALGCLVKDYFPEPVT VSWNSGALTSGVHTFPAVLQSSGLYSLSSVVTVPSSSLGTQTYICNVNH KPSNTKVDKKVEPKSCDKTHTCPPCPAPELLGGPSVFLFPPKPKDTLYIT REPEVTCVVVDVSHEDPEVKFNWYVDGVEVHNAKTKPREEQYNSTYR |
| | VVSVLTVLHQDWLNGKEYKCKVSNKALPAPIEKTISKAKGQPREPQVY TLPPSRDELTKNQVSLTCLVKGFYPSDIAVEWESNGQPENNYKTTPPVL DSDGSFFLYSKLTVDKSRWQQGNVFSCSVMHEALHNHYTQKSLSLSPG K |
| Full-length light chains of Hu164-57wl, Hu164-67wl, Hu164-77wl, and Hu164-87wl (SEQ ID NO: 64) | *ETVVTQEPSLTVSPGGTVTLTCRSSIGAVTTSNYANWVQQKPGQAFRGLIGG TSNRAPWTPARFSGSLLGGKAALTLSGVQPEDEAEYYCALWYSTHYVFGGG TKLTVL*GQPKANPTVTLFPPSSEELQANKATLVCLISDFYPGAVTVAWKA DGSPVKAGVETTKPSKQSNNKYAASSYLSLTPEQWKSHRSYSCQVTHE GSTVEKTVAPTEC<br>(it is also the full-length light chains of Hu164-57yl, Hu164-67yl, Hu164-77yl, and Hu164-87yl) |
| Full-length light chains of Hu164-58wl, Hu164-68wl, Hu164-78w1, and Hu164-88wl (SEQ ID NO: 65) | *ETVVTQEPSFSVSPGGTVTLTCRSSIGAVTTSNYANWVQQTPGQAFRGLIGG TSNRAPGVPDRFSGSILGNKAALTITGAQADDESDYYCALWYSTHYVFGGGT KLTVL*GQPKANPTVTLFPPSSEELQANKATLVCLISDFYPGAVTVAWKA DGSPVKAGVETTKPSKQSNNKYAASSYLSLTPEQWKSHRSYSCQVTHE GSTVEKTVAPTEC<br>(it is also the full-length light chains of Hu164-58yl, Hu164-68yl, Hu164-78yl, and Hu164-88yl) |
| Full-length light chains of Hu164-59wl, Hu164-69wl, Hu164-79wl, and Hu164-89wl (SEQ ID NO: 66) | *ESVVTQPPSVSGAPGQRVTISCRSSIGAVTTSNYANWVQQLPGTAFKGLIGGT SNRAPGVPDRFSGSKSGTSASLAITGLQAEDEADYYCALWYSTHYVFGGGTK LTVL*GQPKANPTVTLFPPSSEELQANKATLVCLISDFYPGAVTVAWKAD GSPVKAGVETTKPSKQSNNKYAASSYLSLTPEQWKSHRSYSCQVTHEG STVEKTVAPTEC<br>(it is also the full-length light chains of Hu164-59yl, Hu164-69yl, Hu164-79yl, and Hu164-89yl) |

(continued)

| Light or heavy chains of full-length antibodies (SEQ ID NO) | Amino acid sequence |
|---|---|
| Ch95 heavy chain (SEQ ID NO: 86) | *EVLLQQSGPVLVKPGPSVKISCKASGFTFTNYDIHWVKQSHGKSLEWIGLVY PYNGGTAYNQKFKDKATLTFDTSSSTAYMELNSLTSEDSAVYYCARWGLIPGT TSYFDVWGTGTTVTVSS*ASTKGPSVFPLAPSSKSTSGGTAALGCLVKDYF PEPVTVSWNSGALTSGVHTFPAVLQSSGLYSLSSVVTVPSSSLGTQTYIC NVNHKPSNTKVDKKVEPKSCDKTHTCPPCPAPELLGGPSVFLFPPKPKD TLMISRTPEVTCVVVDVSHEDPEVKFNWYVDGVEVHNAKTKPREEQY NSTYRVVSVLTVLHQDWLNGKEYKCKVSNKALPAPIEKTISKAKGQPR EPQVYTLPPSRDELTKNQVSLTCLVKGFYPSDIAVEWESNGQPENNYKT TPPVLDSDGSFFLYSKLTVDKSRWQQGNVFSCSVMHEALHNHYTQKSL SLSPGK |
| Ch95 light chain (SEQ ID NO: 87) | *QIVLSQSPPILSASPGEKVTMTCRASSSVSYIHWYQQKPRSSPKPWIYATSNLA SGVPARFSGSGSGTSYSLTISRVEAEDAATYYCQQWNSNPWTFGGGTRLEIK*RTVAAPSVFIFPPSDEQLKSGTASVVCLLNNFYPREAKVQWKVDNALQ SGNSQESVTEQDSKDSTYSLSSTLTLSKADYEKHKVYACEVTHQGLSSP VTKSFNRGEC |
| Full-length heavy chains of Hu95-11wk, Hu95-12wk, Hu95-13wk, and Hu95-14wk (SEQ ID NO: 88) | *EVQLVQSGAEVKKPGASVKVSCKASGFTFTNYDIHWVRQAPGQRLEWMGL VYPYNGGTAYNQKFKDRVTLTFDTSASTAYMELSSLRSEDTAVYYCARWGLIP GTTSYFDVWGQGTTVTVSS*ASTKGPSVFPLAPSSKSTSGGTAALGCLVKD YFPEPVTVSWNSGALTSGVHTFPAVLQSSGLYSLSSVVTVPSSSLGTQTY ICNVNHKPSNTKVDKKVEPKSCDKTHTCPPCPAPELLGGPSVFLFPPKP KDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQ PREPQVYTLPPSRDELTKNQVSLTCLVKGFYPSDIAVEWESNGQPENNY KTTPPVLDSDGSFFLYSKLTVDKSRWQQGNVFSCSVMHEALHNHYTQK SLSLSPGK |
| Full-length heavy chains of Hu95-21wk, Hu95-22wk, Hu95-23wk, and | *EVQLVQSGAEVKKPGASVKVSCKASGFTFTNYDIHWVRQAPGQRLEWIGLV YPYNGGTAYNQKFKDRATLTFDTSASTAYMELSSLRSEDTAVYYCARWGLIPG TTSYFDVWGQGTTVTVSS*ASTKGPSVFPLAPSSKSTSGGTAALGCLVKDY FPEPVTVSWNSGALTSGVHTFPAVLQSSGLYSLSSVVTVPSSSLGTQTYI CNVNHKPSNTKVDKKVEPKSCDKTHTCPPCPAPELLGGPSVFLFPPKPK |
| Hu95-24wk (SEQ ID NO: 89) | DTLMISRTPEVTCVVVDVSHEDPEVKFNWYVDGVEVHNAKTKPREEQ YNSTYRVVSVLTVLHQDWLNGKEYKCKVSNKALPAPIEKTISKAKGQP REPQVYTLPPSRDELTKNQVSLTCLVKGFYPSDIAVEWESNGQPENNYK TTPPVLDSDGSFFLYSKLTVDKSRWQQGNVFSCSVMHEALHNHYTQKS LSLSPGK |
| Full-length heavy chains of Hu95-31wk, Hu95-32wk, Hu95-33wk, and Hu95-34wk (SEQ ID NO: 90) | *EVQLVQSGAEVKKPGASVKVSCKASGFTFTNYDIHWVKQAPGQRLEWMGL VYPYNGGTAYNQKFKDKVTLTFDTSASTAYMELSSLRSEDTAVYYCARWGLIP GTTSYFDVWGQGTTVTVSS*ASTKGPSVFPLAPSSKSTSGGTAALGCLVKD YFPEPVTVSWNSGALTSGVHTFPAVLQSSGLYSLSSVVTVPSSSLGTQTY ICNVNHKPSNTKVDKKVEPKSCDKTHTCPPCPAPELLGGPSVFLFPPKP KDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQ PREPQVYTLPPSRDELTKNQVSLTCLVKGFYPSDIAVEWESNGQPENNY KTTPPVLDSDGSFFLYSKLTVDKSRWQQGNVFSCSVMHEALHNHYTQK SLSLSPGK |

(continued)

| Light or heavy chains of full-length antibodies (SEQ ID NO) | Amino acid sequence |
|---|---|
| Full-length heavy chains of Hu95-41wk, Hu95-42wk, Hu95-43wk, and Hu95-44wk (SEQ ID NO: 91) | *EVQLVQSGAEVKKPGASVKVSCKASGFTFTNYDIHWVKQAPGQRLEWIGLV YPYNGGTAYNQKFKDKATLTFDTSASTAYMELSSLRSEDTAVYYCARWGLIPG TTSYFDVWGQGTTVTVSS*ASTKGPSVFPLAPSSKSTSGGTAALGCLVKDY FPEPVTVSWNSGALTSGVHTFPAVLQSSGLYSLSSVVTVPSSSLGTQTYI CNVNHKPSNTKVDKKVEPKSCDKTHTCPPCPAPELLGGPSVFLFPPKPK DTLMISRTPEVTCVVVDVSHEDPEVKFNWYVDGVEVHNAKTKPREEQ YNSTYRVVSVLTVLHQDWLNGKEYKCKVSNKALPAPIEKTISKAKGQP REPQVYTLPPSRDELTKNQVSLTCLVKGFYPSDIAVEWESNGQPENNYK TTPPVLDSDGSFFLYSKLTVDKSRWQQGNVFSCSVMHEALHNHYTQKS LSLSPGK |
| Full-length heavy chains of Hu95-51wk, Hu95-52wk, Hu95-53wk, and Hu95-54wk (SEQ ID NO: 92) | *EVQLVQSGAEVKKPGASVKVSCRASGFTFTNYAIHWVKQAPGQRLEWMGL VYPYTGGTAYNQKFKDKVTLTFDTSASTAYMELSSLRSEDTAVYYCARWGMI PGTNSYFDVWGQGTTVTVSS*ASTKGPSVFPLAPSSKSTSGGTAALGCLVK DYFPEPVTVSWNSGALTSGVHTFPAVLQSSGLYSLSSVVTVPSSSLGTQT YICNVNHKPSNTKVDKKVEPKSCDKTHTCPPCPAPELLGGPSVFLFPPK PKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKG QPREPQVYTLPPSRDELTKNQVSLTCLVKGFYPSDIAVEWESNGQPENN YKTTPPVLDSDGSFFLYSKLTVDKSRWQQGNVFSCSVMHEALHNHYTQ KSLSLSPGK |
| Full-length heavy chains of Hu95-11yk, Hu95-12yk, Hu95-13yk, and Hu95-14yk (SEQ ID NO: 93) | *EVQLVQSGAEVKKPGASVKVSCKASGFTFTNYDIHWVRQAPGQRLEWMGL VYPYNGGTAYNQKFKDRVTLTFDTSASTAYMELSSLRSEDTAVYYCARWGLIP GTTSYFDVWGQGTTVTVSS*ASTKGPSVFPLAPSSKSTSGGTAALGCLVKD YFPEPVTVSWNSGALTSGVHTFPAVLQSSGLYSLSSVVTVPSSSLGTQTY ICNVNHKPSNTKVDKKVEPKSCDKTHTCPPCPAPELLGGPSVFLFPPKP KDTLYITREPEVTCVVVDVSHEDPEVKFNWYVDGVEVHNAKTKPREE QYNSTYRVVSVLTVLHQDWLNGKEYKCKVSNKALPAPIEKTISKAKGQ PREPQVYTLPPSRDELTKNQVSLTCLVKGFYPSDIAVEWESNGQPENNY KTTPPVLDSDGSFFLYSKLTVDKSRWQQGNVFSCSVMHEALHNHYTQK SLSLSPGK |
| Full-length heavy chains of Hu95-21yk, Hu95-22yk, Hu95-23yk, and Hu95-24yk (SEQ ID NO: 94) | *EVQLVQSGAEVKKPGASVKVSCKASGFTFTNYDIHWVRQAPGQRLEWIGLV YPYNGGTAYNQKFKDRATLTFDTSASTAYMELSSLRSEDTAVYYCARWGLIPG TTSYFDVWGQGTTVTVSS*ASTKGPSVFPLAPSSKSTSGGTAALGCLVKDY FPEPVTVSWNSGALTSGVHTFPAVLQSSGLYSLSSVVTVPSSSLGTQTYI CNVNHKPSNTKVDKKVEPKSCDKTHTCPPCPAPELLGGPSVFLFPPKPK DTLYITREPEVTCVVVDVSHEDPEVKFNWYVDGVEVHNAKTKPREEQ YNSTYRVVSVLTVLHQDWLNGKEYKCKVSNKALPAPIEKTISKAKGQP REPQVYTLPPSRDELTKNQVSLTCLVKGFYPSDIAVEWESNGQPENNYK TTPPVLDSDGSFFLYSKLTVDKSRWQQGNVFSCSVMHEALHNHYTQKS |
| | LSLSPGK |

(continued)

| Light or heavy chains of full-length antibodies (SEQ ID NO) | Amino acid sequence |
|---|---|
| Full-length heavy chains of Hu95-31yk, Hu95-32yk, Hu95-33yk, and Hu95-34yk (SEQ ID NO: 95) | *EVQLVQSGAEVKKPGASVKVSCKASGFTFTNYDIHWVKQAPGQRLEWMGL VYPYNGGTAYNQKFKDKVTLTFDTSASTAYMELSSLRSEDTAVYYCARWGLIP GTTSYFDV*WGQGTTVTVSSASTKGPSVFPLAPSSKSTSGGTAALGCLVKD YFPEPVTVSWNSGALTSGVHTFPAVLQSSGLYSLSSVVTVPSSSLGTQTY ICNVNHKPSNTKVDKKVEPKSCDKTHTCPPCPAPELLGGPSVFLFPPKP KDTLYITREPEVTCVVVDVSHEDPEVKFNWYVDGVEVHNAKTKPREE QYNSTYRVVSVLTVLHQDWLNGKEYKCKVSNKALPAPIEKTISKAKGQ PREPQVYTLPPSRDELTKNQVSLTCLVKGFYPSDIAVEWESNGQPENNY KTTPPVLDSDGSFFLYSKLTVDKSRWQQGNVFSCSVMHEALHNHYTQK SLSLSPGK |
| Full-length heavy chains of Hu95-41yk, Hu95-42yk, Hu95-43yk, and Hu95-44yk (SEQ ID NO: 96) | *EVQLVQSGAEVKKPGASVKVSCKASGFTFTNYDIHWVKQAPGQRLEWIGLV YPYNGGTAYNQKFKDKATLTFDTSASTAYMELSSLRSEDTAVYYCARWGLIPG TTSYFDV*WGQGTTVTVSSASTKGPSVFPLAPSSKSTSGGTAALGCLVKDY FPEPVTVSWNSGALTSGVHTFPAVLQSSGLYSLSSVVTVPSSSLGTQTYI CNVNHKPSNTKVDKKVEPKSCDKTHTCPPCPAPELLGGPSVFLFPPKPK DTLYITREPEVTCVVVDVSHEDPEVKFNWYVDGVEVHNAKTKPREEQ YNSTYRVVSVLTVLHQDWLNGKEYKCKVSNKALPAPIEKTISKAKGQP REPQVYTLPPSRDELTKNQVSLTCLVKGFYPSDIAVEWESNGQPENNYK TTPPVLDSDGSFFLYSKLTVDKSRWQQGNVFSCSVMHEALHNHYTQKS LSLSPGK |
| Full-length heavy chains of Hu95-51yk, Hu95-52yk, Hu95-53yk, and Hu95-54yk (SEQ ID NO: 97) | *EVQLVQSGAEVKKPGASVKVSCRASGFTFTNYAIHWVKQAPGQRLEWMGL VYPYTGGTAYNQKFKDKVTLTFDTSASTAYMELSSLRSEDTAVYYCARWGMI PGTNSYFDV*WGQGTTVTVSSASTKGPSVFPLAPSSKSTSGGTAALGCLVK DYFPEPVTVSWNSGALTSGVHTFPAVLQSSGLYSLSSVVTVPSSSLGTQT YICNVNHKPSNTKVDKKVEPKSCDKTHTCPPCPAPELLGGPSVFLFPPK PKDTLYITREPEVTCVVVDVSHEDPEVKFNWYVDGVEVHNAKTKPRE EQYNSTYRVVSVLTVLHQDWLNGKEYKCKVSNKALPAPIEKTISKAKG QPREPQVYTLPPSRDELTKNQVSLTCLVKGFYPSDIAVEWESNGQPENN YKTTPPVLDSDGSFFLYSKLTVDKSRWQQGNVFSCSVMHEALHNHYTQ KSLSLSPGK |
| Full-length light chains of Hu95-11wk, Hu95-21wk, Hu95-31wk, Hu95-41wk, and Hu95-51wk (SEQ ID NO: 98) | *EIVLTQSPATLSLSPGERATLSCRASSSVSYIHWYQQKPGQAPRPWIYATSNLA SGIPARFSGSGSGTDYTLTISRLEPEDFAVYYCQQWNSNPWT*FGGGTKVEIK RTVAAPSVFIFPPSDEQLKSGTASVVCLLNNFYPREAKVQWKVDNALQ SGNSQESVTEQDSKDSTYSLSSTLTLSKADYEKHKVYACEVTHQGLSSP VTKSFNRGEC <br><br> (it is also the full-length light chains of Hu9S-11yk, Hu95-21yk, Hu95-31yk, Hu95-41yk, and Hu95-51yk) |
| Full-length light chains of Hu95-12wk, Hu95-22wk, Hu95-32wk, Hu95-42wk, and Hu95-52wk (SEQ ID NO: 99) | *EIVLTQSPATLSLSPGERATLSCRASSSVSYIHWYQQKPGQSPRPWIYATSNLA SGVPARFSGSGSGTSYTLTISRLEPEDFAVYYCQQWNSNPWT*FGGGTKVEIK RTVAAPSVFIFPPSDEQLKSGTASVVCLLNNFYPREAKVQWKVDNALQ SGNSQESVTEQDSKDSTYSLSSTLTLSKADYEKHKVYACEVTHQGLSSP VTKSFNRGEC <br><br> (it is also the full-length light chains of Hu95-12yk, Hu95-22yk, Hu95-32yk, Hu95-42yk, and Hu95-52yk) |

(continued)

| Light or heavy chains of full-length antibodies (SEQ ID NO) | Amino acid sequence |
| --- | --- |
| Full-length light chains of Hu95-13wk, Hu95-23wk, Hu95-33wk, Hu95-43wk, and Hu95-53wk (SEQ ID NO: 100) | *ESVLTQPPSVSGAPGQRVTISCRASSSVSYIHWYQQLPGTAPKPWIYATSNLAS GVPDRFSGSKSGTSYSLAITGLQAEDEADYYCQQWNSNPWTFGGGTKVEIK* RTVAAPSVFIFPPSDEQLKSGTASVVCLLNNFYPREAKVQWKVDNALQ SGNSQESVTEQDSKDSTYSLSSTLTLSKADYEKHKVYACEVTHQGLSSP VTKSFNRGEC <br><br> (it is also the full-length light chains of Hu95-13yk, Hu95-23yk, Hu95-33yk, Hu95-43yk, and Hu95-53yk) |
| Full-length light chains of Hu95-14wk, Hu95-24wk, Hu95-34wk, Hu95-44wk, and Hu95-54wk (SEQ ID NO: 101) | *EIVLTQPPSVSGAPGQRVTISCRASSSVSYIHWYQQLPGTSPKPWIYATSNLAS GVPDRFSGSKSGTSYSLAITGLQAEDEADYYCQQWNSNPWTFGGGTKVEIK* RTVAAPSVFIFPPSDEQLKSGTASVVCLLNNFYPREAKVQWKVDNALQ SGNSQESVTEQDSKDSTYSLSSTLTLSKADYEKHKVYACEVTHQGLSSP VTKSFNRGEC <br><br> (it is also the full-length light chains of Hu95-14yk, Hu95-24yk, Hu95-34yk, Hu95-44yk, and Hu95-54yk) |

The sequence of the CTGF antibody mAb1 (from CN1829740B under the name pamrevlumab, also known as FG-3019) as a positive control is as follows:

Heavy chain: (SEQ ID NO: 67)

EGQLVQSGGGLVHPGGSLRLSCAGSGFTFSSYGMHWVRQAPGKGLEWVSGIGT
GGGTYSTDSVKGRFTISRDNAKNSLYLQMNSLRAEDMAVYYCARGDYYGSGS
FFDCWGQGTLVTVSSASTKGPSVFPLAPSSKSTSGGTAALGCLVKDYFPEPVTVS
WNSGALTSGVHTFPAVLQSSGLYSLSSVVTVPSSSLGTQTYICNVNHKPSNTKVD
KRVEPKSCDKTHTCPPCPAPELLGGPSVFLFPPKPKDTLMISRTPEVTCVVVDVS
HEDPEVKFNWYVDGVEVHNAKTKPREEQYNSTYRVVSVLTVLHQDWLNGKE
YKCKVSNKALPAPIEKTISKAKGQPREPQVYTLPPSREEMTKNQVSLTCLVKGF
YPSDIAVEWESNGQPENNYKTTPPVLDSDGSFFLYSKLTVDKSRWQQGNVFSCS
VMHEALHNHYTQKSLSLSPGK

Light chain: (SEQ ID NO: 68)

DIQMTQSPSSLSASVGDRVTITCRASQGISSWLAWYQQKPEKAPKSLIYAASSLQ
SGVPSRFSGSGSGTDFTLTISSLQPEDFATYYCQQYNSYPPTFGQGTKLEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC

**Examples** 1-3. Assays **for Binding Activity of CTGF antibodies**

**I.** ELISA assays **of CTGF antibodies binding to human CTGF protein**

The binding abilities of anti-human CTGF antibodies were measured by ELISA assays of the antibodies and human CTGF protein and Biacore. A 96-well microplate was directly coated with CTGF recombinant protein. The intensity of the signal after an antibody was added was used to measure the binding activity of the antibody to CTGF. The specific experimental method is as follows:

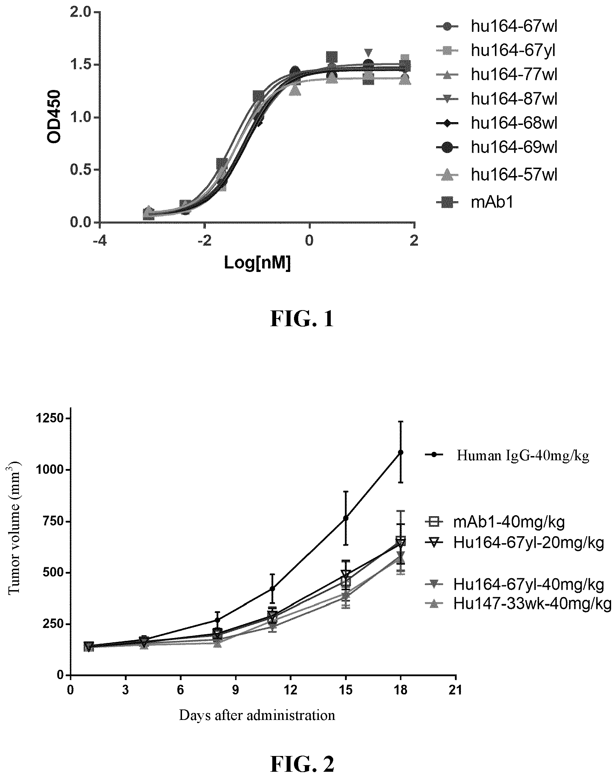

CTGF protein (R&D, Cat No. 9190-CC) was diluted to a concentration of 0.2 $\mu$g/mL with PBS (Shanghai BasalMedia, Cat No. B320) buffer having a pH of 7.4, and added to a 96-well microplate (Corning, Cat No. CLS3590-100EA) at a volume of 50 $\mu$L/well. The plate was left to stand in an incubator at 37 °C for 2 h or at 4 °C overnight (16-18 h). After the liquid was discarded, a PBS-diluted 5% skim milk (BD skim milk, Cat No. 232100) blocking solution was added at 250 $\mu$L/well, and the plate was incubated in an incubator at 37 °C for 3 h or left to stand at 4 °C overnight (16-18 h) for blocking. After blocking, the blocking solution was discarded, and the plate was washed 5 times with PBST buffer (PBS containing 0.1% Tween-20, pH 7.4). Then test antibodies (hybridoma purified antibodies or humanized antibodies) that had been diluted with a sample diluent to different concentrations were added at 50 $\mu$L/well, and the plate was incubated in a 37 °C incubator for 1 h. After incubation, the plate was washed 3 times with PBST, HRP-labeled goat anti-mouse secondary antibody (Jackson Immuno Research, Cat No. 115-035-003) or goat anti-human secondary antibody (Jackson Immuno Research, Cat No. 109-035-003) that had been diluted with a sample diluent was added at 50 $\mu$L/well, and the plate was incubated at 37 °C for 1 h. After the plate was washed 3 times with PBST, TMB chromogenic substrate (KPL, Cat No. 52-00-03) was added at 50 $\mu$L/well, and the plate was incubated at room temperature for 5-10 min. The reaction was stopped by adding 1 M $H_2SO_4$ at 50 $\mu$L/well. Absorbance readings were taken at the wavelength of 450 nm on a microplate reader (Molecular Devices, VERSA max). The data were analyzed using GraphPad Prism 5, and the EC50 values of the CTGF antibodies binding to human CTGF protein were calculated. The experimental results are shown in FIG. 1 and Table 13.

Table 13. The results of the ELISA assays of CTGF antibodies binding to human CTGF protein

| Test sample | ELISA assay EC50 of binding to human CTGF protein (nM) | Test sample | ELISA assay EC50 of binding to human CTGF protein (nM) |
|---|---|---|---|
| Hu147-11wk | 0.045 | Hu164-57wl | 0.044 |
| Hu147-12wk | 0.046 | Hu164-67wl | 0.044 |
| Hu147-13wk | 0.043 | Hu164-67yl | 0.060 |
| Hu147-14wk | 0.073 | Hu164-77wl | 0.055 |
| Hu147-15wk | 0.034 | Hu164-87wl | 0.055 |
| Hu147-21wk | 0.067 | Hu164-68wl | 0.069 |
| Hu147-22wk | 0.056 | Hu164-69wl | 0.063 |
| Hu147-23wk | 0.058 | mAb1 | 0.067 |
| Hu147-24wk | 0.048 | Hu95-51yk | 0.080 |
| Hu147-31wk | 0.056 | | |
| Hu147-32wk | 0.063 | | |
| Hu147-33wk | 0.054 | | |
| Hu147-34wk | 0.134 | | |

The experimental results show that all the humanized full-length anti-CTGF antibodies of the present disclosure have a good binding effect on human CTGF protein.

**II. ELISA assays of CTGF antibodies binding to cynomolgus monkey CTGF protein**

The binding abilities of anti-monkey CTGF antibodies were measured by ELISA assays of the antibodies and monkey CTGF protein. A 96-well microplate was directly coated with anti-mFc protein. Subsequently, Fc-tagged cyno-CTGF protein was allowed to bind to the plate. Then an antibody was added. The intensity of the signal after the addition was used to measure the binding activity of the antibody to cyno-CTGF. The specific experimental method is as follows: Anti-mFc protein (Sigma, Cat No. M4280) was diluted to a concentration of 2 μg/mL with PBS (Shanghai BasalMedia, Cat No. B320) buffer having a pH of 7.4, and added to a 96-well microplate (Corning, Cat No. CLS3590-100EA) at a volume of 50 μL/well. The plate was left to stand in an incubator at 37 °C for 2 h. After the liquid was discarded, a PBS-diluted 5% skim milk (BD skim milk, Cat No. 232100) blocking solution was added at 250 μL/well, and the plate was incubated in an incubator at 37 °C for 3 h or left to stand at 4 °C overnight (16-18 h) for blocking. After blocking, the blocking solution was discarded, and the plate was washed 3 times with PBST buffer (PBS containing 0.1% Tween-20, pH 7.4). Then 50 μL of 1 μg/mLcyno-CTGF-mFc protein was added to each well. The plate was incubated in a 37 °C incubator for 1 h and then washed 3 times with PBST buffer (PBS containing 0.1% Tween-20, pH 7.4). Then test antibodies (hybridoma purified antibodies or humanized antibodies) that had been diluted with a sample diluent to different concentrations were added at 50 μL/well, and the plate was incubated in a 37 °C incubator for 1 h. After incubation, the plate was washed 3 times with PBST, HRP-labeled goat anti-mouse secondary antibody (Jackson Immuno Research, Cat No. 115-035-003) or goat anti-human secondary antibody (Jackson Immuno Research, Cat No. 109-035-003) that had been diluted with a sample diluent was added at 50 μL/well, and the plate was incubated at 37 °C for 1 h. After the plate was washed 5 times with PBST, TMB chromogenic substrate (KPL, Cat No. 52-00-03) was added at 50 μL/well, and the plate was incubated at room temperature for 5-10 min. The reaction was stopped by adding 1 M $H_2SO_4$ at 50 μL/well. Absorbance readings were taken at the wavelength of 450 nm on a microplate reader (Molecular Devices, VERSA max). The data were analyzed using GraphPad Prism 5, and the EC50 values of the CTGF antibodies binding to cynomolgus monkey CTGF were calculated. The experimental results are shown in Table 14.

Table 14. The results of the ELISA assays of CTGF antibodies binding to cynomolgus monkey CTGF protein

| Test sample | EC50 (nM) | Test sample | EC50 (nM) | Test sample | EC50 (nM) |
|---|---|---|---|---|---|
| Hu147-11wk | 0.052 | Hu147-23wk | 0.063 | Hu164-57wl | 0.029 |
| Hu147-12wk | 0.062 | Hu147-24wk | 0.080 | Hu164-67wl | 0.028 |
| Hu147-13wk | 0.043 | Hu147-31wk | 0.070 | Hu164-77wl | 0.038 |
| Hu147-14wk | 0.077 | Hu147-32wk | 0.079 | Hu164-87wl | 0.034 |
| Hu147-15wk | 0.053 | Hu147-3 3wk | 0.044 | Hu164-68wl | 0.036 |
| Hu147-21wk | 0.053 | Hu147-34wk | 0.144 | Hu164-69wl | 0.032 |
| Hu147-22wk | 0.073 | Hu95-51yk | 0.059 | mAb1 | 0.086 |

The experimental results show that all the humanized anti-CTGF antibodies of the present disclosure have a good binding effect on monkey CTGF protein.

**III. ELISA assays of CTGF antibodies and mouse CTGF protein**

The binding abilities of anti-mouse CTGF antibodies were measured by ELISA assays of the antibodies and mouse CTGF protein. A 96-well microplate was directly coated with mouse-CTGF fusion protein. The intensity of the signal after an antibody was added was used to measure the binding activity of the antibody to mouse-CTGF. The specific experimental method is as follows:
Mouse CTGF-his protein was diluted to a concentration of 0.5 μg/mL with PBS (Shanghai BasalMedia, Cat No. B320) buffer having a pH of 7.4, and added to a 96-well microplate (Corning, Cat No. CLS3590-100EA) at a volume of 50 μL/well. The plate was left to stand in an incubator at 37 °C for 2 h. After the liquid was discarded, a PBS-diluted 5% skim milk (BD skim milk, Cat No. 232100) blocking solution was added at 250 μL/well, and the plate was incubated in an incubator at 37 °C for 3 h or left to stand at 4 °C overnight (16-18 h) for blocking. After blocking, the blocking solution was discarded, and the plate was washed 3 times with PBST buffer (pH 7.4 PBS containing 0.1% Tween-20). Then test antibodies (hybridoma purified antibodies or humanized antibodies) that had been diluted with a sample diluent to different concentrations were added at 50 μL/well, and the plate was incubated in a 37 °C incubator for 1 h. After incubation, the plate was washed 3 times with PBST, HRP-labeled goat anti-mouse secondary antibody (Jackson Immuno Research, Cat No. 115-035-003) or goat anti-human secondary antibody (Jackson Immuno Research, Cat No. 109-035-003) that had been diluted with a sample diluent was added at 50 μL/well, and the plate was incubated at 37 °C for 1 h. After the plate was washed 5 times with PBST, TMB chromogenic substrate (KPL, Cat No. 52-00-03) was added at 50 μL/well, and the plate was incubated at room temperature for 5-10 min. The reaction was stopped by adding 1 M $H_2SO_4$ at 50 μL/well. Absorbance readings were taken at the wavelength of 450 nm on a microplate reader (Molecular Devices, VERSA max). The data were analyzed using GraphPad Prism 5, and the EC50 values of the CTGF antibodies binding to mouse CTGF were calculated. The experimental results are shown in Table 15.

Table 15. The results of the ELISA assays of CTGF antibodies binding to mouse CTGF protein

| Test sample | EC50 (nM) | Test sample | EC50 (nM) | Test sample | EC50 (nM) |
|---|---|---|---|---|---|
| Hu147-11wk | 0.228 | Hu147-23wk | 0.200 | Hu164-57wl | 0.041 |
| Hu147-12wk | 0.249 | Hu147-24wk | 0.263 | Hu164-67wl | 0.049 |
| Hu147-13wk | 0.127 | Hu147-31wk | 0.320 | Hu164-77wl | 0.058 |
| Hu147-14wk | 0.431 | Hu147-32wk | 0.349 | Hu164-87wl | 0.067 |
| Hu147-15wk | 0.159 | Hu147-3 3wk | 0.258 | Hu164-68wl | 0.065 |
| Hu147-21wk | 0.247 | Hu147-34wk | 0.664 | Hu164-69wl | 0.059 |
| Hu147-22wk | 0.336 | | | mAb1 | 0.077 |

The experimental results show that all the humanized full-length anti-CTGF antibodies of the present disclosure have a good binding effect on mouse CTGF protein.

**Examples 1-4. Function-Blocking Experiment with CTGF Antibodies**

In this experiment, the activity of the test humanized anti-CTGF antibodies blocking human TGF-β1 function was measured using a Biacore instrument.

The antigen human CTGF was firstly fixed onto a CM5 biosensor chip (Cat. # BR-1005-30, GE) by amino coupling at a level of 1500 RU. Then high-concentration CTGF antibody (150 μg/ml) was allowed to flow over the surface of the chip for 150 s, and 50 nM TGF-β1 protein was injected for 120 s. The reaction signals were detected in real time using a Biacore T200 instrument to obtain an association and dissociation curve. After dissociation was complete in each test cycle, the biosensor chip was washed thoroughly and regenerated with a regeneration buffer.

The data obtained from the experiment were statistically analyzed using GE Biacore T200 Evaluation version 3.0 software.

Blocking efficiency = [1 - (sample response value / blank control response value)] × 100%.

The specific results are shown in Table 16.

The specific results are shown in Table 16.

Table 16. The results of the function-blocking experiment with CTGF antibodies

| Test sample | Percent function blocking (%) | Test sample | Percent function blocking (%) |
|---|---|---|---|
| Hu147-11wk | 50.8% | Hu147-24wk | 52.8% |
| Hu147-12wk | 51.6% | Hu147-31wk | 55.0% |
| Hu147-13wk | 53.3% | Hu147-32wk | 55.2% |
| Hu147-14wk | 50.9% | Hu147-3 3wk | 57.3% |
| Hu147-15wk | 52.3% | Hu147-34wk | 56.2% |
| Hu147-21wk | 51.6% | Hu164-67wl | 55.5% |
| Hu147-22wk | 52.3% | Hu164-67yl | 53.9% |
| Hu147-23wk | 54.6% | mAb1 | 41.3% |

The experimental results show that all the humanized anti-CTGF antibodies of the present disclosure have high function-blocking activity.

**Biacore assays for affinities of anti-CTGF antibodies**

In the assays, the affinities of the test humanized anti-CTGF antibodies for human CTGF and mouse CTGF were measured using a Biacore instrument.

A Protein A biosensor chip (Cat. # 29127556, GE) was allowed to affinity capture a certain amount of a test antibody, and then a certain concentration of human or mouse CTGF antigen was allowed to flow over the surface of the chip. The reaction signals were detected in real time using a Biacore instrument to obtain an association and dissociation curve. After each cycle of dissociation was complete, the biochip was washed thoroughly and regenerated with a pH 1.5 glycine-hydrochloric acid regeneration solution (Cat. # BR-1003-54, GE). The running buffer for the experiment was 1×HBS-EP buffer (Cat. # BR-1001-88, GE).

The data obtained from the experiment were fitted using GE Biacore T200 Evaluation version 3.0 software with the (1:1) Langmuir model to obtain affinity values, which are shown in detail in Tables 17 and 18.

Table 17. The reaction affinities of CTGF antibodies for human CTGF protein

| Antibody | ka (1/Ms) | kd (1/s) | KD (M) |
|---|---|---|---|
| Ch147 | 1.49E+07 | 6.93E-03 | 4.66E-10 |
| Ch164 | 6.62E+07 | 8.83E-03 | 1.33E-10 |
| Ch95 | 7.85E+06 | 3.02E-02 | 3.85E-09 |
| Hu147-11wk | 2.98E+07 | 3.36E-02 | 1.13E-09 |
| Hu147-12wk | 3.41E+07 | 3.30E-02 | 9.69E-10 |
| Hu147-13wk | 2.97E+07 | 2.40E-02 | 8.08E-10 |
| Hu147-14wk | 4.89E+07 | 5.54E-02 | 1.13E-09 |
| Hu147-21wk | 1.66E+07 | 1.62E-02 | 9.74E-10 |
| Hu147-22wk | 1.40E+07 | 1.42E-02 | 1.01E-09 |
| Hu147-23wk | 1.37E+07 | 1.12E-02 | 8.18E-10 |
| Hu147-31wk | 1.78E+07 | 1.59E-02 | 8.94E-10 |
| Hu147-32wk | 1.46E+07 | 1.33E-02 | 9.10E-10 |
| Hu147-33wk | 1.45E+07 | 1.04E-02 | 7.13E-10 |
| Hu147-34wk | 1.47E+07 | 1.55E-02 | 1.05E-09 |
| Hu164-57wl | 4.26E+07 | 2.94E-03 | 6.91E-11 |
| Hu164-67wl | 5.17E+07 | 1.96E-03 | 3.79E-11 |
| Hu164-67yl | 3.43E+07 | 2.27E-03 | 6.62E-11 |
| Hu164-77wl | 5.15E+07 | 1.76E-02 | 3.43E-10 |
| Hu164-87wl | 1.05E+08 | 2.04E-02 | 1.94E-10 |
| Hu164-68wl | 3.84E+07 | 1.16E-02 | 3.02E-10 |
| Hu164-69wl | 8.71E+07 | 2.80E-02 | 3.22E-10 |
| Hu95-21wk | 4.09E+06 | 3.03E-02 | 7.39E-09 |
| Hu95-31wk | 5.59E+06 | 3.26E-02 | 5.84E-09 |
| Hu95-41wk | 7.70E+06 | 4.08E-02 | 5.29E-09 |
| Hu95-42wk | 3.32E+06 | 1.91E-02 | 5.74E-09 |
| Hu95-51yk | 2.84E+07 | 1.02E-03 | 3.58E-11 |

Table 18. The reaction affinities of CTGF antibodies for mouse CTGF protein

| Antibody | ka (1/Ms) | kd (1/s) | KD (M) |
|----------|-----------|----------|--------|
| Ch147 | 3.12E+07 | 3.84E-02 | 1.23E-09 |
| Ch164 | 1.23E+08 | 1.01E-02 | 8.22E-11 |
| Ch95 | 1.38E+07 | 2.35E-02 | 1.71E-09 |
| Hu147-11wk | 1.20E+08 | 3.74E-01 | 3.11E-09 |
| Hu147-12wk | 3.75E+07 | 1.16E-01 | 3.09E-09 |
| Hu147-13wk | 5.73E+07 | 1.46E-01 | 2.55E-09 |
| Hu147-23wk | 8.18E+07 | 1.73E-01 | 2.12E-09 |
| Hu147-31wk | 3.83E+07 | 1.07E-02 | 2.79E-09 |
| Hu147-32wk | 1.37E+08 | 3.43E-01 | 2.49E-09 |
| Hu147-33wk | 9.01E+07 | 1.74E-01 | 1.93E-09 |
| Hu164-57wl | 7.35E+07 | 2.37E-03 | 3.22E-11 |
| Hu164-67wl | 6.36E+07 | 1.17E-03 | 1.84E-11 |
| Hu164-67yl | 5.34E+07 | 1.94E-03 | 3.63E-11 |
| Hu164-77wl | 5.98E+07 | 8.83E-03 | 1.48E-10 |
| Hu164-87wl | 5.24E+07 | 4.40E-03 | 8.40E-11 |
| Hu164-68wl | 5.70E+07 | 7.60E-03 | 1.33E-10 |
| Hu164-69wl | 6.17E+07 | 7.74E-03 | 1.26E-10 |
| Hu95-21wk | 5.29E+06 | 2.11E-02 | 3.99E-09 |
| Hu95-31wk | 7.84E+06 | 2.25E-02 | 2.87E-09 |
| Hu95-41wk | 1.39E+07 | 3.39E-02 | 2.44E-09 |
| Hu95-42wk | 5.66E+06 | 1.37E-02 | 2.42E-09 |
| Hu95-51yk | 2.56E+07 | 5.16E-04 | 2.02E-11 |

The experimental results show that the chimeric antibodies Ch147 and Ch164 derived from the murine antibodies mAb147 and mAb164 and the humanized anti-CTGF antibodies can all bind to human CTGF and mouse CTGF with high affinity; all the substitutions with various human antibody germline FR regions and back mutations did not affect the affinity of the humanized antibodies, and some of the modifications could even enhance the affinity of antibodies for antigens.

**Examples 1-5. Inhibition of TGFβ-Induced *In Vitro* Migration of C2C12 Cells by CTGF Antibodies**

C2C12 cells (mouse myoblasts, Cell Bank, Chinese Academy of Sciences, #GNM26) were digested with 0.25% pancreatin (Gibco, #25200-072), centrifuged, and then resuspended in a serum-free DMEM medium (Gibco, #10564-029). Cell-antibody mixtures and TGFβ1-antibody mixtures were prepared with a serum-free DMEM medium; the cell density was $2 \times 10^5$/mL, the concentration of recombinant human TGFβ1 (Cell signaling Technology, #8915LC) was 10 ng/mL, and the concentrations of test antibodies were all 30 μg/mL.

The upper lid and filter membrane of a ChemoTx® Disposable Chemotaxis System (Neuro Probe, #106-8) were removed, and the TGFβ-antibody mixtures were added to the lower chamber at 30 μL/well, each group in quadruplicate. The filter membrane was put back on, and the cell-antibody mixtures were added to the membrane at 50 μL/well, each group in quadruplicate. The upper lid was put back on, and the system was placed into an incubator (37 °C, 5% $CO_2$).

After 48 h of incubation, a clean paper towel was used to soak up the liquid on the filter membrane. The filter membrane was removed, 10 μL of pre-cooled 0.25% pancreatin was added to each well in the lower chamber, and the filter membrane was put back on. After 5 min of digestion, the system was centrifuged at 1000 rpm for 1 min. The filter membrane was removed, and 20 μL of Cell Titer-Glo solution (Promega, #G7573) was added to each well in the lower chamber. The system was incubated at room temperature for 10 min. The liquid was transferred to a 384-well white-bottom plate (Thermo Scientific, #267462), and the plate was read by chemiluminescence using a microplate reader (BMG, #PheraStar). The data were analyzed and processed using Graphpad Prism 6.

$$\text{Inhibition rate} = [(\text{TGF}\beta \text{ mean value - sample group mean value}) / (\text{TGF}\beta \text{ group mean value - untreated group mean value})] \times 100\%$$

Table 19. The results of the inhibition *of in vitro* migration of C2C12 cells by CTGF antibodies

| Test sample | Percent inhibition of *in vitro* migration of C2C12 cells (%) |
|---|---|
| Hu147-11wk | 72.2% |
| Hu147-33wk | 63.8% |
| Hu164-57wl | 75.4% |
| Hu164-67wl | 78.8% |
| Hu164-67yl | 76.5% |
| Hu164-77wl | 72.8% |
| Hu164-87wl | 76.4% |
| Hu164-68wl | 76.3% |
| Hu164-69wl | 74.2% |
| Hu95-51yk | 80.0% |
| mAb1 | 63.0% |

The results show that the humanized anti-CTGF antibodies of the present disclosure can inhibit the *in vitro* migration ability of C2C12 cells after TGFβ1 induction and exhibited greater inhibition of the migration ability of C2C12 cells than the positive control antibody mAb1.

**Examples 1-6. Inhibition of TGFβ-Induced *In Vitro* Migration of PANC1 Cells by CTGF Antibodies**

PANC-1 cells (human pancreatic cancer cells, ATCC, #CRL-1469) were digested with 0.25% pancreatin (Gibco, #25200-072), centrifuged, and then resuspended in a DMEM medium (Gibco, #10564-029) containing 0.1% fetal bovine serum (Gibco, #10099-141). Cell-antibody mixtures and TGFβ-antibody mixtures were prepared with a DMEM medium containing 0.1% fetal bovine serum; the cell density was $4 \times 10^5$/mL, the concentration of recombinant human TGFβ (Cell signaling Technology, #8915LC) was 10 ng/mL, and the concentrations of test antibodies were all 30 μg/mL.

The upper lid and filter membrane of a ChemoTx® Disposable Chemotaxis System (Neuro Probe, #106-8) were removed, and the TGFβ-antibody mixtures were added to the lower chamber at 30 μL/well, each group in quadruplicate. The filter membrane was put back on, and the cell-antibody mixtures were added to the membrane at 50 μL/well, each group in quadruplicate. The upper lid was put back on, and the system was placed into an incubator (37 °C, 5% $CO_2$).

After 48 h of incubation, a clean paper towel was used to soak up the liquid on the filter membrane. The filter membrane was removed, 10 μL of pre-cooled 0.25% pancreatin was added to each well in the lower chamber, and the filter membrane was put back on. After 5 min of digestion, the system was centrifuged at 1000 rpm for 1 min. Cells that settled to the bottom of the plate were counted under an inverted microscope (Leica, #DMIL LED). The data were analyzed and processed using Graphpad Prism 6.

$$\text{Inhibition rate} = [(\text{TGF}\beta \text{ mean value - sample group mean value}) / (\text{TGF}\beta \text{ group mean value - untreated group mean value})] \times 100\%$$

Table 20. The results of the inhibition of *in vitro* migration of PANC1 cells by CTGF antibodies

| Test sample | Percent inhibition of *in vitro* migration of PANC1 cells (%) | Test sample | Percent inhibition of *in vitro* migration of PANC1 cells (%) |
|---|---|---|---|
| Hu147-11wk | 73.9% | Hu164-77wl | 74.3% |
| Hu147-33wk | 77.0% | Hu164-87wl | 82.1% |
| Hu164-57wl | 81.2% | Hu164-68wl | 76.9% |
| Hu164-67wl | 75.7% | Hu164-69wl | 76.9% |
| Hu164-67yl | 78.0% | mAb1 | 71.7% |
| Hu95-51yk | 80.0% | | |

The results show that all the humanized anti-CTGF antibodies of the present disclosure can inhibit the *in vitro* migration ability of PANC1 cells after TGFβ1 induction.

**Example 1-7. Inhibition *of In Vitro* Proliferation of PANC1 in Soft Agar by CTGF Antibodies**

Deionized water was added to agar (BD, #214220), and the mixture was heated to make a 1.4% gel solution. A 2× DMEM medium (Thermo, #12100046) was mixed with the gel solution in a ratio of 1:1 (v/v), and 500 μL of the mixture was added to each well of a 24-well plate (Costar, #3524) and left at 4 °C to coagulate.
PANC-1 cells (human pancreatic cancer cells, ATCC, #CRL-1469) were digested with 0.25% pancreatin (Gibco, #25200-072) and centrifuged, and then the cell density was adjusted to $5 \times 10^4$ cells/mL with a DMEM medium (GE, #SH30243.01) containing 4% fetal bovine serum (Gibco, #10099-141). 2 mg/mL antibodies were prepared with a 2× DMEM medium. The cells, antibodies, and 1.4% gel solution were mixed in a ratio of 2:1:1 (v/v), and 200 μL of the mixture was added to each well of a 24-well plate in which resolving gel had been added. After the upper gel coagulated, 200 μL of a DMEM medium containing 2% fetal bovine serum was added to the 24-well plate. After 28 days of culture in an incubator (37 °C, 5% CO2), photographic records were made using a high-content analysis system (Molecular Devices, ImageXpress), and the area of cell clones formed was analyzed. Inhibition rate = (1- sample group mean value / untreated group mean value) × 100%. The experimental results are shown in Table 21.

Table 21. The results of the inhibition of *in vitro* proliferation of PANC1 cells in soft agar by CTGF antibodies

| Test sample | Percent inhibition of *in vitro* proliferation of PANC1 cells (%) |
|---|---|
| Hu147-11wk | 23.76% |
| Hu147-33wk | 22.30% |
| Hu164-67wl | 24.2% |
| Hu164-67yl | 21.7% |
| Hu95-51yk | 20.24% |

The results show that the humanized antibodies from the mAb147 and mAb164 clones have significant inhibitory effects on the *in vitro* proliferation of human pancreatic cancer cells (PANC-1 cells).

**Examples 1-8. Biological Activity of CTGF Antibodies in Animals**

**I. Inhibition of bleomycin-induced mouse pulmonary fibrosis by CTGF antibodies**

Experimental method: 40 mice were randomly divided by body weight into the following 4 groups: normal control group (PBS, ip, qod), mAb1 group (10 mg/kg, ip, qod), Hu164-67wl (10 mg/kg, ip, qod) group, and Hu164-67yl (10 mg/kg, ip, qod) group. Each group included 10 mice. The specific grouping is shown in Table 21 below. On the 1st day of the experiment, corresponding solvents and antibodies (10 mL/kg, the normal control and model groups were given PBS) were intraperitoneally injected according to body weight. After 2 h, modeling was performed by bleomycin nebulization (nebulization was performed at 50 mg/8 mL for 30 min and held for 5 min). Then the solvents or antibodies were intraperitoneally injected once every other day according to the groups. The experimental period was 21 days, and the intraperitoneal administration was performed 11 times in total. On the 22nd day of the experiment, mice were anesthetized by an intraperitoneal injection of 1% pentobarbital sodium (10 mL/kg) and then fixed onto an operation table in a supine position. A 1 cm skin incision was made along the median line of the neck, and the lower end of the incision reached the entrance of the chest. The subcutaneous connective tissues and muscles were bluntly separated with forceps to expose the trachea. The connective tissues on both sides of the trachea and between the trachea and the esophagus were separated to isolate the trachea free. A venous indwelling needle was inserted and fixed by ligation with a surgical suture at a place where the cannula entered the trachea. 0.8 mL of PBS was drawn with a 1 mL syringe, and the syringe was connected to the inlet end of the venous indwelling needle. PBS was slowly injected into the trachea, stayed for 30 s, and then slowly withdrawn. An opalescent foamy liquid could be observed during the withdrawal. The lavage was repeated three times, and the lavage fluid was transferred to an EP tube. Then 0.5 mL of PBS was drawn, and the above steps were repeated. The two lavage fluids were mixed and centrifuged at 1500 rpm for 5 min, and the supernatant was stored at -20 °C before analysis. The BALF supernatant was centrifuged again at 10,000 rpm for 10 min, and then the supernatant was taken and tested for soluble collagen (kit: Biocolor, Lot No. AA883).

Table 22. Grouping and dosing regimen

| Group | Number of animals | Drug | Dose (mg/mL) | Route of administration | Frequency of administration |
|---|---|---|---|---|---|
| 1 | 10 | PBS | - | i.p. | Once every other day |
| 2 | 10 | mAb1 | 10 | i.p. | Once every other day |
| 3 | 10 | Hu164-67wl | 10 | i.p. | Once every other day |
| 4 | 10 | Hu164-67yl | 10 | i.p. | Once every other day |

Excel statistical software was used: the average values were calculated as avg (average); SD (standard diviation) values were calculated as STDEV (standard diviation); SEM (standard error of mean) values were calculated as STDEV/SQRT (square root) (number of animals per group); GraphPad Prism software was used for plotting, and the data were statistically analyzed using two-way ANOVA or one-way ANOVA. The experimental results are shown in Table 23.

Table 23. The experimental results of the inhibition of bleomycin-induced mouse pulmonary fibrosis by CTGF antibodies

| Drug | mAb1 (10mpk) | Hu164-67wl (10mpk) | Hu164-67yl (10mpk) |
|---|---|---|---|
| Increased collagen inhibition rate | 53.2% | 72.8% (p<0.05) | 80.7% (p<0.05) |

The results show that Hu164-67wl or Hu164-67yl exhibited a strong inhibitory effect on pulmonary fibrosis in mice.

**II. Inhibition of subcutaneous xenograft tumors of Su86.86 human pancreatic cancer cells by CTGF antibodies**

Experimental method: 200 $\mu$L of SU86.86 cells (ATCC, CRL-1837, $3.0 \times 10^6$ cells) were inoculated subcutaneously into the right flank of Nu/Nu nude mice. Eleven days after the inoculation, when the tumor volume reached about 140 $mm^3$, mice that were too heavy or mice with tumors that were too big or too small were excluded. Mice were randomly divided by tumor volume into 5 groups of 10, and administration was started on that day. Antibodies were administered intraperitoneally twice a week for 18 days in total. The tumor volume and body weight were measured 1-2 times a week, and the data were recorded. The grouping and dosing regimen are shown in Table 24.

Table 24. Grouping and dosing regimen

| Group | Number of animals | Drug | Dose (mg/kg) | Route of administration | Frequency of administration |
|---|---|---|---|---|---|
| 1 | 10 | Human IgG | 40 | i.p | Twice a week |
| 2 | 10 | mAb1 | 40 | i.p | Twice a week |
| 3 | 10 | Hu164-67yl | 40 | i.p | Twice a week |
| 4 | 10 | Hu164-67yl | 20 | i.p | Twice a week |
| 5 | 10 | Hu147-33wk | 40 | i.p | Twice a week |

Data were recorded using Excel statistical software: the average values were calculated as avg; the SD values were calculated as STDEV; the SEM values were calculated as STDEV/SQRT (number of animals per group); GraphPad Prism software was used for plotting, and statistical analysis of the data was performed using Two-way ANOVA or One-way ANOVA.

$$\text{Tumor volume (V) was calculated as: } V = 1/2 \times L_{long} \times L_{short}^2$$

$$\text{Relative tumor proliferation rate T/C (\%)} = (T_t - T_0) / (C_t - C_0) \times 100,$$

where $T_t$ and Ct are the tumor volumes of the treatment group and control group at the end of the experiment; To and Co are the tumor volumes at the beginning of the experiment.

$$\text{Tumor growth inhibition TGI (\%)} = 1 - \text{T/C (\%)}.$$

Experimental results:

During days 1-18 of the experiment (D0 stands for the start of the experiment and D18 for day 18), administration was performed by subcutaneous injection twice a week, and the inhibitory effects of CTGF antibodies on SU86.86 tumor growth were measured. The experimental results are shown in Table 24 and FIG. 2. Compared to the blank control group of isotype human IgG, the mAb1-40 mg/kg, Hu164-67yl-40 mg/kg, Hu164-67yl-20 mg/kg, and Hu147-33wk (40 mg/kg) groups exhibited 45%, 53%, 47%, and 54% tumor growth inhibition, respectively. The mAb1-40 mg/kg, Hu164-67yl -40 mg/kg, Hu164-67yl-20 mg/kg, and Hu147-33wk (40 mg/kg) groups can significantly inhibit Su86.86 tumor growth (p < 0.001), and the inhibitory effects on SU86.86 tumors are dose-dependent to a certain extent. In addition, there was no significant change in the body weight of each group of mice.

Table 25. The results of the inhibition of SU86.86 mouse xenograft tumors by CTGF antibodies

| Group | Mean tumor volume (mm$^3$) | | Mean tumor volume (mm$^3$) | | % Tumor growth inhibition | P (vs blank control) |
|---|---|---|---|---|---|---|
| | D0 | SEM | D18 | SEM | D18 | |
| Human IgG | 144.35 | 12.30 | 1087.10 | 148.21 | - | |
| mAb1 (40mg/kg) | 141.18 | 11.72 | 655.75 | 144.46 | 45 | p<0.001 |
| Hu164-67yl (40mg/kg) | 138.75 | 8.71 | 582.95 | 76.14 | 53 | p<0.001 |
| Hu164-67yl (20mg/kg) | 139.87 | 9.33 | 640.26 | 96.70 | 47 | p<0.001 |
| Hu147-33wk (40mg/kg) | 138.00 | 10.11 | 569.66 | 77.40 | 54 | p<0.001 |

**II. Formulation**

**The equipment, antibodies, and methods used in the preparation of formulations and detection are shown below.**

**SEC molecular exclusion chromatography:**

This is a method for analyzing the separation of a solute by the relative relationship between the pore size of the gel pores and the size of the polymer sample molecule coil. SEC% (SEC monomer content percentage) = A monomer / A total $\times$ 100% (A monomer represents the peak area of the main peak monomer in the sample, and A total represents the sum of all peak areas).

Instrument for SEC determination: Agilent 1260; column: waters, XBrige BEH200Å SEC (300 $\times$ 7.8 mm 3.5 $\mu$m).

**NR-CE capillary gel electrophoresis:**

This is a method of moving the gel into a capillary as a supporting medium for electrophoresis and separating according to the molecular weight of the sample under a certain voltage.

Non-reduced CE purity percentage = A main peak/A total × 100% (A main peak represents the light chain main peak area + the heavy chain main peak area, and A total represents the sum of all peak areas).

Instrument for CE determination: Beckman, model: plus800.

**iCIEF imaged capillary isoelectric focusing electrophoresis:**

This is a technique for separating according to the difference of isoelectric points pI of proteins.

iCIEF neutral peak content percentage = neutral peak area/total area × 100% (total area represents the sum of areas of acidic, neutral and basic peaks).

Manufacturer of instrument for the iCIEF determination: simple protein, model: muarice.

**Osmotic pressure measurement:**

The freezing point method was used for measuring the osmotic pressure. The freezing point of a solution is measured by using a high-sensitivity temperature-sensing element on the basis of the proportional relation between the freezing point depression value and the molar concentration of the solution, and then converted into the osmotic pressure through electric quantity. Manufacturer of instrument: Loser, model: OM815.

**Protein concentration measurement:**

Instrument for protein concentration measurement: ultraviolet-visible spectrophotometer; model: Nano Drop oneC; optical path length: 1 mm. The following antibody concentrations are calculated based on protein concentrations.

**CTGF antibodies**

The anti-CTGF antibody used in the following examples was Hu164-67yl, wherein the antibody used in samples 1-6 of Example 2-1, Example 2-2, Example 2-3, and Example 2-4 and samples 1 and 2 of Examples 2-5 was obtained by affinity chromatography after protein expression; the antibody used in samples 7 and 8 of Examples 2-4 and samples 3-9 of Examples 2-5 was obtained by further subjecting the aforementioned antibody to ion exchange chromatography and filtration.

**Example 2-1. Formulation Buffer System and pH Value Screening**

Liquid formulations comprising 200 mg/mL Hu164-67yl were prepared. The formulations further comprised 0.4 mg/mL polysorbate 80 (PS80), 70 mg/mL sucrose, and different buffer systems. The buffer systems were 50 mM acetic acid-sodium acetate (AA) pH 5.0, 5.5; 50 mM histidine-acetate (His-AA) pH 5.5; 50 mM sodium succinate (SA) pH 5.5; 50 mM sodium citrate (CA) pH 5.5; 50 mM histidine-hydrochloride buffer (His-HCl) pH 5.5, 6.0, 6.5; and 50 mM sodium dihydrogen phosphate-disodium hydrogen phosphate (PB) pH 6.5, 7.0. Each of the formulations was filtered and bottled, and the bottles were stoppered and capped. Then the formulations were left to stand at a constant temperature of 40 °C for 1 month, and the indexes such as SEC and non-reduced CE were evaluated.

The results are shown in Table 26. The SEC data show that in the case of standing at 40 °C for 1 month, the reductions in the main peaks of the samples using AA (pH 5.5), His-AA (pH 5.5), CA (pH 5.5), and His-HCl (pH 5.5) are smaller than those of the other samples. The NR-CE data show that in the case of standing at 40 °C for 1 month, the reduction in the main peak of the sample using His-HCl (pH 5.5) is the smallest (5.1%).

Table 26. The results of the pH and buffer system screening

| Buffer system | Conditions | SEC% | △SEC% | NR-CE% | △NR-CE % |
|---|---|---|---|---|---|
| 50mM AA pH 5.0 | D0 | 97.8 | | 93.3 | |
| | 40°C M1 | 91.5 | 6.3 | 79.9 | 13.4 |
| 50mM AA pH 5.5 | D0 | 97.8 | | 93.6 | |
| | 40°C M1 | 93.2 | 4.6 | 84.0 | 9.6 |
| 50mM His-AA pH 5.5 | D0 | 97.9 | | 93.6 | |
| | 40°C M1 | 92.0 | 5.9 | 83.5 | 10.1 |
| 50mM CA pH 5.5 | D0 | 97.9 | | 94.2 | |
| | 40°C M1 | 92.8 | 5.1 | 82.2 | 12.0 |
| 50mM SA pH 5.5 | D0 | 97.9 | | 93.4 | |
| | 40°C M1 | 87.3 | 10.6 | 76.9 | 16.5 |
| 50mM His-HCl pH 5.5 | D0 | 97.9 | | 93.4 | |
| | 40°C M1 | 92.9 | 5.0 | 88.3 | 5.1 |
| 50mM His-HCl pH 6.0 | D0 | 98.0 | | 93.7 | |
| | 40°C M1 | 91.2 | 6.9 | 88.0 | 5.7 |
| 50mM His-HCl pH 6.5 | D0 | 98.0 | | 93.4 | |
| | 40°C M1 | 89.8 | 8.2 | 86.3 | 7.1 |
| 50mM PB pH 6.5 | D0 | 97.9 | | 93.8 | |
| | 40°C M1 | 88.0 | 9.9 | 86.3 | 7.5 |
| 50mM PB pH 7.0 | D0 | 97.8 | | 93.9 | |
| | 40°C M1 | 84.9 | 12.9 | 82.5 | 11.4 |

Note: in the table, "D" denotes day; for example, D7 denotes 7 days, and so on; DO denotes the start of the experiment; "M" denotes month; for example, M1 denotes 1 month, and so on.

**Example 2-2. Long-Term Stability Testing**

A formulation comprising 200 mg/mL Hu164-67yl, 50 mM His-HCl (pH 5.5), 70 mg/mL sucrose, and 0.4 mg/mL PS80 was prepared. Samples were left under 25 °C and 4 °C conditions to investigate stability. The results are shown in Table 27.

Table 27. Long-term stability testing

| Conditions | SEC% | NR-CE% | iCIEF% |
|---|---|---|---|
| D0 | 97.9 | 93.4 | 57.1 |
| 4°C M3 | 96.7 | 93.7 | 57.6 |
| 4°C M5 | 97.4 | 93.4 | 57.5 |
| 25°C M3 | 94.0 | 92.0 | 51.4 |
| 25°C M5 | 94.3 | 90.3 | 46.0 |

Note: in the table, "M" denotes month; for example, M3 denotes 3 months, and so on. The same applies hereinafter.

The results show that: after the samples were left to stand at 25 °C for 5 months, the purity slightly decreased, but the decrease was within an acceptable range; after the samples were left to stand at 4 °C for 5 months, all the test indexes did not change significantly, and accordingly, this formula has better stability.

**Example 2-3. Protein Concentration Screening**

Liquid formulations were prepared according to the following formulas:

1) 200 mg/mL Hu164-67yl, 50 mM His-HCl (pH 6.0), 0.4 mg/mL PS80, and 70 mg/mL sucrose
2) 150 mg/mL Hu164-67yl, 50 mM His-HCl (pH 6.0), 0.4 mg/mL PS80, and 70 mg/mL sucrose

The two formulations were filtered and bottled, and the bottles were stoppered and capped. The samples were subjected to 40 °C stability testing and long-term stability testing, with SEC, iCIEF, and non-reduced CE as indexes for evaluation.

Table 28. The results of the protein concentration screening

| Group | Conditions | SEC% | △SEC% | NR-CE% | △NR-CE% | iCIEF% | △iCIEF% |
|---|---|---|---|---|---|---|---|
| 1 | D0 | 98.0 | | 93.7 | | 56.9 | |
| | 40°C M1 | 91.2 | 6.8 | 88.0 | 5.7 | 35.7 | 21.2 |
| | 25°C M1 | 96.7 | 1.3 | 94.1 | | 52.1 | 4.8 |
| | 25°C M3 | 93.2 | 4.8 | 93.2 | 0.5 | 43.0 | 13.9 |
| | 25°C M5 | 92.3 | 5.7 | 90.2 | 3.5 | 32.8 | 24.1 |
| | 4°C M3 | 96.5 | 1.5 | 93.9 | | 56.8 | 0.1 |
| | 4°C M5 | 97.0 | 1.0 | 93.0 | 0.7 | 56.9 | 0.0 |
| 2 | D0 | 98.0 | | 93.3 | | 56.8 | |
| | 40°C M1 | 91.7 | 6.3 | 87.6 | 5.7 | 36.0 | 20.8 |
| | 25°C M1 | 97.0 | 1.0 | 93.2 | 0.1 | 53.5 | 3.3 |
| | 25°C M3 | 93.9 | 4.1 | 93.2 | 0.1 | 50.2 | 6.6 |
| | 25°C M5 | 94.1 | 3.9 | 89.6 | 3.7 | 45.2 | 11.6 |
| | 4°C M3 | 96.6 | 1.4 | 94.3 | | 56.2 | 0.6 |
| | 4°C M5 | 97.3 | 0.7 | 93.1 | 0.2 | 56.3 | 0.5 |

The results show that after samples 1) and 2) were left to stand under 40 °C M1, 25 °C M3, and 4 °C M5 conditions, there was no significant difference in purity. After standing under 25 °C M5 conditions, the reductions in the SEC and iCIEF main peaks of sample 1) were slightly greater than those of sample 2).

**Examples 2-4. Surfactant Screening**

Formulations comprising 150 mg/mL Hu164-67yl, 50 mM His-HCl pH 5.5, 70 mg/mL sucrose, and different surfactants were prepared. Each of the formulations was filtered and bottled, and the bottles were stoppered and capped. Samples were taken and subjected to shaking (25 °C, 300 rpm), 5 freeze-thaw cycles (FT5C, conditions: -35 °C -2 to 8 °C), 40 °C high-temperature standing, and long-term stability (4 °C, M3) testing, and SEC, non-reduced CE, and iCIEF were examined. The specific formula designs are shown in Table 29.

Table 29. The results of the surfactant type and concentration screening

| Group | Buffer system | Conditions | SEC% | NR-CE% | iCIEF% |
|---|---|---|---|---|---|
| 1 | 0.2mg/mL PS20 | D0 | 97.8 | 93.3 | 56.9 |
| | | Shaking | 97.4 | 95.1 | 57.1 |
| | | FT5C | 96.8 | 92.2 | 56.8 |
| | | 4°C M3 | 97.6 | 93.1 | 58.7 |

(continued)

| Group | Buffer system | Conditions | SEC% | NR-CE% | iCIEF% |
|---|---|---|---|---|---|
| 2 | 0.4mg/mL PS20 | D0 | 97.8 | 93.4 | 56.6 |
| | | Shaking | 97.4 | 95.0 | 57.0 |
| | | FT5C | 96.7 | 92.5 | 56.7 |
| | | 4°C M3 | 97.5 | 93.4 | 58.7 |
| 3 | 0.6 mg/mL PS20 | D0 | 97.8 | 93.9 | 57.0 |
| | | Shaking | 97.3 | 94.8 | 57.5 |
| | | FT5C | 96.6 | 92.1 | 54.0 |
| | | 4°C M3 | 97.2 | 93.5 | 58.1 |
| 4 | 0.2 mg/mL PS80 | D0 | 97.7 | 93.3 | 56.9 |
| | | Shaking | 97.3 | 95.1 | 57.1 |
| | | FT5C | 96.6 | 92.1 | 56.6 |
| | | 4°C M3 | 97.5 | 93.7 | 58.0 |
| 5 | 0.4 mg/mL PS80 | D0 | 97.7 | 93.8 | 57.2 |
| | | Shaking | 96.8 | 95.0 | 57.1 |
| | | FT5C | 96.6 | 92.0 | 56.7 |
| | | 4°C M3 | 97.5 | 93.7 | 57.7 |
| 6 | 0.6 mg/mL PS80 | D0 | 97.7 | 93.3 | 57.0 |
| | | Shaking | 97.2 | 94.9 | 57.1 |
| | | FT5C | 96.5 | 92.0 | 56.2 |
| | | 4°C M3 | 97.5 | 93.4 | 57.7 |
| 7 | 0.4 mg/mL poloxamer 188 | D0 | 98.4 | 95.2 | 61.9 |
| | | 40°C M1 | 94.9 | 91.1 | 41.5 |
| 8 | 0.4 mg/mL PS80 | D0 | 98.4 | 94.7 | 61.8 |
| | | 40°C M1 | 94.2 | 90.8 | 40.2 |

The experimental results show that there was no significant difference in the purity results of the samples comprising different types and concentrations of polysorbate. After standing at 40 °C for 1 month, there was no significant difference in the purity of two samples comprising the same concentration of PS80 and poloxamer 188.

**Examples 2-5. Formulation Formula Optimization**

Liquid formulations were prepared according to the following formulas:

1) 200 mg/mL Hu164-67yl, 50 mM His-HCl (pH 6.0), 70 mg/mL sucrose, and 0.4 mg/mL PS80
2) 200 mg/mL Hu164-67yl, 50 mM His-HCl (pH 6.0), 70 mg/mL trehalose, and 0.4 mg/mL PS80
3) 150 mg/mL Hu164-67yl, 50 mM His-HCl (pH 5.5), 70 mg/mL sucrose, and 0.4 mg/mL PS80
4) 150 mg/mL Hu164-67yl, 50 mM His-HCl (pH 5.5), 70 mg/mL sucrose, 0.4 mg/mL PS80, and 2% PEG 3350
5) 150 mg/mL Hu164-67yl, 50 mM His-HCl (pH 5.5), 70 mg/mL sucrose, 0.4 mg/mL PS80, and 50 mM arginine
6) 150 mg/mL Hu164-67yl, 50 mM His-HCl (pH 5.5), 70 mg/mL sucrose, 0.4 mg/mL PS80, and 1% mannitol
7) 150 mg/mL Hu164-67yl, 50 mM His-HCl (pH 5.5), 70 mg/mL sucrose, 0.4 mg/mL PS80, and 0.5 mM EDTA
8) 150 mg/mL Hu164-67yl, 50 mM His-HCl (pH 5.5), 70 mg/mL sucrose, 0.4 mg/mL PS80, and 5 mM EDTA
9) 150 mg/mL Hu164-67yl, 50 mM His-HCl (pH 5.5), 70 mg/mL sucrose, 0.4 mg/mL PS80, and 10 mM EDTA

Each of the formulations was filtered and bottled, and the bottles were stoppered and capped. Samples were subjected to 40 °C stability testing, with SEC, iCIEF, and non-reduced CE as indexes for evaluation.

Table 30. Formula Optimization

| Group | Conditions | SEC% | △SEC% | NR-CE% | △NR-CE% | iCIEF% | △iCIEF% |
|---|---|---|---|---|---|---|---|
| 1 | D0 | 98.0 | | 93.7 | | 56.9 | |
| | 40°C M1 | 91.2 | 6.1 | 88.0 | 5.7 | 35.7 | 21.2 |
| 2 | D0 | 98.0 | | 93.5 | | 56.8 | |
| | 40°C M1 | 90.8 | 7.2 | 88.9 | 4.6 | 34.0 | 22.8 |
| 3 | D0 | 98.4 | | 94.7 | | 61.8 | |
| | 40°C M1 | 94.2 | 4.2 | 90.8 | 3.9 | 40.2 | 21.6 |
| 4 | D0 | 98.3 | | 94.4 | | 61.5 | |
| | 40°C M1 | 94.3 | 4.0 | 90.5 | 3.9 | 40.6 | 20.9 |
| 5 | D0 | 98.4 | | 94.5 | | 61.7 | |
| | 40°C M1 | 94.2 | 4.2 | 91.0 | 3.5 | 40.6 | 21.1 |
| 6 | D0 | 98.0 | | 95.1 | | 60.7 | |
| | 40°C M1 | 94.1 | 3.9 | 91.1 | 4.0 | 40.1 | 20.6 |
| 7 | D0 | 98.3 | | 94.8 | | 61.7 | |
| | 40°C M1 | 94.7 | 3.6 | 91.8 | 3.0 | 41.0 | 20.7 |
| 8 | D0 | 98.3 | | 94.6 | | 61.6 | |
| | 40°C M1 | 94.7 | 3.6 | 91.6 | 3.0 | 40.9 | 20.7 |
| 9 | D0 | 98.3 | | 94.5 | | 62.2 | |
| | 40°C M1 | 94.7 | 3.6 | 92.2 | 2.3 | 40.7 | 21.5 |

The results show that: after the samples were left to stand at 40 °C for 1 month, there was no significant difference in purity between the samples in which only sucrose was added and the samples in which other stabilizers were added. The formula stability was good under these conditions. The results suggest that addition of stabilizers other than sucrose will not affect the formula stability, and the formulas that do not comprise other stabilizers have achieved the stability of the formulas that comprise other stabilizers.

**Claims**

1. A pharmaceutical composition, comprising an anti-CTGF antibody and a buffer, wherein the anti-CTGF antibody comprises a heavy chain variable region and a light chain variable region, wherein:

    i) the heavy chain variable region comprises HCDR1, HCDR2, and HCDR3 having identical sequences to those of a heavy chain variable region set forth in SEQ ID NO: 8, and the light chain variable region comprises LCDR1, LCDR2, and LCDR3 having identical sequences to those of a light chain variable region set forth in SEQ ID NO: 9;
    ii) the heavy chain variable region comprises HCDR1, HCDR2, and HCDR3 having identical sequences to those of a heavy chain variable region set forth in SEQ ID NO: 6, and the light chain variable region comprises LCDR1, LCDR2, and LCDR3 having identical sequences to those of a light chain variable region set forth in SEQ ID NO: 7;
    ii-i) the heavy chain variable region comprises HCDR1, HCDR2, and HCDR3 having identical sequences to those of a heavy chain variable region set forth in SEQ ID NO: 69, and the light chain variable region comprises LCDR1, LCDR2, and LCDR3 having identical sequences to those of a light chain variable region set forth in SEQ ID NO: 70; or
    ii-ii) the heavy chain variable region comprises HCDR1, HCDR2, and HCDR3 having identical sequences to those of a heavy chain variable region set forth in SEQ ID NO: 85, and the light chain variable region comprises LCDR1, LCDR2, and LCDR3 having identical sequences to those of a light chain variable region set forth in SEQ ID NO: 70; the buffer is a histidine salt buffer, preferably a histidine-hydrochloride buffer or a histidine-acetate buffer, and more preferably a histidine-hydrochloride buffer.

2. The pharmaceutical composition according to claim 1, wherein the anti-CTGF antibody comprises a heavy chain variable region and a light chain variable region, wherein:

the heavy chain variable region comprises HCDR1, HCDR2, and HCDR3 set forth in SEQ ID NO: 16, SEQ ID NO: 17, and SEQ ID NO: 18, respectively, and the light chain variable region comprises LCDR1, LCDR2, and LCDR3 set forth in SEQ ID NO: 19, SEQ ID NO: 20, and SEQ ID NO: 21, respectively;
preferably, the heavy chain variable region has an amino acid sequence set forth in SEQ ID NO: 34, and the light chain variable region has an amino acid sequence set forth in SEQ ID NO: 30;
more preferably, the anti-CTGF antibody comprises a heavy chain set forth in SEQ ID NO: 61 and a light chain set forth in SEQ ID NO: 64.

3. The pharmaceutical composition according to claim 1 or 2, wherein the pharmaceutical composition has a pH of about 5.0 to about 6.5, preferably about 5.0 to about 6.0, and more preferably about 5.5.

4. The pharmaceutical composition according to any one of claims 1 to 3, wherein the anti-CTGF antibody is at a concentration of about 100 mg/mL to about 200 mg/mL, preferably about 150 mg/mL to about 200 mg/mL, and more preferably about 150 mg/mL.

5. The pharmaceutical composition according to any one of claims 1 to 4, wherein the pharmaceutical composition further comprises a surfactant, and the surfactant is preferably a polysorbate or poloxamer, more preferably polysorbate 80, polysorbate 20, or poloxamer 188, and most preferably polysorbate 80.

6. The pharmaceutical composition according to claim 5, wherein the surfactant is at a concentration of about 0.05 mg/mL to about 1.0 mg/mL, preferably about 0.2 mg/mL to about 0.6 mg/mL, and more preferably about 0.4 mg/mL.

7. The pharmaceutical composition according to any one of claims 1 to 6, wherein the pharmaceutical composition further comprises a sugar, and the sugar is preferably selected from one or more of sucrose, mannitol, and trehalose, and is more preferably sucrose.

8. The pharmaceutical composition according to claim 7, wherein the sugar is at a concentration of about 20 mg/mL to about 100 mg/mL, preferably about 40 mg/mL to about 80 mg/mL, and more preferably about 70 mg/mL.

9. The pharmaceutical composition according to any one of claims 1 to 8, wherein the buffer is at a concentration of about 5 mM to about 100 mM, preferably about 30 mM to about 70 mM, and more preferably about 50 mM.

10. The pharmaceutical composition according to any one of claims 1 to 9, wherein the pharmaceutical composition further comprises a stabilizer, and the stabilizer is selected from the group consisting of PEG, arginine, and EDTA.

11. The pharmaceutical composition according to any one of claims 1 to 10, comprising the following components:

(a) the anti-CTGF antibody at about 150 mg/mL to about 200 mg/mL, (b) about 0.2 mg/mL to about 0.6 mg/mL polysorbate, (c) about 40 mg/mL to about 80 mg/mL sugar, and (d) about 30 mM to about 70 mM histidine salt buffer; the pharmaceutical composition having a pH of about 5.0 to about 6.0;
preferably comprising the following components:
(a) the anti-CTGF antibody at about 150 mg/mL, (b) about 0.4 mg/mL polysorbate 80, (c) about 70 mg/mL sucrose, and (d) about 50 mM histidine-hydrochloride buffer; the pharmaceutical composition having a pH of about 5.5 to about 5.7; the anti-CTGF antibody comprising a heavy chain set forth in SEQ ID NO: 61 and a light chain set forth in SEQ ID NO: 64.

12. A lyophilized formulation, wherein the lyophilized formulation can, upon reconstitution, form the pharmaceutical composition according to any one of claims 1 to 11.

13. A method for preparing a lyophilized formulation, comprising a step of lyophilizing the pharmaceutical composition according to any one of claims 1 to 11.

14. A lyophilized formulation, wherein the formulation is obtained by lyophilizing the pharmaceutical composition according to any one of claims 1 to 11.

15. A reconstituted solution, wherein the reconstituted solution is prepared by reconstituting the lyophilized formulation according to claim 12 or 14;

preferably, the reconstituted solution comprises:
(a) the anti-CTGF antibody at about 150 mg/mL to about 200 mg/mL, (b) about 0.2 mg/mL to about 0.6 mg/mL polysorbate, (c) about 40 mg/mL to about 80 mg/mL sugar, and (d) about 30 mM to about 70 mM histidine salt buffer; the pharmaceutical composition having a pH of about 5.0 to about 6.0;
more preferably, the reconstituted solution comprises:
(a) the anti-CTGF antibody at about 150 mg/mL, (b) about 0.4 mg/mL polysorbate 80, (c) about 70 mg/mL sucrose, and (d) about 50 mM histidine-hydrochloride buffer; the pharmaceutical composition having a pH of about 5.5 to about 5.7; the anti-CTGF antibody comprising a heavy chain set forth in SEQ ID NO: 61 and a light chain set forth in SEQ ID NO: 64.

16. The pharmaceutical composition according to any one of claims 1 to 11 or the reconstituted solution according to claim 15, being a formulation for intravenous, subcutaneous, intraperitoneal, or intramuscular injection, preferably a formulation for intravenous injection.

17. An article of manufacture, comprising a container, wherein the container contains the pharmaceutical composition according to any one of claims 1 to 11, the lyophilized formulation according to claim 12 or 14, or the reconstituted solution according to claim 15.

18. A method for treating or preventing a CTGF-associated disease, wherein the method comprises administering to a subject an effective amount of the pharmaceutical composition according to any one of claims 1 to 11, the lyophilized formulation according to claim 12 or 14, the reconstituted solution according to claim 15, or the article of manufacture according to claim 17;

wherein the CTGF-associated disease is preferably a fibrotic disease, hypertension, diabetes, myocardial infarction, arthritis, a CTGF-associated disease of cellular proliferation, atherosclerosis, glaucoma, or cancer;
preferably, the fibrotic disease is selected from the group consisting of: idiopathic pulmonary fibrosis, diabetic nephropathy, diabetic retinopathy, osteoarthritis, scleroderma, chronic heart failure, liver cirrhosis, and renal fibrosis;
preferably, the cancer is selected from the group consisting of: acute lymphoblastic leukemia, dermatofibroma, breast cancer, angiolipoma, angioleiomyoma, desmoplastic cancer, prostate cancer, ovarian cancer, colorectal cancer, pancreatic cancer, gastrointestinal cancer, and liver cancer.

**FIG. 1**

**FIG. 2**

## INTERNATIONAL SEARCH REPORT

International application No.

**PCT/CN2021/135203**

### A. CLASSIFICATION OF SUBJECT MATTER

C07K 16/22(2006.01)i; C12N 15/13(2006.01)i; C12N 15/63(2006.01)i; A61K 39/395(2006.01)i; A61P 9/10(2006.01)i; A61P 9/12(2006.01)i; A61P 19/02(2006.01)i; A61P 27/06(2006.01)i; A61P 35/00(2006.01)i; A61P 1/16(2006.01)i; A61P 3/10(2006.01)i; A61P 11/00(2006.01)i; A61P 13/12(2006.01)i; A61P 17/00(2006.01)i

According to International Patent Classification (IPC) or to both national classification and IPC

### B. FIELDS SEARCHED

Minimum documentation searched (classification system followed by classification symbols)

C07K C12N A61K A61P

Documentation searched other than minimum documentation to the extent that such documents are included in the fields searched

Electronic data base consulted during the international search (name of data base and, where practicable, search terms used)

CNABS, DWPI, CNTXT, USTXT, EPTXT, WOTXT, JPTXT, CNKI, Web of Science, 万方数据检索系统, WANFANG DATA SEARCH SYSTEM, pubmed, baidu, patentics, Genbank, EBI, 中国专利生物序列检索系统, China Patents Biological Sequence Search System, STN: CTGF, 结缔组织生长因子, connective tissue growth factor, 抗体, antibody, McAb, Mab, IgG, scFv, Fab, 组氨酸盐缓冲液, histidine-hydrochloride, 聚山梨酯, Surfactant, SEQ ID NOs: 6-9, 16-21, 30, 34, 61, 64, 69, 70, 85

### C. DOCUMENTS CONSIDERED TO BE RELEVANT

| Category* | Citation of document, with indication, where appropriate, of the relevant passages | Relevant to claim No. |
|---|---|---|
| PX | WO 2020244540 A1 (JIANGSU HENGRUI MEDICINE CO., LTD. et al.) 10 December 2020 (2020-12-10) claims 1-15, and description, sequence list, sequences 6-9, 16-21, 30, 34, 61, 64, 69, 70, and 85 | 1-18 |
| A | US 2014127224 A1 (NEFF, T. B. et al.) 08 May 2014 (2014-05-08) entire document | 1-18 |
| A | US 2004248206 A1 (LIN, A. Y. et al.) 09 December 2004 (2004-12-09) entire document | 1-18 |
| A | CN 102666587 A (FIBROGEN, INC.) 12 September 2012 (2012-09-12) entire document | 1-18 |
| A | US 2012263680 A1 (MOERAE MATRIX, INC.) 18 October 2012 (2012-10-18) entire document | 1-18 |
| A | WO 2010042201 A1 (FIBROGEN, INC.) 15 April 2010 (2010-04-15) entire document | 1-18 |

☑ Further documents are listed in the continuation of Box C.   ☑ See patent family annex.

| | | |
|---|---|---|
| * Special categories of cited documents: | "T" | later document published after the international filing date or priority date and not in conflict with the application but cited to understand the principle or theory underlying the invention |
| "A" document defining the general state of the art which is not considered to be of particular relevance | | |
| "E" earlier application or patent but published on or after the international filing date | "X" | document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" document referring to an oral disclosure, use, exhibition or other means | | |
| "P" document published prior to the international filing date but later than the priority date claimed | "&" | document member of the same patent family |

| Date of the actual completion of the international search | Date of mailing of the international search report |
|---|---|
| **14 February 2022** | **28 February 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2021/135203**

## C. DOCUMENTS CONSIDERED TO BE RELEVANT

| Category* | Citation of document, with indication, where appropriate, of the relevant passages | Relevant to claim No. |
|---|---|---|
| A | US 2014343258 A1 (ASTELLAS PHARMA INC.) 20 November 2014 (2014-11-20) entire document | 1-18 |
| A | WO 2020219868 A1 (REGENXBIO INC.) 29 October 2020 (2020-10-29) entire document | 1-18 |

Form PCT/ISA/210 (second sheet) (January 2015)

**INTERNATIONAL SEARCH REPORT**

International application No.

**PCT/CN2021/135203**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**EP 4 257 603 A1**

**INTERNATIONAL SEARCH REPORT**

International application No.

**PCT/CN2021/135203**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **18**
because they relate to subject matter not required to be searched by this Authority, namely:

[1] Claim 18 relates to a method for treating or preventing diseases related to CTGF, which belongs to a subject matter defined in PCT Rule 39.1that does not warrant a search conducted by the international searching authority: (4) a method for treatment of a human body or animal body by surgery or therapy and a diagnostic method implemented on a human body or animal body. The international search is made on the basis of a use in preparation of a corresponding medicine.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
Information on patent family members

International application No.

**PCT/CN2021/135203**

| Patent document cited in search report | | | Publication date (day/month/year) | Patent family member(s) | | | Publication date (day/month/year) |
|---|---|---|---|---|---|---|---|
| WO | 2020244540 | A1 | 10 December 2020 | TW | 202110880 | A | 16 March 2021 |
| | | | | CA | 3142092 | A1 | 10 December 2020 |
| US | 2014127224 | A1 | 08 May 2014 | US | 9102721 | B2 | 11 August 2015 |
| | | | | WO | 2012100262 | A1 | 26 July 2012 |
| US | 2004248206 | A1 | 09 December 2004 | RU | 2005141492 | A | 20 July 2007 |
| | | | | RU | 2330861 | C2 | 10 August 2008 |
| | | | | EP | 2338914 | A1 | 29 June 2011 |
| | | | | CA | 2526509 | A1 | 16 December 2004 |
| | | | | CA | 2526509 | C | 06 August 2013 |
| | | | | EP | 1631591 | A2 | 08 March 2006 |
| | | | | EP | 1631591 | B1 | 21 December 2011 |
| | | | | US | 2019023778 | A1 | 24 January 2019 |
| | | | | DK | 1631591 | T3 | 10 April 2012 |
| | | | | EP | 2322549 | A1 | 18 May 2011 |
| | | | | CN | 103539858 | A | 29 January 2014 |
| | | | | US | 2016024198 | A1 | 28 January 2016 |
| | | | | AU | 2004245514 | A1 | 16 December 2004 |
| | | | | AU | 2004245514 | B2 | 01 October 2009 |
| | | | | NO | 20060027 | L | 03 January 2006 |
| | | | | NO | 336451 | B1 | 24 August 2015 |
| | | | | IN | 83DELNP2005 | A | 30 October 2009 |
| | | | | IN | 83DELNP2006 | A | 24 August 2007 |
| | | | | IN | 233809 | A1 | 24 April 2009 |
| | | | | IL | 172328 | A | 31 January 2012 |
| | | | | CN | 1829740 | A | 06 September 2006 |
| | | | | CN | 1829740 | B | 29 May 2013 |
| | | | | NZ | 543522 | A | 30 June 2008 |
| | | | | US | 2021230259 | A1 | 29 July 2021 |
| | | | | IN | 1993DELNP2009 | A | 19 June 2009 |
| | | | | US | 2009017043 | A1 | 15 January 2009 |
| | | | | US | 7871617 | B2 | 18 January 2011 |
| | | | | WO | 2004108764 | A2 | 16 December 2004 |
| | | | | WO | 2004108764 | A3 | 03 March 2005 |
| | | | | US | 2014073047 | A1 | 13 March 2014 |
| | | | | US | 9034643 | B2 | 19 May 2015 |
| | | | | US | 2011091468 | A1 | 21 April 2011 |
| | | | | KR | 20060030032 | A | 07 April 2006 |
| | | | | KR | 101244113 | B1 | 18 March 2013 |
| | | | | US | 7405274 | B2 | 29 July 2008 |
| | | | | JP | 2007525194 | A | 06 September 2007 |
| | | | | JP | 5624707 | B2 | 12 November 2014 |
| | | | | US | 2016257740 | A1 | 08 September 2016 |
| | | | | JP | 2012143239 | A | 02 August 2012 |
| | | | | JP | 5753506 | B2 | 22 July 2015 |
| | | | | ES | 2379662 | T3 | 30 April 2012 |
| | | | | BR | PI0410963 | A | 04 July 2006 |
| | | | | BR | PI0410963 | B1 | 08 October 2019 |
| | | | | BR | PI0410963 | B8 | 25 May 2021 |
| | | | | AT | 538136 | T | 15 January 2012 |
| CN | 102666587 | A | 12 September 2012 | EP | 2448971 | A1 | 09 May 2012 |

Form PCT/ISA/210 (patent family annex) (January 2015)

<div align="center">

**INTERNATIONAL SEARCH REPORT**
Information on patent family members

</div>

| | International application No. |
|---|---|
| | **PCT/CN2021/135203** |

| Patent document cited in search report | | | Publication date (day/month/year) | Patent family member(s) | | | Publication date (day/month/year) |
|---|---|---|---|---|---|---|---|
| | | | | US | 2012164151 | A1 | 28 June 2012 |
| | | | | US | 8771692 | B2 | 08 July 2014 |
| | | | | WO | 2011002525 | A1 | 06 January 2011 |
| | | | | US | 2014356374 | A1 | 04 December 2014 |
| US | 2012263680 | A1 | 18 October 2012 | SG | 10201604560 T | A | 28 July 2016 |
| | | | | MX | 2013011771 | A | 30 July 2014 |
| | | | | MX | 359516 | B | 01 October 2018 |
| | | | | SG | 194135 | A1 | 29 November 2013 |
| | | | | KR | 20140063517 | A | 27 May 2014 |
| | | | | KR | 101862291 | B1 | 29 May 2018 |
| | | | | ES | 2711670 | T3 | 06 May 2019 |
| | | | | CA | 3042808 | A1 | 18 October 2012 |
| | | | | EP | 2696888 | A2 | 19 February 2014 |
| | | | | EP | 2696888 | A4 | 20 April 2016 |
| | | | | EP | 2696888 | B1 | 05 December 2018 |
| | | | | JP | 2014533235 | A | 11 December 2014 |
| | | | | JP | 6031510 | B2 | 24 November 2016 |
| | | | | US | 2019022169 | A1 | 24 January 2019 |
| | | | | RU | 2013150249 | A | 20 May 2015 |
| | | | | RU | 2620066 | C2 | 22 May 2017 |
| | | | | US | 9642888 | B2 | 09 May 2017 |
| | | | | MX | 326830 | B | 08 January 2015 |
| | | | | MX | 329474 | B | 17 April 2015 |
| | | | | CN | 104302310 | A | 21 January 2015 |
| | | | | AU | 2012242768 | A1 | 24 October 2013 |
| | | | | AU | 2012242768 | B2 | 12 October 2017 |
| | | | | NZ | 616672 | A | 29 April 2016 |
| | | | | WO | 2012142320 | A2 | 18 October 2012 |
| | | | | WO | 2012142320 | A8 | 20 February 2014 |
| | | | | WO | 2012142320 | A3 | 11 June 2015 |
| | | | | HK | 1255005 | A1 | 02 August 2019 |
| | | | | DK | 2696888 | T3 | 25 March 2019 |
| | | | | CN | 108014340 | A | 11 May 2018 |
| | | | | BR | 112013026313 | A2 | 27 December 2016 |
| | | | | BR | 112013026313 | A8 | 30 January 2018 |
| | | | | CA | 2832910 | A1 | 18 October 2012 |
| | | | | CA | 2832910 | C | 02 July 2019 |
| | | | | CN | 111110850 | A | 08 May 2020 |
| WO | 2010042201 | A1 | 15 April 2010 | None | | | |
| US | 2014343258 | A1 | 20 November 2014 | US | 9587015 | B2 | 07 March 2017 |
| | | | | TW | 201333034 | A | 16 August 2013 |
| | | | | PT | 2796550 | T | 18 April 2018 |
| | | | | EA | 201491240 | A1 | 28 November 2014 |
| | | | | EA | 029290 | B1 | 30 March 2018 |
| | | | | BR | 112014015405 | A2 | 13 June 2017 |
| | | | | ES | 2665851 | T3 | 27 April 2018 |
| | | | | KR | 20140107507 | A | 04 September 2014 |
| | | | | IN | 4615CHN2014 | A | 18 September 2015 |
| | | | | AR | 089425 | A1 | 20 August 2014 |
| | | | | PL | 2796550 | T3 | 31 August 2018 |

**INTERNATIONAL SEARCH REPORT**
Information on patent family members

International application No.

**PCT/CN2021/135203**

| Patent document cited in search report | | | Publication date (day/month/year) | Patent family member(s) | | | Publication date (day/month/year) |
|---|---|---|---|---|---|---|---|
| | | | | CN | 104011206 | A | 27 August 2014 |
| | | | | CA | 2859627 | A1 | 27 June 2013 |
| | | | | MX | 2014007681 | A | 25 November 2014 |
| | | | | MX | 345019 | B | 11 January 2017 |
| | | | | JP | WO2013094723 | A1 | 27 April 2015 |
| | | | | JP | 6040943 | B2 | 07 December 2016 |
| | | | | EP | 2796550 | A1 | 29 October 2014 |
| | | | | EP | 2796550 | A4 | 19 August 2015 |
| | | | | EP | 2796550 | B1 | 28 February 2018 |
| | | | | WO | 2013094723 | A1 | 27 June 2013 |
| WO | 2020219868 | A1 | 29 October 2020 | AU | 2020262416 | A1 | 16 December 2021 |
| | | | | TW | 202106708 | A | 16 February 2021 |
| | | | | WO | 2020219868 | A9 | 16 September 2021 |
| | | | | CA | 3137284 | A1 | 29 October 2020 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 1829740 B

**Non-patent literature cited in the description**

- **KABAT et al.** Sequences of Proteins of Immunological Interest. Public Health Service, National Institutes of Health, 1991
- **LEFRANC M.P.** *Dev. Comp. Immunol.,* 2003, vol. 27, 55-77

- Antibodies: A Laboratory Manual and Molecular Cloning: A Laboratory Manua. Cold Spring Harbor Laboratory